# EUROPEAN PATENT APPLICATION

(11) **EP 3 278 808 A1**
(43) Date of publication of application: **07.02.2018**
(21) Application number: 17188681.5
(22) Date of filing: 12.08.2011
(51) Int. Cl.: A61K 35/12, A61K 35/44, A61K 38/17, A61P 35/00, C12N 5/0789, A61K 35/28

(54) **IMPROVED HEMATOPOIETIC STEM AND PROGENITOR CELL THERAPY**

(30) Priority: 12.08.2010 US 373212 P
(62) Divisional of application: 11817137.0
(71) Applicant: Fate Therapeutics, Inc., San Diego, California 92121 (US)
(72) Inventor: SHOEMAKER, Daniel, San Diego, CA 92130 (US); MULTANI, Pratik, San Diego, CA 92130 (US); DESPONTS, Caroline, San Diego, CA 92126 (US); ROBBINS, David, Temecula, CA 92592 (US); GRAYSON, Paul, La Jolla, CA 92037 (US); MENDLEIN, John, Encinitas, CA 92024 (US)
(74) Representative: Schiweck, Weinzierl & Koch Patentanwälte Partnerschaft mbB

(57) **Abstract**

The invention provides improved methods for cell therapy. In particular, the invention provides therapeutic compositions of modified hematopoietic stem and/or progenitor cells having improved engraftment and homing properties, and methods of making the therapeutic composition. The invention further provides methods of improving the efficacy of hematopoietic stem and progenitor cell transplantation including transplanting the therapeutic composition to subjects in need of hematopoietic system reconstitution.

## Description

### RELATED APPLICATIONS

This application claims the benefit under 35 U.S.C. § 119(e) of U.S. Provisional Patent Application No. 61/373,212 filed August 12, 2010, which is herein incorporated by reference in its entirety.

### BACKGROUND

### Technical Field

The present invention generally relates to cell therapy. Particularly, the present invention relates to improved cell therapies for the hematopoietic system. More particularly, the present invention relates to improved methods for reconstituting the hematopoietic system of an individual.

### Description of the Related Art

Regenerative medicine is a field of medical research developing treatments to repair or restore specific cells, tissues, and organs in the body. One aspect of regenerative therapy being pursued is the use of hematopoietic stem cell transplants to treat an expanding list of cancers and degenerative disorders. According to the National Marrow Donor Program® (NMDP), an estimated 45,000 to 50,000 hematopoietic cell transplants (bone marrow, peripheral blood stem cells (PBSC), or cord blood transplants), including approximately 20,000 allogeneic hematopoietic cell transplants, are performed annually worldwide to treat patients with life-threatening malignant and non-malignant diseases (Horowitz MM. Uses and Growth of Hematopoietic Cell Transplantation. In: Blume KG, Forman SJ, Appelbaum FR, eds. Thomas' Hematopoietic Cell Transplantation. 3rd ed. Malden, Mass: Blackwell; 2004:9-15). Moreover, approximately 4,800 patients are transplanted annually using unrelated donors or cord blood units through the NMDP.

Since it began operations in 1987, the NMDP has facilitated more than 38,000 marrow and cord blood transplants to give patients a second chance at life. Traditionally, hematopoietic stem cell transplants from bone marrow were used to treat patients suffering from various types of leukemias, anemias, lymphomas, myelomas, immune deficiency disorders, and solid tumors, e.g., breast and ovarian cancer. However, bone marrow transplantation is painful for donors and moreover, it is often difficult and time consuming to find the requisite degree of HLA donor matched tissue, especially in particular ethnic populations. In addition, allogeneic bone marrow transplants are often associated with a significant incidence of graft-versus-host-disease (GVHD).

In many instances, patients receiving hematopoietic stem cell transplants have advanced cancer or other metabolic disorders, which are life threatening. Thus, any delays in finding a donor having a suitably matched HLA tissue type can compromise the patient outcome, often resulting in fatality. Accordingly, recent growth in the number of NMDP unrelated donor transplants has been especially dramatic, with more than 4,800 transplants in 2009 alone, compared with 4,300 in 2008. In addition, the proportion of allogeneic transplants using unrelated donors or cord blood units has increased steadily. In 2006, more than one-third of allogeneic transplants performed worldwide used unrelated donors. In 2009, 75% of adult donors - more than 2,800 - donated PBSC to patients through the NMDP. In 2009, 1,056 cord blood transplants were facilitated by the NMDP, which represents 22% of the total number of NMDP transplants in 2009. This is an 18% increase from 2008, when the NMDP facilitated 898 cord blood transplants.

Allogeneic hematopoietic stem cell transplants have been performed using umbilical cord blood because the blood is more easily obtainable, carries a lower risk to the recipient of graft-versus-host disease, is painless for the donor, and requires less of an HLA tissue type match between donor and recipient.

However, several drawbacks are perceived to exist in using human cord blood transplants, including the risk that hematopoietic stem and progenitor cells from the cord blood transplant may not engraft.

Another drawback of using cord blood transplants is that it takes longer for the cord blood cells to engraft in the patient, which puts the patient at high risk for infection. In addition, cord blood transplants are a newer treatment approach. Thus, clinicians can be discouraged from using them because they do not have as much information about patients' long-term results after cord blood transplants as they do for marrow transplants. Moreover, cord blood transplants also have all the same risks as marrow and peripheral blood transplants.

Additionally, a significant barrier to using cord blood as a source of cells for human blood transplants is that there are often not enough blood-forming cells in a single cord blood unit for the size of the patient or to treat the particular indication. Because the size of a single cord (*i.e.,* the number of blood-forming cells in a single cord) is often insufficient for a blood transplant, two cords may be required, increasing the risks of GVHD and failure to engraft. Thus, numerous approaches have been tried to expand the number of human hematopoietic stem and progenitor cells in cord blood within isolated grafts in *ex vivo* settings, which may allow transplantation using a single cord, but these efforts have had limited success.

Thus, the promise of restorative or regenerative hematopoietic stem cell therapies has not been realized, in part, due to difficulties translating promising animal models protocols into human clinical practice, low efficacy of existing clinical protocols, high incidence of complications, e.g., graft-versus-host disease, and relatively few sufficiently matched donors.

Accordingly, there exists a need in the art for methods that can increase the efficiency of hematopoietic stem and progenitor cell engraftment to the bone marrow, in order to broaden the applicability and increase the success of hematopoietic stem cell transplantation. The present invention provides solutions to these problems and further provides other uses and advantages that will be apparent to persons skilled in the art.

### SUMMARY OF THE INVENTION

The invention provides improved hematopoietic stem and progenitor cell transplantation methods. Moreover, the invention provides a superior preparation of hematopoietic stem or progenitor cells that have increased engraftment/engraftment potential and/or increased expansion. In various embodiments, the cells are expanded or proliferated *in vivo.* In various other embodiments, cells are treated *ex vivo,* administered to a subject and expanded or proliferated *in vivo.*

In one aspect, the invention provides a therapeutic composition comprising a population of cells comprising at least about one million human hematopoietic stem or progenitor cells wherein a) the hematopoietic stem or progenitor cells have been contacted *ex vivo* at a temperature of about 37°C with an agent that increases CXCR4 gene expression in the cells; b) gene expression of CXCR4 is increased in the hematopoietic stem or progenitor cells by at least about 2 fold compared to the expression of CXCR4 in a non-contacted hematopoietic stem or progenitor cell; and c) wherein the therapeutic composition comprises a sterile, therapeutically acceptable suspension of hematopoietic stem or progenitor cells ready for administration to a patient.

In a particular embodiment, the gene expression of CXCR4 in the hematopoietic stem or progenitor cells of the therapeutic composition is increased by at least about 3 fold compared to the expression of CXCR4 in a non-contacted hematopoietic stem or progenitor cell.

In one embodiment, the population of cells comprises a collection of CD34⁺ cells wherein gene expression of CXCR4 in the collection of CD34⁺ cells is increased by at least about 3 fold compared to a non-contacted collection of CD34⁺ cells.

In another embodiment, the gene expression of CXCR4 is increased by about 3 fold to about 8 fold compared to the expression of CXCR4 in a non-contacted hematopoietic stem or progenitor cell.

In yet another embodiment of the therapeutic composition, gene expression of CXCR4 is increased by at least about 4 fold compared to the expression of CXCR4 in a non-contacted hematopoietic stem or progenitor cell.

In yet another embodiment of the therapeutic composition, gene expression of CXCR4 is increased by at least about 6 fold compared to the expression of CXCR4 in a non-contacted hematopoietic stem or progenitor cell. In still another embodiment of the therapeutic composition, gene expression of CXCR4 is increased by at least about 7 fold compared to the expression of CXCR4 in a non-contacted hematopoietic stem or progenitor cell.

In another embodiment of the therapeutic composition, gene expression of CXCR4 is increased by at least about 8 fold compared to the expression of CXCR4 in a non-contacted hematopoietic stem or progenitor cell. In yet another embodiment of the therapeutic composition, gene expression of CXCR4 is increased by at least about 10 fold compared to the expression of CXCR4 in a non-contacted hematopoietic stem or progenitor cell.

In still another embodiment of the invention, gene expression of CXCR4 is increased by at least about 12 fold compared to the expression of CXCR4 in a non-contacted hematopoietic stem or progenitor cell.

In an additional embodiment of the invention, gene expression of CXCR4 is increased by at least about 16 fold compared to the expression of CXCR4 in a non-contacted hematopoietic stem or progenitor cell.

In some embodiments, gene expression of CXCR4 in the hematopoietic stem or progenitor cells comprising the therapeutic composition is increased by about 8 to about 18 fold compared to the expression of CXCR4 in a non-contacted hematopoietic stem or progenitor cell.

In various embodiments of the invention, the agent that increases CXCR4 gene expression in the hematopoietic stem or progenitor cells is selected from the group consisting of a cAMP enhancer, a Gα-s activator, and a compound that selectively binds the PGE₂ EP₄ receptor.

In particular embodiments, the agent that increases CXCR4 gene expression in the hematopoietic stem or progenitor cells is PGE₂, or a PGE₂ analogue or derivative. In more particular embodiments, the agent that increases CXCR4 gene expression in the hematopoietic stem or progenitor cells is 16,16-dimethyl PGE₂.

In one embodiment of the therapeutic composition of the invention, the hematopoietic stem or progenitor cells have been contacted with the agent for a time of at least about one hour. In various embodiments, the hematopoietic stem or progenitor cells have been contacted with the agent for a time of about one hour to about six hours. In particular embodiments, the hematopoietic stem or progenitor cells have been contacted with the agent for a time of about two hours to about six hours. In more particular embodiments, the hematopoietic stem or progenitor cells comprising the therapeutic composition have been contacted with the agent for a time of about two hours.

In particular embodiments of the invention, the hematopoietic stem or progenitor cells comprising the therapeutic composition comprise a gene expression signature wherein expression of one or more signature genes is increased by at least about 2 fold compared to the expression of the one or more signature genes in a noncontacted hematopoietic stem or progenitor cell, wherein the signature gene is selected from the group consisting of: hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), and Fos-related antigen 2 (FOSL2).

In more particular embodiments, expression of at least two of the signature genes is increased by at least 5, 10, 15, or 20 fold compared to the expression of the two signature genes in a non-contacted hematopoietic stem or progenitor cell. In more particular embodiments, expression of each of the signature genes is increased by at least about 2 fold compared to the expression of the signature genes in a non-contacted hematopoietic stem or progenitor cell.

In various embodiments, the population of cells comprising the therapeutic composition comprises less than about .10, .50, 1.0, 3, 5, 10, 15, 20, or 30% CD34⁺ cells. In some embodiments, the population of cells comprises at least about .01% and no more than about 50% CD34⁺ cells.

In some embodiments, the population of cells is not expanded *ex vivo.*

In various embodiments, the therapeutic composition is generated at a point-of-care and is administered into a patient without culturing the population of cells. In some embodiments, the therapeutic composition is generated within 24 hours of administering the composition to the patient. In some embodiments, the therapeutic composition is generated within 12 hours of administering the composition to the patient. In some embodiments, the therapeutic composition is generated within 6 hours of administering the composition to the patient. In some embodiments, the therapeutic composition is generated on the day of infusion of the composition.

In some embodiments, the therapeutic composition is substantially free of the agent. In various embodiments, the therapeutic composition comprises hematopoietic stem or progenitor cells suspended in a solution of 5% human serum albumin and dextran.

In some embodiments, the therapeutic composition comprises less than about 30%, 25%, 20%, 15%, 10% or 5% mesenchymal stem cells. In particular embodiments, the therapeutic composition comprises no more than about 10% mesenchymal stem cells.

In some embodiments, the therapeutic composition comprises less than about 30%, 25%, 20%, 15%, 10% or 5% endothelial progenitor cells. In particular embodiments, the therapeutic composition comprises no more than about 10% endothelial progenitor cells.

In particular embodiments of the invention, the population of cells comprises cells positive for the cell surface marker CD34, and comprises less than about 30%, 25%, 20%, 15%, 10% or 5% of cells positive for a cell surface marker selected from the group consisting of CD73, CD140B, CD14 and VWF.

In particular embodiments, the population of cells comprising the therapeutic composition of the invention comprises CD34⁺ cells and comprises less than about 30%, 25%, 20%, 15%, 10% or 5% CD14⁺/CD45⁻ cells. In other embodiments of the invention, the population of cells comprises CD34⁺ cells and comprises less than about 30%, 25%, 20%, 15%, 10% or 5% VWF⁺ cells. In other embodiments of the invention, the population of cells comprises CD34⁺ cells and comprises less than about 30%, 25%, 20%, 15%, 10% or 5% CD140B+ cells.

In more particular embodiments, the population of cells comprises CD34⁺ hematopoietic stem or progenitor cells and comprises less than about 30%, 25%, 20%, 15%, 10% or 5% of CD14+/CD45- cells, VWF⁺ cells, CD73⁺ cells, and CD140B⁺ cells. In some embodiments, the population of cells is positive for the cell surface marker CD34 and is negative for at least one cell surface marker from the group consisting of CD14, VWF, CD73, and CD140B. In other embodiments, the population of cells is positive for the cell surface marker CD34 and is negative for the cell surface markers CD14, VWF, CD73, and CD140B.

In various embodiments of the invention, at least about 15% of cells within the population of cells express CXCR4 protein.

In some embodiments, the population of cells is obtained from bone marrow, umbilical cord blood, or mobilized peripheral blood.

In particular embodiments, the population of cells is HLA haplotyped. In more particular embodiments, the population of cells is HLA haplotyped based on the group consisting of HLA-A, HLA-B, HLA-C, and HLA-DRB1. In some embodiments, the population of HLA haplotyped cells is matched with a specific human subject. In some embodiments, the population of HLA haplotyped cells has 4 out of 6 HLA matches with a specific human subject.

In another embodiment, the invention provides a therapeutic composition comprising a population of cells comprising at least about one million human hematopoietic stem or progenitor cells wherein a) the hematopoietic stem or progenitor cells have been contacted *ex vivo* at a temperature of about 37°C with 16,16-dmPGE₂ for a time of about two hours; b) the hematopoietic stem or progenitor cells comprise a collection of CD34⁺ cells wherein gene expression of CXCR4 is increased in the collection of CD34⁺ cells by at least about 3 fold compared to the expression of CXCR4 in non-contacted CD34⁺ cells; and c) wherein the therapeutic composition comprises a sterile, therapeutically acceptable suspension of hematopoietic stem or progenitor cells ready for administration to a patient.

In some embodiments, the population of cells comprises a collection of CD34⁺ cells wherein gene expression of CXCR4 in the collection of CD34⁺ cells is increased by at least about 3 fold compared to a non-contacted collection of CD34⁺ cells.

In various embodiments, the gene expression of CXCR4 is increased by about 3 fold to about 8 fold compared to the expression of CXCR4 in a non-contacted hematopoietic stem or progenitor cell. In more particular embodiments, gene expression of CXCR4 is increased by at least about 4 fold compared to the expression of CXCR4 in a non-contacted hematopoietic stem or progenitor cell.

In other particular embodiments, gene expression of CXCR4 is increased by at least about 6 fold compared to the expression of CXCR4 in a non-contacted hematopoietic stem or progenitor cell. In other particular embodiments, gene expression of CXCR4 is increased by at least about 7 fold compared to the expression of CXCR4 in a non-contacted hematopoietic stem or progenitor cell. In further embodiments, the gene expression of CXCR4 is increased by at least about 8 fold compared to the expression of CXCR4 in a non-contacted hematopoietic stem or progenitor cell. In other embodiments, gene expression of CXCR4 is increased by at least about 10 fold compared to the expression of CXCR4 in a non-contacted hematopoietic stem or progenitor cell. In other embodiments, gene expression of CXCR4 is increased by at least about 12 fold compared to the expression of CXCR4 in a non-contacted hematopoietic stem or progenitor cell. In other embodiments, gene expression of CXCR4 is increased by at least about 16 fold compared to the expression of CXCR4 in a non-contacted hematopoietic stem or progenitor cell. In other embodiments, gene expression of CXCR4 is increased by about 8 to about 18 fold compared to the expression of CXCR4 in a non-contacted hematopoietic stem or progenitor cell.

In some embodiments, the hematopoietic stem or progenitor cells comprise a gene expression signature wherein expression of one or more signature genes is increased by at least about 2 fold compared to the expression of the one or more signature genes in a noncontacted hematopoietic stem or progenitor cell, wherein the signature gene is selected from the group consisting of: hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), and Fos-related antigen 2 (FOSL2).

In other embodiments, expression of at least two of the signature genes is increased by at least 5, 10, 15, or 20 fold compared to the expression of the two signature genes in a non-contacted hematopoietic stem or progenitor cell.

In a particular embodiment, expression of each of the signature genes is increased by at least about 2 fold compared to the expression of the signature genes in a non-contacted hematopoietic stem or progenitor cell.

In other embodiments, the population of cells does not comprise more than about .10, .50, 1.0, 3, 5, 10, 15, 20, or 30% CD34⁺ cells. In more particular embodiments, the population of cells comprises at least about .01% and no more than about 50% of CD34⁺ cells.

In various embodiments, the population of cells is not expanded *ex vivo.*

In particular embodiments, the population of cells is obtained from bone marrow, umbilical cord blood, or mobilized peripheral blood.

In some embodiments, the population of cells is HLA haplotyped.

In another embodiment, the invention provides a therapeutic composition comprising a population of haplotyped cells comprising at least about one million human hematopoietic stem or progenitor cells wherein a) the hematopoietic stem or progenitor cells have been contacted *ex vivo* at a temperature of about 37°C with an agent that increases CXCR4 gene expression in the cells; b) gene expression of CXCR4 is increased in the hematopoietic stem or progenitor cells by at least about 2 fold compared to the expression of CXCR4 in a non-contacted hematopoietic stem or progenitor cell; and c) wherein the therapeutic composition comprises a sterile, therapeutically acceptable suspension of hematopoietic stem or progenitor cells ready for administration to a patient.

In a particular embodiment, the population of halpotyped cells is haplotyped based on the group consisting of HLA-A, HLA-B, HLA-C, and HLA-DRB1. In more particular embodiments, the population of halpotyped cells is haplotyped based on the group consisting of HLA-DRB3/4/5, HLA-DQB1, and DPB1. In some embodiments, the population of haplotyped cells is matched with a specific human subject. In various embodiments, the population of HLA haplotyped cells has 4 out of 6 HLA matches with a specific human subject.

In some embodiments, gene expression of CXCR4 in the hematopoietic stem or progenitor cells is increased by at least about 3 fold compared to the expression of CXCR4 in a non-contacted hematopoietic stem or progenitor cell. In particular embodiments, gene expression of CXCR4 is increased by about 3 fold to about 8 fold compared to the expression of CXCR4 in a non-contacted hematopoietic stem or progenitor cell. In other particular embodiments, gene expression of CXCR4 is increased by at least about 4 fold compared to the expression of CXCR4 in a non-contacted hematopoietic stem or progenitor cell.

In particular embodiments, the agent that increases CXCR4 gene expression in the hematopoietic stem or progenitor cells is selected from the group consisting of a cAMP analogue or enhancer, a Gα-s activator, and a compound that selectively binds the PGE₂ EP₄ receptor. In more particular embodiments, the agent that increases CXCR4 gene expression in the hematopoietic stem or progenitor cells is 16,16-dimethyl PGE₂.

In various embodiments, the hematopoietic stem or progenitor cells have been contacted with the agent for a time of about one hour to about six hours.

In some embodiments, the hematopoietic stem or progenitor cells comprise a gene expression signature wherein expression of one or more signature genes is increased by at least about 2 fold compared to the expression of the one or more signature genes in a noncontacted hematopoietic stem or progenitor cell, wherein the signature gene is selected from the group consisting of: hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), and Fos-related antigen 2 (FOSL2).

In particular embodiments, expression of at least two of the signature genes is increased by at least 5, 10, 15, or 20 fold compared to the expression of the two signature genes in a non-contacted hematopoietic stem or progenitor cell.

In other embodiments, expression of each of the signature genes is increased by at least about 2 fold compared to the expression of the signature genes in a non-contacted hematopoietic stem or progenitor cell. In other embodiments, expression of each of the signature genes is increased by at least about 4 fold compared to the expression of the signature genes in a non-contacted hematopoietic stem or progenitor cell. In other embodiments, expression of each of the signature genes is increased by at least about 6 fold compared to the expression of the signature genes in a non-contacted hematopoietic stem or progenitor cell.

In yet other embodiments, the population of cells comprises less than about .10, .50, 1.0, 3, 5, 10, 15, 20, or 30% CD34⁺ cells.

In more particular embodiments, the population of cells comprises at least about .01% and no more than about 50% of CD34⁺ cells.

In some embodiments, the population of cells is not expanded *ex vivo.*

In other embodiments, the therapeutic composition is generated at a point-of-care and is administered into a patient without culturing the population of cells. In some embodiments, the therapeutic composition is generated less than about 24 hours before administering the composition to the patient. In some embodiments, the therapeutic composition is generated less than about 12 hours before administering the composition to the patient. In some embodiments, the therapeutic composition is generated less than about 6 hours before administering the composition to the patient. In some embodiments, the therapeutic composition is generated on the day of infusion of the composition.

In other embodiments, the population of cells comprising the therapeutic composition is obtained from bone marrow, umbilical cord blood, or mobilized peripheral blood.

In another embodiment, the invention contemplates, in part, a method of preparing a therapeutic composition for use in a hematopoietic stem or progenitor cell transplant comprising: contacting a population of cells comprising hematopoietic stem or progenitor cells, *ex vivo* or *in vitro,* at a temperature of about 37°C, under conditions sufficient to modify the gene expression of the hematopoietic stem or progenitor cells to result in hematopoietic stem or progenitor cells comprising a gene expression signature comprising increased expression, as compared to non-contacted hematopoietic stem or progenitor cells, of one or more of the following genes: hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), Fos-related antigen 2 (FOSL2), or CXC chemokine receptor 4 (CXCR4).

In another embodiment, the invention contemplates, in part, a method of increasing hematopoietic stem or progenitor cell engraftment in a subject comprising: contacting a population of cells that comprises hematopoietic stem or progenitor cells *ex vivo* at a temperature of about 37°C with one or more agents selected from the group consisting of PGE₂ and an agent having dmPGE₂ activity; washing the population of cells to substantially remove the agent; and administering the contacted population of cells to a subject; wherein the population of cells is contacted with the agent under conditions sufficient to increase the engraftment of the contacted hematopoietic stem or progenitor cells in the subject compared to non-contacted hematopoietic stem or progenitor cells.

In one embodiment, the invention contemplates, in part, a method of treating a subject in need of hematopoietic system reconstitution comprising: selecting a subject in need of hematopoietic reconstitution; contacting a population of cells that comprises hematopoietic stem or progenitor cells *ex vivo* at a temperature of about 37°C with one or more agents selected from the group consisting of PGE₂ and an agent having dmPGE₂ activity; washing the population of cells to substantially remove the agent; and administering the contacted population of cells to the subject; wherein the population of cells is contacted with the agent under conditions sufficient to increase the engraftment of the contacted hematopoietic stem or progenitor cells in the subject compared to non-contacted hematopoietic stem or progenitor cells thereby treating the subject in need of hematopoietic system reconstitution.

In an additional embodiment, the invention contemplates, in part, a method of treating a subject in need of hematopoietic system reconstitution comprising: selecting the subject in need of hematopoietic reconstitution; contacting a population of cells that comprises hematopoietic stem or progenitor cells at a temperature of about 37°C with one or more agents selected from the group consisting of: a prostaglandin E₂ (PGE₂) or an agent having dmPGE₂ activity; washing the population of cells to substantially remove the agent; and administering the contacted population of cells to the subject; wherein the population of cells is contacted with the agent under conditions sufficient to increase the engraftment of the contacted hematopoietic stem or progenitor cells in the subject compared to non-contacted hematopoietic stem or progenitor cells thereby treating the subject in need of hematopoietic system reconstitution.

In a further embodiment, the invention contemplates, in part, a method of preparing a population of cells for increasing hematopoietic stem or progenitor cell expansion *in vivo* comprising: contacting a population of cells comprising hematopoietic stem or progenitor cells, *ex vivo* or *in vitro,* at a temperature of about 37°C, with one or more agents selected from the group consisting of: a prostaglandin E₂ (PGE₂) or an agent having dmPGE₂ activity; wherein the population of cells is contacted with the agent under conditions sufficient to increase the expansion of the contacted hematopoietic stem or progenitor cells *in vivo* compared to non-contacted hematopoietic stem or progenitor cells.

In particular embodiments of the methods of the invention, the population of cells is obtained from bone marrow, umbilical cord blood, or mobilized peripheral blood.

In other particular embodiments of the invention, the agent having dmPGE₂ activity is dmPGE₂, a cAMP analogue or enhancer, or a Gα-s activator.

In certain embodiments, the agent is PGE₂ or an analogue thereof.

In certain particular embodiments, the PGE₂ analogue is 16,16-dimethyl PGE₂.

In other particular embodiments, the agent is a cAMP enhancer.

In certain embodiments of the invention, the conditions sufficient to modify the gene expression of, increase the engraftment of or engraftment potential of, or increase the expansion of, the contacted hematopoietic stem or progenitor cell population comprise contacting the cell population with the one or more agents for a time of about 1 hour to about 6 hours, wherein at least one of the agents comprises an agent that increases PGE₂R₂ or PGE₂R4 signaling in the hematopoietic stem or progenitor cells. In certain embodiments, the hematopoietic stem or progenitor cells are contacted at a temperature of about 37°C for a time of about 2 hours with a concentration of about 10 µM of the agent that increases PGE₂R₂ or PGE₂R4 signaling in the hematopoietic stem or progenitor cells.

In other embodiments, the hematopoietic stem or progenitor cell population is contacted with a concentration of about 10 µM or more 16,16-dimethyl PGE₂ and for a time of about 1 hour to about 6 hours.

In additional embodiments, the cell population is contacted with a concentration of about 10 µM 16,16-dimethyl PGE₂ and for a time of about 2 hours.

In particular embodiments, the increase in the engraftment potential of the contacted hematopoietic stem or progenitor cells in the cell population comprises an increase in gene expression of one or more of hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), Fos-related antigen 2 (FOSL2), or CXC chemokine receptor 4 (CXCR4) compared to non-contacted hematopoietic stem or progenitor cells, an increase in capacity for self-renewal compared to non-contacted hematopoietic stem or progenitor cells, and no substantial decrease in cell viability compared to non-contacted hematopoietic stem or progenitor cells.

In some embodiments of the invention, the population of cells comprising hematopoietic stem or progenitor cells is prepared in an endotoxin-free vessel comprising a temperature indicating device comprising at least one temperature indicator that produces a signal that indicates the temperature of the vessel and an elapsed time indicating device that comprises at least one elapsed time indicator; and wherein the vessel is suitable for cell storage, treatment of cells, washing of cells, and cell infusion.

In certain embodiments of the invention, including those methods of the invention for preparing a population of cells for a hematopoietic stem or progenitor transplant, and for preparing a population of cells for increasing hematopoietic stem or progenitor cell expansion, the contacted population of cells is administered to a subject in need thereof, such as a subject in need of cell therapy, and the method of the invention further comprises administering the contacted population of cells to a subject in need thereof.

In certain particular embodiments, the subject has acute myelogenous leukemia (AML), acute lymphoblastic leukemia (ALL), chronic myelogenous leukemia (CML), chronic lymphocytic leukemia (CLL), juvenile myelomonocytic leukemia, Hodgkin's lymphoma, non-Hodgkin's lymphoma, multiple myeloma, severe aplastic anemia, Fanconi's anemia, paroxysmal nocturnal hemoglobinuria (PNH), pure red cell aplasia, amegakaryocytosis/congenital thrombocytopenia, severe combined immunodeficiency syndrome (SCID), Wiskott-Aldrich syndrome, beta-thalassemia major, sickle cell disease, Hurler's syndrome, adrenoleukodystrophy, metachromatic leukodystrophy, myelodysplasia, refractory anemia, chronic myelomonocytic leukemia, agnogenic myeloid metaplasia, familial erythrophagocytic lymphohistiocytosis, solid tumors, chronic granulomatous disease, mucopolysaccharidoses, or Diamond Blackfan.

In certain other particular embodiments, the subject has breast cancer, ovarian cancer, brain cancer, prostate cancer, lung cancer, colon cancer, skin cancer, liver cancer, pancreatic cancer, or sarcoma.

In other certain embodiments, the subject has bone marrow ablative or non-myeolablative chemotherapy or radiation therapy..

In further embodiments, the subject is a bone marrow donor.

In particular embodiments, the population of cells comprises one or more cord blood units. In certain embodiments, the subject is administered one or more cord blood units. In other embodiments, the subject is administered a partial cord blood unit. In other particular embodiments, the subject is administered one cord blood unit.

In other embodiments, the population of cells comprising hematopoietic stem or progenitor cells is autogeneic to the subject.

In certain embodiments, the population of cells is mobilized from the peripheral blood or bone marrow of the subject.

In further embodiments, the population of cells comprising hematopoietic stem or progenitor cells is allogeneic to the subject.

### BRIEF DESCRIPTION OF THE SEVERAL VIEWS OF THE DRAWINGS

Figure 1 shows a diagrammatic representation of the prostaglandin E₂ receptor ₂/receptor 4 G-protein coupled receptor cell signaling pathway present in hematopoietic stem and progenitor cells.
Figure 2 shows an experimental flowchart for the analyses of purified populations of CD34⁺ cells or human cord blood treated with 16,16-dimethyl PGE₂ under different sets of experimental conditions.
Figure 3 shows the results for cAMP assays in CD34⁺ cells treated with 16,16-dimethyl PGE₂ under different sets of experimental conditions.
Figure 4 shows a scatterplot of gene expression data of vehicle treated CD34⁺ cells on the x-axis versus gene expression data of CD34⁺ cells treated with 10 µM 16,16-dimethyl PGE₂ on the y-axis. Noted are the 8 fold increase in CREM expression and the 18 fold increase in CXCR4 expression in CD34⁺ cells treated with 10 µM 16,16-dimethyl PGE₂ compared to vehicle treated cells.
Figure 5 shows an experimental flowchart for the analysis of treatment time on the gene expression of CD34⁺ cells treated with 10 µM 16,16-dimethyl PGE₂.
Figure 6 shows the gene expression profiles of CD34⁺ cells treated with 10 µM 16,16-dimethyl PGE₂ (y-axis) versus vehicle treated cells (x-axis) for treatment times of 5, 15, 30, 60, and 120 minutes. Gene expression profiles were obtained after the 120 minute incubation period.
Figure 7 shows the gene expression profiles of CD34⁺ cells treated at 37°C for 120 minutes with either 100 nM, 1 µM, 10 µM, or 100 µM 16,16-dimethyl PGE₂ (y-axis) versus vehicle treated cells (x-axis).
Figure 8 shows the gene expression profiles of CD34⁺ cells purified from cord blood that was treated at 37°C for 120 minutes with either 100 nM, 1 µM, 10 µM, 25 µM, or 50 µM 16,16-dimethyl PGE₂ (y-axis) versus vehicle treated cells (x-axis).
Figure 9 shows the gene expression profiles of CD34⁺ cells treated with 10 µM 16,16-dimethyl PGE₂ for 60 minutes (top panels) or 120 minutes (bottom panels) and either treated at 37°C (left panels) or 4°C (top right panel) or 25°C (bottom right panel) (y-axis) versus vehicle treated cells (x-axis).
Figure 10 shows that CD34⁺ cells incubated with 10 µM 16,16-dimethyl PGE₂ for 120 minutes at 37°C does not decrease cell viability compared to vehicle treated cells or cells treated with 10 µM 16,16-dimethyl PGE₂ for 120 minutes at 4°C.
Figure 11 shows that CD34⁺ cells incubated with 10 µM 16,16-dimethyl PGE₂ for 120 minutes at 37°C does not decrease the ability of the cells to form colony forming units compared to vehicle treated cells or cells treated with 10 µM 16,16-dimethyl PGE₂ for 120 minutes at 4°C. Colony-forming unit granulocyte -monocyte (CFU-GM); Colony-forming unit erythroid (CFU- E); Burst-forming unit erythroid (BFU-E); multi-lineage colonies-forming unit mix (CFU-GM/M/Eosi).

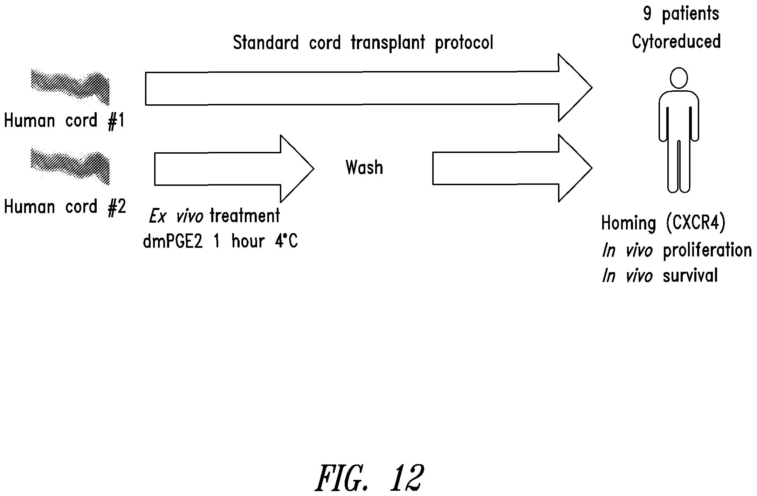

Figure 12 shows a schematic for the clinical trials using human cord blood treated with 16,16-dimethyl PGE₂.
Figure 13 shows genome-wide expression analysis of the 16,16-dimethyl PGE₂ treatment protocol. Figure 13A shows gene expression in cells treated at 4°C for 1 hour. Cell were treated with 10 µM 16,16-dimethyl PGE₂ (y-axis; N=3) compared to DMSO controls (N=3). Figure 13B shows gene expression in cells treated at 37°C for 2 hours. Cell were treated with 10 µM 16,16-dimethyl PGE₂ (y-axis; N=3) compared to DMSO controls (N=3).

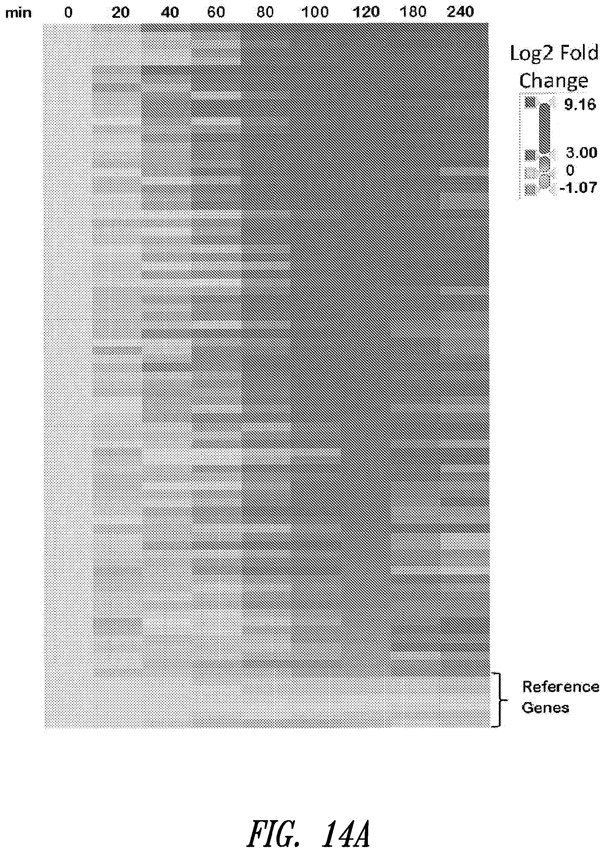

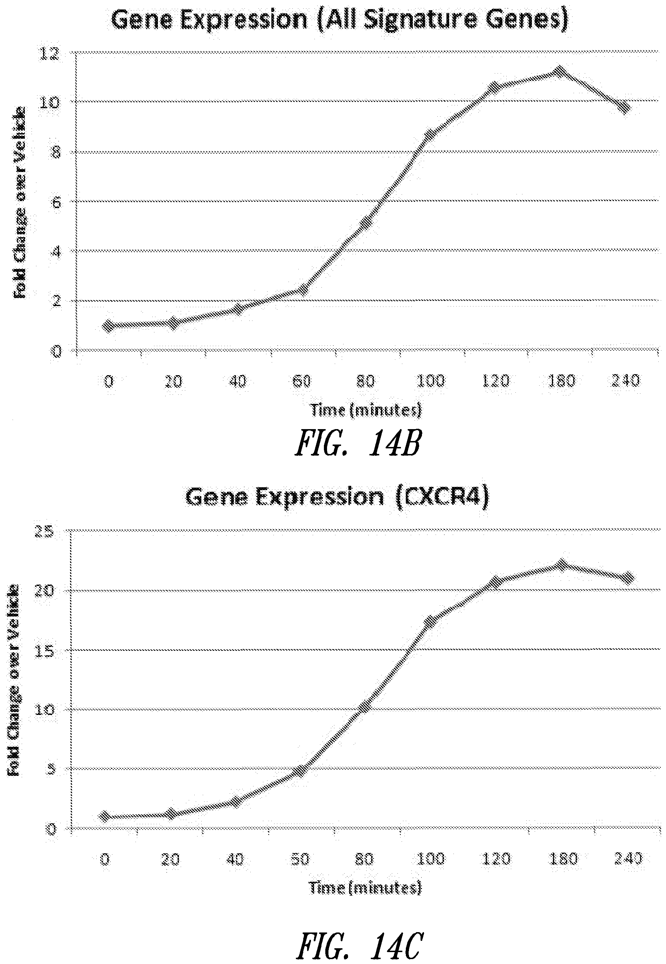

Figure 14 shows gene expression analysis validation studies for cells incubated treated with 10 µM 16,16-dimethyl PGE₂ incubation at 37°C using the Fluidigm gene expression platform. Figure 14A shows the gene expression of a group of selected genes in CD34⁺ cells treated with 10 µM 16,16-dimethyl PGE₂ at 37°C for 0, 20, 40, 60, 80, 120, 180 and 240 minutes compared to vehicle treated controls. Figure 14B shows the average gene expression of the signature genes listed in Table 3 in CD34⁺ cells treated with 10 µM 16,16-dimethyl PGE₂ at 37°C for 0, 20, 40, 60, 80, 120, 180 and 240 minutes compared to vehicle treated controls. The following genes performed poorly and were excluded in determining the average gene expression shown in Figure 14B: ARPC2, CXCL5, CXCL6, FGF9, GNAL, GULP1, LRIG2, PDE4D, PLAT(1), PLAT(2), SSTR1, SYT4 and TMCC3. The following housekeeping genes were used for normalization in determining the average gene expression shown in Panel B: ACTB, GAPDH, HPTR1, and QARS. Figure 14C shows the average gene expression of CXCR4 in CD34⁺ cells treated with 10 µM 16,16-dimethyl PGE₂ at 37°C for 0, 20, 40, 60, 80, 120, 180 and 240 minutes compared to vehicle treated controls. All gene expression profiles for Figure 14 were obtained after treatment of cells for the specified time and without further incubation of the cells after treatment.

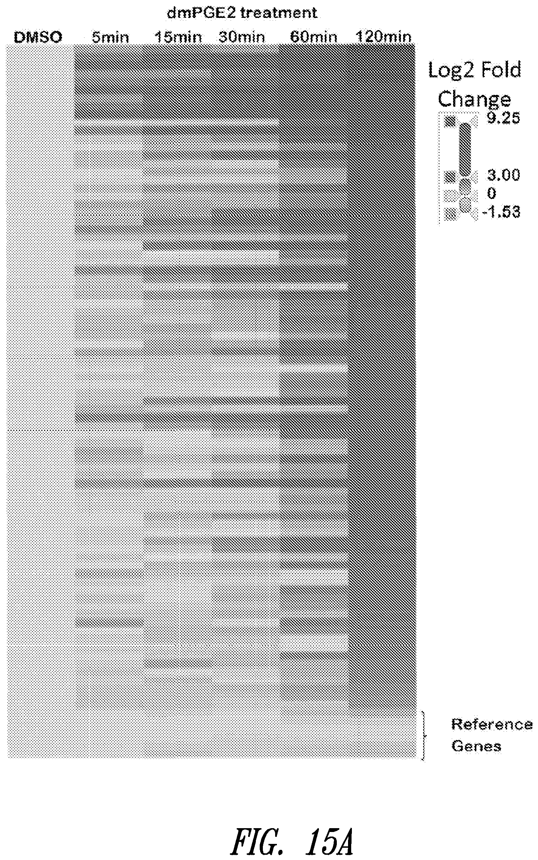

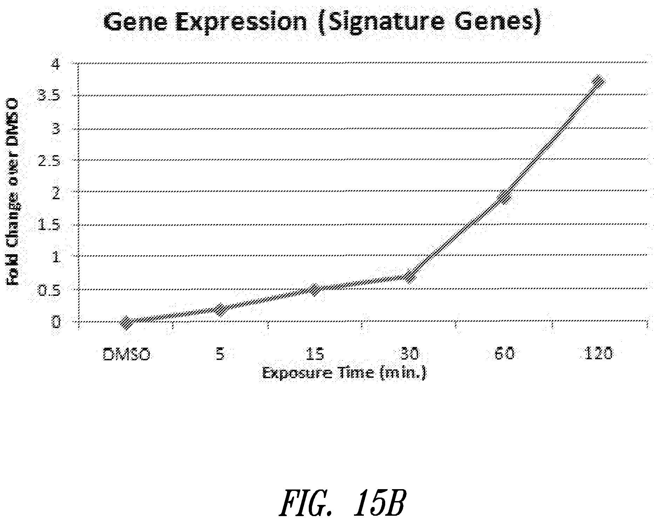

Figure 15 shows cells treated with 10 µM 16,16-dimethyl PGE₂ pulse treatment sufficient to drive the full biological effect or DMSO treated cells. CD34+ cells were incubated with 10 µM 16,16-dimethyl PGE₂ for different times as shown (0, 20, 40, 80 and 120 minutes) followed by a recovery period without 16,16-dimethyl PGE₂ at 37°C such that the total incubation time was 120 minutes. Data was analyzed using the Fluidigm gene expression platform. Figures 15A and 15B show the average gene expression of the signature genes listed in Table 3 in CD34⁺ cells treated with 10 µM 16,16-dimethyl PGE₂ or DMSO at 37°C for 5, 15, 30, 60, and 120 minutes compared to vehicle treated controls. The following genes performed poorly and were excluded in determining the average gene expression shown in Figure 15B: ACDY7, CCND1, CREB5, GULP1, MPPE1, PDE3B, PTGER2, RGS2, and YPEL4. The following housekeeping genes were used for normalization in determining the average gene expression shown in Figure 15B: ACTB, ARPC2, GAPDG, HPRT1, LRIG2, and QARS. Gene expression profiles were obtained after the 120 minute incubation period.

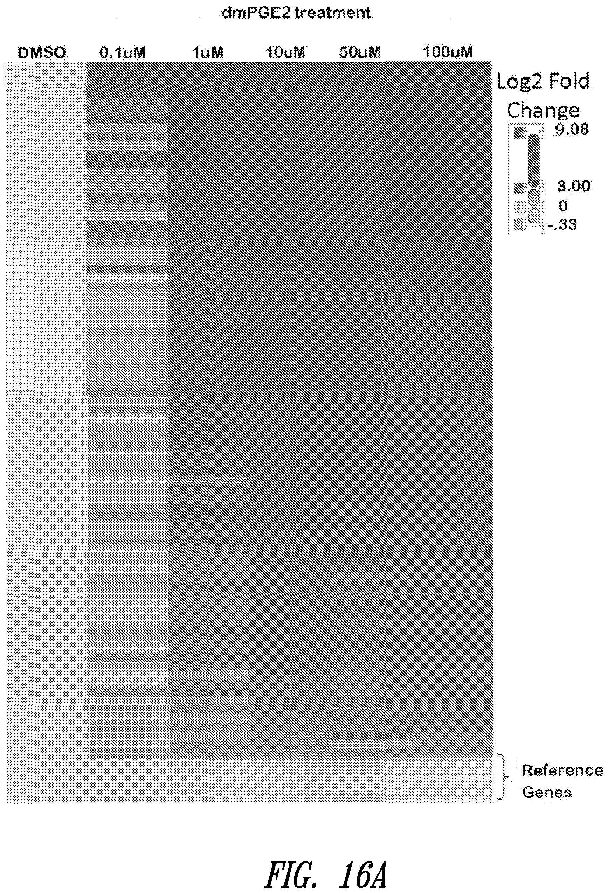

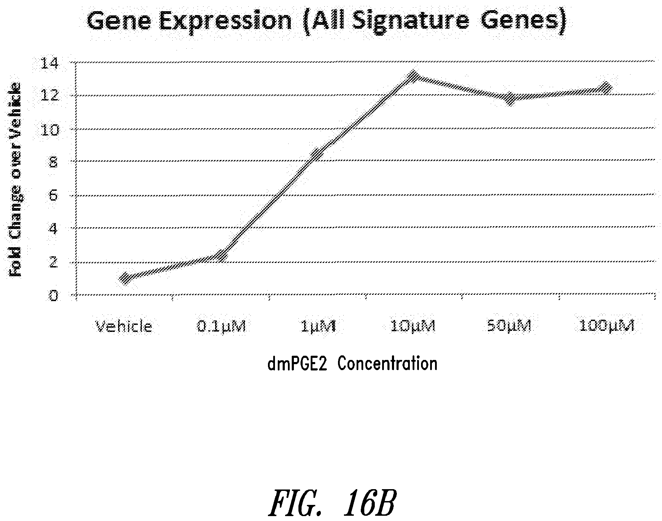

Figure 16 shows the effect of 16,16-dimethyl PGE₂ concentration or treatment with DMSO on gene expression using the Fluidigm gene expression platform. Figures 16A and 16B show the average gene expression of the signature genes listed in Table 3 in CD34⁺ cells treated with 0, 0.1, 1, 10, 50 and 100µM 16,16-dimethyl PGE₂ or DMSO for 120 minutes at 37°C compared to vehicle treated controls. The following genes performed poorly and were excluded in determining the average gene expression shown in Figure 16B: ACDY7, CCND1, CREB5, GULP1, FGFR1, FLJ27352, MPPE1, PDE4D, PTGER2, PDG3B, and YPEL4. The following housekeeping genes were used for normalization in determining the average gene expression shown in Figure 16B: ACTB, ARPC2, GAPDH, HPRT1, LRIG2, and QARS.

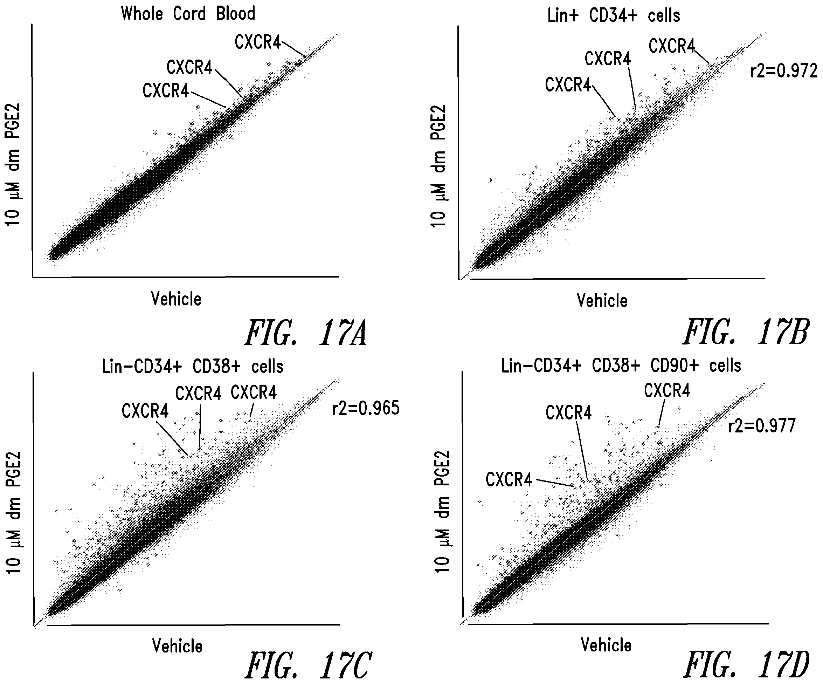

Figure 17 shows the gene expression analysis of cells treated with 10 µM 16,16-dimethyl PGE₂ at 37°C for 2 hours compared to DMSO treated cells. Figure 17A shows genome-wide expression analysis of whole cord blood cells treated with 10 µM 16,16-dimethyl PGE₂ at 37°C for 2 hours compared to cord blood cells treated with DMSO. Figure 17B shows genome-wide expression analysis of Lin+ CD34⁺ cells treated with 10 µM 16,16-dimethyl PGE₂ at 37°C for 2 hours compared to Lin(+) CD34⁺ cells treated with DMSO. Figure 17C shows genome-wide expression analysis of Lin(-) CD34⁺ CD38⁺ cells treated with 10 µM 16,16-dimethyl PGE₂ at 37°C for 2 hours compared to Lin(-) CD34⁺ CD38⁺ cells treated with DMSO. Figure 17D shows genome-wide expression analysis of Lin(-) CD34⁺ CD38⁻ CD90⁺ cells treated with 10 µM 16,16-dimethyl PGE₂ at 37°C for 2 hours compared to Lin(-) CD34⁺ CD38-CD90⁺ cells treated with DMSO.

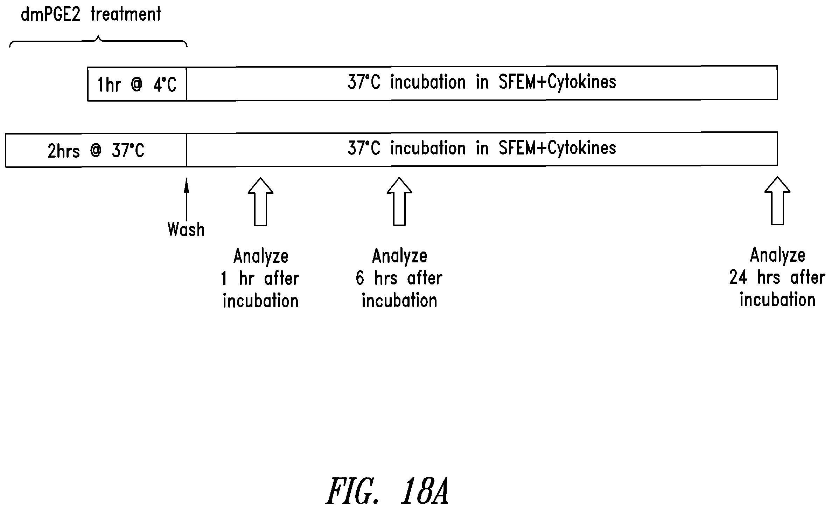

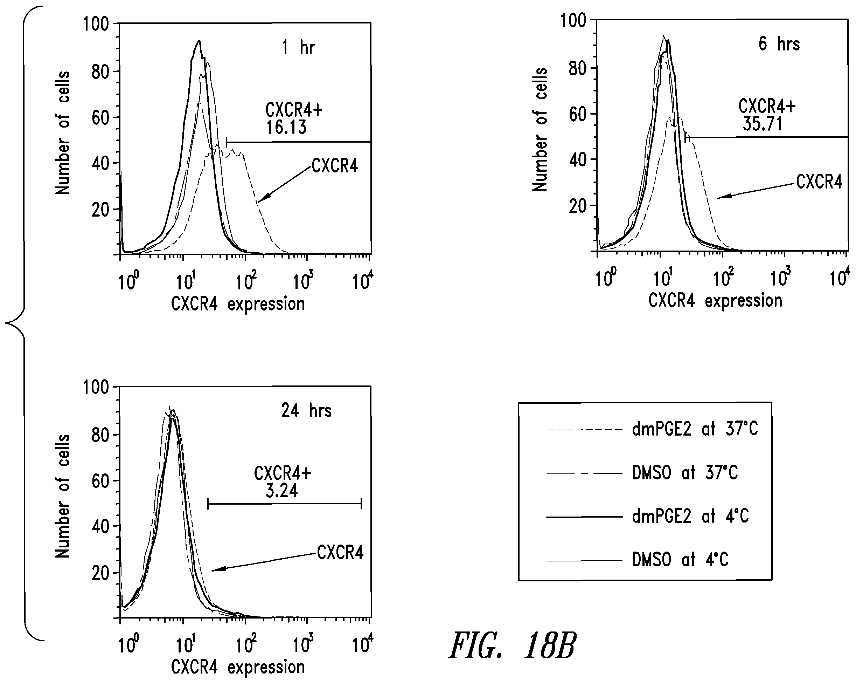

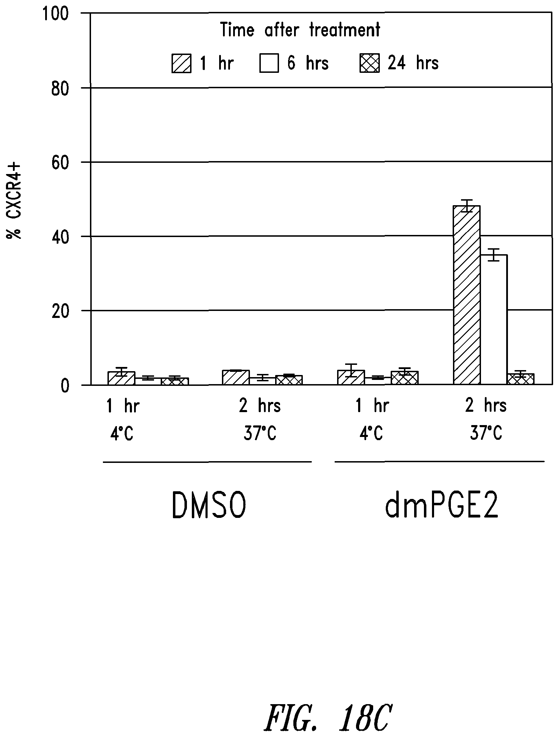

Figure 18 shows CXCR4 expression in cells treated with 10 µM 16,16-dimethyl PGE₂ treated at different temperatures for different lengths of time. Figure 18A shows the experimental conditions compared in this series of experiments. Figure 18B shows the CXCR4 cell-surface expression at 1, 6, and 24 hours post-treatment in cells treated with 10 µM 16,16-dimethyl PGE₂ or DMSO at 4°C for 1 hour and cells treated with 10 µM 16,16-dimethyl PGE₂ or DMSO at 37°C for 2 hours. Figure 18C shows the percentage CXCR4 expressing cells on the cell surface at 1, 6, and 24 hours post-treatment in cells treated with 10 µM 16,16-dimethyl PGE₂ or DMSO at 4°C for 1 hour and cells treated with 10 µM 16,16-dimethyl PGE₂ or DMSO at 37°C for 2 hours.
Figure 19 shows viability and proliferation analysis of CD34⁺ cells treated with 10 µM 16,16-dimethyl PGE₂ at the times and temperatures indicated. The treated cells were then analyzed using an *in vivo* CFU-S assay. Figure 19A shows that the 37°C incubation increases the number of hematopoietic progenitor cells.
Figure 19B shows cell viability data for human whole cord blood cells incubated with 16,16-dimethyl PGE₂ at various concentrations for 120 minutes at 4°C, 25°C, and 37°C. Figure 19C shows cell viability data for human CD34+ cells incubated with 16,16-dimethyl PGE₂ at various concentrations for 120 minutes at 4°C and 37°C. Figure 19D shows an increase in CFU-C colony formation in CD34⁺ cells treated with dmPGE₂ at 37°C compared to CD34⁺ cells treated with DMSO or with dmPGE₂ at 4°C.
Figure 20 shows an experimental flowchart for an *in vitro* chemotaxis functional assay. CD34⁺ cells are treated with 10 µM 16,16-dimethyl PGE₂ or DMSO control for 4 hours, and then transferred to a migration well assay for 4 hours in the presence of 0 - 50ng/ml SDF1α.
Figure 21 shows representative data for an *in vitro* chemotaxis functional assay. CD34⁺ cells are treated with 10 µM 16,16-dimethyl PGE₂ or DMSO control for 4 hours, and then transferred to a migration well assay for 4 hours in the presence of 0 - 50ng/ml SDF1α.
Figure 22A shows genome-wide expression analysis of CD34⁺ cells treated with 10 µM 16,16-dimethyl PGE₂ for 120 minutes at 4°C, 25°C, or 37°C (y-axis) versus vehicle treated cells (x-axis). Figure 22B provides the average fold changes for a subset of signature genes in the cells from the expression analysis illustrated in Figure 22A.

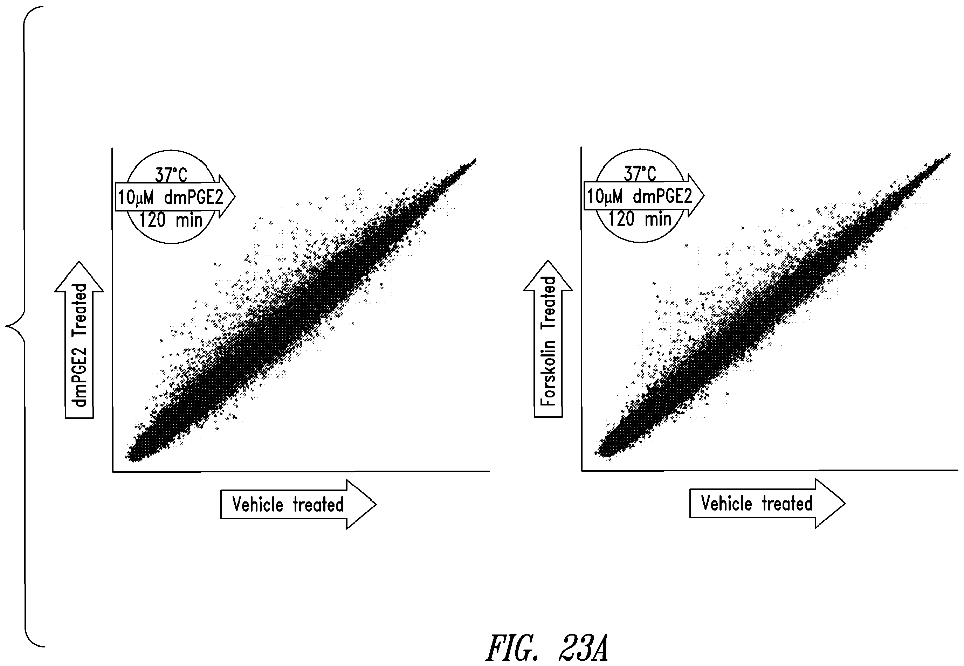

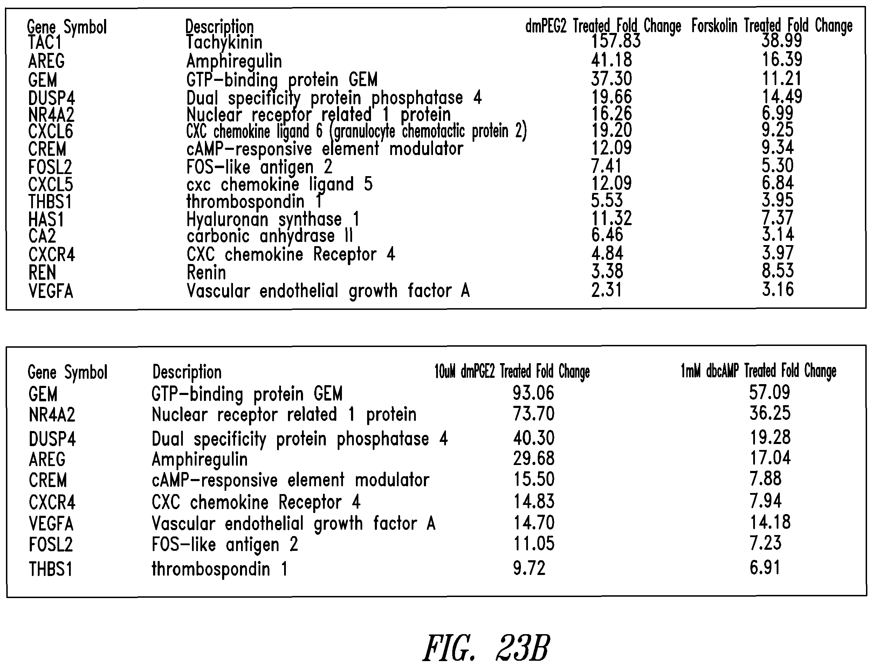

Figure 23A shows genome-wide expression analysis of CD34⁺ cells treated at 37°C with 10 µM dmPGE₂ or forskolin (y-axis) for 120 minutes versus vehicle treated cells (x-axis). Figure 23B (top panel) shows the average fold changes for a subset of signature genes illustrated in Figure 23A in the cells treated with dmPGE₂ or forskolin. Figure 23B (bottom panel) shows the average fold changes by Fluidigm qPCR for a subset of signature genes in CD34⁺ cells treated at 37°C with 1mM dbcAMP for 120 minutes or treated with dmPGE2 for 120 minutes.
Figure 24 shows an experimental strategy for performing hematopoietic cell transplants in mice using the therapeutic compositions of the invention.

### DETAILED DESCRIPTION

### A. Introduction

The invention provides therapeutic compositions and methods to improve the efficacy of hematopoietic stem or progenitor cell transplantation and addresses the multifaceted challenges faced by the medical profession in this field of regenerative cell therapy. The inventors analyzed several biological parameters of populations of hematopoietic stem and progenitor cells treated with agents that modify gene expression of the cells, including agents that stimulate the prostaglandin pathway and upregulate gene and cell-surface expression of CXCR4, in order to develop methods to increase the efficacy of hematopoietic stem and progenitor cells used in stem cell transplants. A cell population's effectiveness in reconstituting a subject's hematopoietic system upon transplantation depends on such properties as the cell population's ability to home to and engraft in the bone marrow, self-renew, and proliferate *in vivo.* The invention provides a method for modulating a cell population to improve such cell properties and provide resultant therapeutic improvements in hematopoietic reconstitution.

Specifically, the invention provides a therapeutic composition comprising an enhanced population of human hematopoietic stem or progenitor cells, and methods of making and using the enhanced therapeutic composition in stem cell transplants. The therapeutic composition of the invention comprises a population of human hematopoietic stem or progenitor cells that have been modified *ex vivo* to enhance the therapeutic properties of the cell population prior to use of the cell population in transplantation therapies. The modified cells of the therapeutic composition demonstrate increased ability to home to and engraft in the bone marrow, and additionally possess improved cell viability and self-renewal capabilities.

The therapeutic properties of the hematopoietic stem and progenitor cells of the therapeutic composition, including engraftment and homing ability of the cells, are increased by a method of treating the cell population *ex vivo* with an agent that modifies the expression of genes in the cell believed to be associated with cell homing and engraftment, including CXCR4. The method of the invention thus primes the cells comprising the therapeutic composition to achieve the most beneficial therapeutic effect upon transplantation of the cells. In the method of the invention, the hematopoietic stem or progenitor cells of the therapeutic composition are treated with the agent *ex vivo* at physiologically relevant temperatures, resulting in increased expression of genes associated with the beneficial biological properties of the cells, such as homing, engraftment, and in *vivo* expansion of the cell population. The therapeutic composition comprising the enhanced hematopoietic stem or progenitor cells is demonstrated in the examples described below to have advantages in homing, engraftment, and proliferation.

The therapeutic composition therefore provides a method of improving the engraftment potential of blood cells, including harvested blood cells and, for clarity, cord blood, and a method for increasing homing, viability, and self-renewal in transplanted hematopoietic cells. In addition, the present invention provides methods of *in vivo* hematopoietic stem and progenitor cell expansion. The therapeutic compositions and methods described in the instant invention may allow the use of a partial or single cord unit in cord blood transplantations.

Current standard of care for manipulating hematopoietic stem cells prior to transplantation requires strict temperature control at 4°C to maximize cell viability and successful engraftment upon transplantation. The invention demonstrates that stimulation of the prostaglandin cell signaling pathway in hematopoietic stem and progenitor cells under conditions believed to be associated with decreased cell viability and agent half-life (such as manipulation of cells at 37°C for a period of two hours) unexpectedly results in increased ability of cells to home to the bone marrow, increased self-renewal, and increased engraftment potential of the stem/progenitor cells, without negatively affecting cell viability. More particularly, the inventors discovered that prolonged exposure (of at least one hour) of hematopoietic stem and progenitor cells with a treatment agent that exerts PGE₂ activity at physiologically relevant temperatures, such as body temperature, is required to achieve a full biological effect. Notably, treatment of hematopoietic stem or progenitor cells for short durations of time at physiologically relevant temperatures results in increased cAMP production, but unexpectedly does not result in increased expression of genes believed to be associated with cell homing and engraftment. Longer cell treatment times at physiologically relevant temperature are required to achieve increased gene expression, and are demonstrated by the present invention to be necessary to achieve the desired biological effects of increased cell homing and engraftment. Without wishing to be bound to any particular theory, the methods described herein result in increased proliferation and engraftment potential of hematopoietic stem and progenitor cells upon administration to a subject.

Prostaglandin E₂ (PGE₂) exerts its function by acting on a number of different prostaglandin receptors on various cell types, activating various signaling pathways including, without limitation, the PI3-kinase (PI3-K or PI3K) pathway. These prostaglandin receptors represent a sub-family of the cell surface seven-transmembrane receptors referred to as G-protein-coupled receptors (GPCRs). There are four subtypes of prostaglandin E₂ receptors, PGE₂R1, PGE₂R₂, PGE₂R₃ and PGE₂R₄. When activated by a suitable ligand, or agonist, such as a prostaglandin or analogue thereof, e.g., a PGE₂R₂ or PGE₂R4 agonist, these prostaglandin receptors initiate a variety of downstream biological functions. For example, stimulation/activation of PGE₂R₂ and/or PGE₂R4 cell signaling in hematopoietic stem and progenitor cells is coupled, in part, to G-protein alpha-s (Gα-s or Gα-s) activation and stimulation of adenylate cyclase.

Activation of adenylyl cyclase catalyzes the conversion of ATP into cAMP. Increases in concentration of the second messenger cAMP can lead to the activation of cyclic nucleotide-gated ion channels, exchange proteins activated by cAMP such as RAPGEF3. Specificity of signaling between a GPCR and its ultimate molecular target through a cAMP dependent pathway may be achieved through formation of a multi protein complex, including the GPCR, adenylyl cyclase, and the effector protein.

As noted above, cyclic AMP activates protein kinase A (PKA, also known as cAMP-dependent protein kinase). PKA is normally inactive as a tetrameric holoenzyme, consisting of 2 catalytic and 2 regulatory units (C₂R₂), with the regulatory units blocking the catalytic centers of the catalytic units. Cyclic AMP binds to specific locations on the regulatory units of PKA, dissociates the regulatory and catalytic subunits, and thereby activates the catalytic units, enabling them to phosphorylate substrate proteins. Not all protein kinases respond to cAMP, as several types of protein kinases are not cAMP dependent, including, for example, protein kinase C.

The active subunits of PKA may catalyze the transfer of phosphate from ATP to specific serine or threonine residues of protein substrates. The phosphorylated protein kinases may act directly on ion channels in the cell, or may activate or inhibit other enzymes. PKA also phosphorylates specific proteins that bind to promoter regions of DNA, causing increased expression of specific genes. Further downstream effects depend on the various roles of PKA, which may differ based on the type of cell. For instance, activated PKA may phosphorylate a number of other proteins, including, for example, proteins that convert glycogen into glucose, proteins that promote muscle contraction in heart leading to an increase in heart rate, and transcription factors that regulate gene expression.

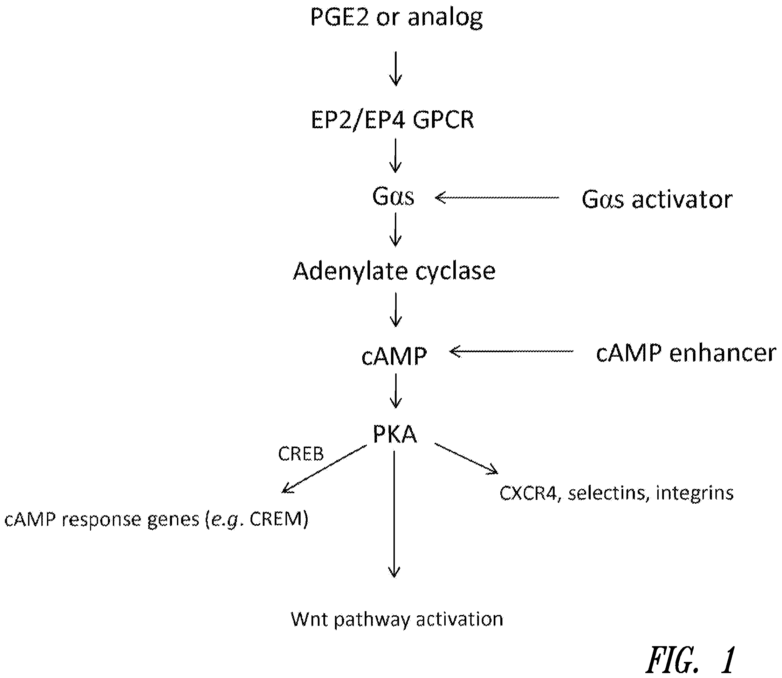

Thus, stimulation of PGE₂R₂ and PGE₂R4 cell signaling pathways may lead to increased activation of transcription factors such as cAMP response element binding protein (CREB) and CREB target genes, *e.g.,* cAMP response element modulator (CREM) (see Figure 1). Administration of hematopoietic stem and progenitor cells that have increased cAMP may maintain hematopoietic stem/progenitor cell viability, increase homing, increase self-renewal, and provide for increased engraftment and increased expansion of the transplanted cell population *in vivo.*

Stimulation/activation of PGE₂R₂ and PGE₂R4 cell signaling is also associated with increased phosphorylation of glycogen synthase kinase-3 (GSK-3) and increased B-catenin signaling (Hull *et al.,* 2004; Regan, 2003), both of which indicate activation of the Wnt pathway. PGE₂ stimulation of the Wnt pathway may actively enhance hematopoietic stem/progenitor proliferation, and self-renewal through signaling from the stem cell niche as well as within the cells themselves (North et al., 447(7147) Nature 1007-11 (2007)). Activation of the Wnt pathway in hematopoietic stem and progenitor cells may also lead to increased expansion of the population of cells *in vivo.*

PGE₂R4 stimulation has also been shown to activate the PI3K pathway, and may also be important to achieving the desired biological effects of increased stem cell homing, proliferation, survival, and engraftment. Stimulation/activation of PGE₂R₂ and PGE₂R4 cell signaling pathways, such as the PI3K pathway, may also increase expression of genes important for stem cell homing and engraftment, *e.g.,* CXC chemokine receptor 4 (CXCR4), selectins, integrins.

Before the current discovery, hematopoietic stem and progenitor cells were treated with compounds under the belief that the EP receptors could be fully saturated with the tested compound under conditions that maximized the viability of the cells and also the stability of the treatment compound. Treatment at 4°C, per current standard of care for hematopoietic stem cell transplants, was believed to provide improved cell viability of treated cells, as well as an expectation of increased half-life of the tested compounds at this temperature compared to higher temperatures, *e.g.,* 25°C or 37°C and longer incubation times, *e.g.,* two, three, four, or more hours.

Without wishing to be bound to any particular theory, the invention contemplates, in part, that engraftment of hematopoietic stem and progenitor cells, the ability of cells to home to the bone marrow, and the self-renewal of cells may be increased, and cell viability maintained, by treating the cells at increased temperatures for extended periods of incubation with agents that increase expression of genes associated with homing and engraftment.

Relevant agents include, for example, improved compositions of prostaglandin E₂ and agents having dmPGE₂ activity, including cAMP analogues and enhancers, and/or Gα-s activators. Moreover, the present invention demonstrates that administration of cells treated with such agents, including prostaglandin E₂ and agents having dmPGE₂ activity, at physiologically relevant temperatures (such as body temperature, or 37°C) for extended periods of incubation (*i.e.,* at least one hour) leads not only to increased cell engraftment but also results in an *in vivo* expansion of the hematopoietic stem and progenitor cell population.

Accordingly, in various embodiments, the invention provides a therapeutic composition comprising human hematopoietic stem or progenitor cells that have been contacted *ex vivo* at a temperature of about 37°C with an agent capable of increasing CXCR4 gene expression in the cells. The invention also provides methods of preparing hematopoietic stem and progenitor cells for use as a therapeutic composition for hematopoietic reconstitution comprising contacting a population of human hematopoietic stem and/or progenitor cells with an agent capable of increasing CXCR4 gene expression in the cells, such as an agent that stimulates the prostaglandin pathway, under conditions that optimize engraftment and expansion of the hematopoietic stem or progenitor cell population.

The articles "a," "an," and "the" are used herein to refer to one or to more than one (i.e. to at least one) of the grammatical object of the article. By way of example, "an element" means one element or more than one element.

The use of the alternative (e.g., "or") should be understood to mean either one, both, or any combination thereof of the alternatives. As used herein, the terms "include" and "comprise" are used synonymously.

As used herein, the term "about" or "approximately" refers to a quantity, level, value, number, frequency, percentage, dimension, size, amount, weight or length that varies by as much as 15%, 10%, 9%, 8%, 7%, 6%, 5%, 4%, 3%, 2% or 1% to a reference quantity, level, value, number, frequency, percentage, dimension, size, amount, weight or length. In one embodiment, the term "about" or "approximately" refers a range of quantity, level, value, number, frequency, percentage, dimension, size, amount, weight or length ± 15%, ± 10%, ± 9%, ± 8%, ± 7%, ± 6%, ± 5%, ± 4%, ± 3%, ± 2%, or ± 1% about a reference quantity, level, value, number, frequency, percentage, dimension, size, amount, weight or length.

Throughout this specification, unless the context requires otherwise, the words "comprise", "comprises" and "comprising" will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that no other elements are optional and may or may not be present depending upon whether or not they affect the activity or action of the listed elements

Reference throughout this specification to "one embodiment" or "an embodiment" means that a particular feature, structure or characteristic described in connection with the embodiment is included in at least one embodiment of the present invention. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

### B. Therapeutic Compositions of the Invention

The invention provides a therapeutic composition comprising a population of human hematopoietic stem or progenitor cells suspended in a sterile, therapeutically acceptable solution suitable for administration to a patient. The therapeutic composition of the invention comprises a population of human hematopoietic stem or progenitor cells wherein the hematopoietic stem or progenitor cells have been contacted *ex vivo* with one or more agents capable of increasing CXCR4 gene expression in the cells, and where the cells are characterized by a gene expression signature comprising increased expression, relative to non-contacted stem or progenitor cells, of CXCR4. The hematopoietic stem or progenitor cells may be characterized based upon increased levels of gene and cell-surface CXCR4 expression.

In the therapeutic composition of the invention, gene expression of CXCR4 in the hematopoietic stem or progenitor cells is increased by at least 2, 3, 4, 5, 10, 15, or 20 fold compared to the expression of CXCR4 in non-contacted cells.

The therapeutic composition of the invention may be further characterized by a gene expression signature wherein expression of one or more signature genes selected from the group consisting of hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), and Fos-related antigen 2 (FOSL2) is increased, relative to non-contacted cells.

As used herein, a "non-contacted" cell is a cell that has not been treated, e.g., cultured, contacted, or incubated with an agent other than a control agent. Cells contacted with DMSO (a control agent), or contacted with another vehicle are non-contacted cells.

A "signature gene", as used herein, means any gene in the signature gene set provided in Table 3. For example, signature genes include hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), Fos-related antigen 2 (FOSL2), and CXC chemokine receptor 4 (CXCR4). For clarity, signature genes do not include housekeeping genes.

Expression of a signature gene may be increased by 2 or more fold compared to non-contacted cells, and in particular embodiments is increased by at least 2, 3, 4, 5, 6, 10, 15, or 20 fold. In some embodiments, expression of one or more signature genes is increased in cells comprising the therapeutic composition of the invention. In particular embodiments, expression of at least 2, 3, 4, or more of the signature genes is increased by at least 2, 3, 4, 5, 6, 10, 15, or 20 fold compared to non-contacted cells. In various embodiments, expression of a signature gene may be increased by at least 6 fold compared to non-contacted cells.

In particular embodiments of the invention, the gene expression of CXCR4 is increased by at least about 4 fold and the gene expression of CREM is increased by at least about 10 fold.

The human hematopoietic stem or progenitor cells comprising the therapeutic composition may also be characterized by a gene expression profile wherein the average fold change of all signature genes is at least about 2, 4, or 6 fold. In some embodiments, the average fold change of all signature genes is at least about 4. In some embodiments, the average fold change of all signature genes is at least about 6. In some embodiments, the average fold change of at least 40%, 50%, 60%, 70%, 75%, 80%, 85%, or 90% of the signature genes is at least 6 fold. In some embodiments, the average fold change of at least 40%, 50%, 60%, 70%, 75%, 80%, 85%, or 90% of the signature genes is at least 3, 4, 5, 6, 7, 8, 9 or 10 fold. In particular embodiments, the therapeutic composition may be characterized by a gene expression profile having the average fold change for all signature genes as depicted in Figure 14(B), Figure 15(B), or Figure 16(B).

The gene expression signature of the human hematopoietic stem or progenitor cells comprising the therapeutic composition may be analyzed, *i.e.,* obtained, after cells are treated with an agent, or cells may be incubated for some period of time after treatment before analyzing the gene expression signature of the cells. For example, cells may be treated *ex vivo* with an agent, washed to remove the agent, and the gene expression analyzed without further incubation of the cells. Alternatively, in some embodiments cells are treated with an agent, washed to remove the agent from the cell population, and then the cells are incubated *ex vivo* for some period of time prior to analyzing the gene expression signature of the cells.

In some embodiments, cells are washed to remove agent and then incubated for one to six hours before the gene expression signature of the cells is analyzed. In some embodiments, cells are washed and then incubated for at least about an hour before the gene expression signature of the cells is analyzed. In some embodiments, cells are washed and then incubated for about two hours before the gene expression signature of the cells is analyzed.

"Gene expression" as used herein refers to the relative levels of expression and/or pattern of expression of a gene in a biological sample, such as the hematopoietic stem and progenitor cells, or population of hematopoietic stem or progenitor cells, in a therapeutic composition of the invention. The expression of a gene may be measured at the level of cDNA, RNA, mRNA, or combinations thereof. "Gene expression profile" or "gene expression signature" refers to the levels of expression of multiple different genes measured for the same sample, *i.e.,* a population of cells.

Any methods available in the art for detecting expression of the genes characterizing the cells comprising the therapeutic composition of the invention are encompassed herein. As used herein, the term "detecting expression" means determining the quantity or presence of an RNA transcript or its expression product of a gene. Methods for detecting expression of genes, that is, gene expression profiling, include methods based on hybridization analysis of polynucleotides, methods based on sequencing of polynucleotides, immunohistochemistry methods, and proteomics-based methods. The methods generally detect expression products *(e.g.,* mRNA) of the genes of interest. In some embodiments, PCR-based methods, such as reverse transcription PCR (RT-PCR) (Weis et al., TIG 8:263-64, 1992), and array-based methods such as microarray (Schena et al., Science 270:467-70, 1995) are used. By "microarray" is intended an ordered arrangement of hybridizable array elements, such as, for example, polynucleotide probes, on a substrate. The term "probe" refers to any molecule that is capable of selectively binding to a specifically intended target biomolecule, for example, a nucleotide transcript or a protein encoded by or corresponding to an intrinsic gene. Probes can be synthesized by one of skill in the art, or derived from appropriate biological preparations. Probes may be specifically designed to be labeled. Examples of molecules that can be utilized as probes include, but are not limited to, RNA, DNA, aptamers, proteins, antibodies, and organic molecules

General methods for RNA extraction are well known in the art and are disclosed in standard textbooks of molecular biology, including Ausubel et al., ed., Current Protocols in Molecular Biology, John Wiley & Sons, New York 1987-1999. Methods for RNA extraction from paraffin embedded tissues are disclosed, for example, in Rupp and Locker (Lab Invest. 56:A67, 1987) and De Andres et al. (Biotechniques 18:42-44, 1995). In particular, RNA isolation can be performed using a purification kit, a buffer set and protease from commercial manufacturers, such as Qiagen (Valencia, Calif.), according to the manufacturer's instructions. For example, total RNA from cells in culture can be isolated using Qiagen RNeasy mini-columns. Other commercially available RNA isolation kits include MASTERPURE. Complete DNA and RNA Purification Kit (Epicentre, Madison, Wis.) and Paraffin Block RNA Isolation Kit (Ambion, Austin, Tex.). Total RNA from tissue samples can be isolated, for example, using RNA Stat-60 (Tel-Test, Friendswood, Tex.). Additionally, large numbers of tissue samples can readily be processed using techniques well known to those of skill in the art, such as, for example, the single-step RNA isolation process of Chomczynski (U.S. Pat. No. 4,843,155).

Isolated RNA can be used in hybridization or amplification assays that include, but are not limited to, PCR analyses and probe arrays. One method for the detection of RNA levels involves contacting the isolated RNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 60, 100, 250, or 500 nucleotides in length and sufficient to specifically hybridize under stringent conditions to an intrinsic gene of the present invention, or any derivative DNA or RNA. Hybridization of an mRNA with the probe indicates that the intrinsic gene in question is being expressed.

In one embodiment, the mRNA is immobilized on a solid surface and contacted with a probe, for example by running the isolated mRNA on an agarose gel and transferring the mRNA from the gel to a membrane, such as nitrocellulose. In an alternative embodiment, the probes are immobilized on a solid surface and the mRNA is contacted with the probes, for example, in an Agilent gene chip array. A skilled artisan can readily adapt known mRNA detection methods for use in detecting the level of expression of the intrinsic genes of the present invention.

An alternative method for determining the level of gene expression in a sample involves the process of nucleic acid amplification, for example, by RT-PCR (U.S. Pat. No. 4,683,202), ligase chain reaction (Barany, Proc. Natl. Acad. Sci. USA 88:189-93, 1991), self sustained sequence replication (Guatelli et al., Proc. Natl. Acad. Sci. USA 87:1874-78, 1990), transcriptional amplification system (Kwoh et al., Proc. Natl. Acad. Sci. USA 86:1173-77, 1989), Q-Beta Replicase (Lizardi et al., Bio/Technology 6:1197, 1988), rolling circle replication (U.S. Pat. No. 5,854,033), or any other nucleic acid amplification method, followed by the detection of the amplified molecules using techniques well known to those of skill in the art. These detection schemes are especially useful for the detection of nucleic acid molecules if such molecules are present in very low numbers.

In particular aspects of the invention, gene expression is assessed by quantitative RT-PCR. Numerous different PCR or QPCR protocols are known in the art and exemplified herein below and can be directly applied or adapted for use using the presently-described compositions for the detection and/or quantification of the genes listed in Table 3. Generally, in PCR, a target polynucleotide sequence is amplified by reaction with at least one oligonucleotide primer or pair of oligonucleotide primers. The primer(s) hybridize to a complementary region of the target nucleic acid and a DNA polymerase extends the primer(s) to amplify the target sequence. Under conditions sufficient to provide polymerase-based nucleic acid amplification products, a nucleic acid fragment of one size dominates the reaction products (the target polynucleotide sequence which is the amplification product). The amplification cycle is repeated to increase the concentration of the single target polynucleotide sequence. The reaction can be performed in any thermocycler commonly used for PCR. However, preferred are cyclers with real-time fluorescence measurement capabilities, for example, SMARTCYCLER (Cepheid, Sunnyvale, Calif.), ABI PRISM 7700. (Applied Biosystems, Foster City, Calif.), ROTOR-GENE (Corbett Research, Sydney, Australia), LIGHTCYCLER (Roche Diagnostics Corp, Indianapolis, Ind.), ICYCLER (Biorad Laboratories, Hercules, Calif.) and MX4000 (Stratagene, La Jolla, Calif.).

Quantitative PCR (QPCR) (also referred as real-time PCR) is preferred under some circumstances because it provides not only a quantitative measurement, but also reduced time and contamination. In some instances, the availability of full gene expression profiling techniques is limited due to requirements for fresh frozen tissue and specialized laboratory equipment, making the routine use of such technologies difficult in a clinical setting. As used herein, "quantitative PCR (or "real time QPCR") refers to the direct monitoring of the progress of PCR amplification as it is occurring without the need for repeated sampling of the reaction products. In quantitative PCR, the reaction products may be monitored via a signaling mechanism (e.g., fluorescence) as they are generated and are tracked after the signal rises above a background level but before the reaction reaches a plateau. The number of cycles required to achieve a detectable or "threshold" level of fluorescence varies directly with the concentration of amplifiable targets at the beginning of the PCR process, enabling a measure of signal intensity to provide a measure of the amount of target nucleic acid in a sample in real time.

In another embodiment of the invention, microarrays are used for expression profiling. Microarrays are particularly well suited for this purpose because of the reproducibility between different experiments. DNA microarrays provide one method for the simultaneous measurement of the expression levels of large numbers of genes. Each array consists of a reproducible pattern of capture probes attached to a solid support. Labeled RNA or DNA is hybridized to complementary probes on the array and then detected by laser scanning. Hybridization intensities for each probe on the array are determined and converted to a quantitative value representing relative gene expression levels. See, for example, U.S. Pat. Nos. 6,040,138, 5,800,992 and 6,020,135, 6,033,860, and 6,344,316. High-density oligonucleotide arrays are particularly useful for determining the gene expression profile for a large number of RNAs in a sample.

Microarray analysis can be performed by commercially available equipment, following manufacturer's protocols, such as by using the Affymetrix GenChip technology, Illumina Bead Array technology, or Agilent ink jet microarray technology.

"Normalization" may be used to remove sample-to-sample variation. For microarray data, the process of normalization aims to remove systematic errors by balancing the fluorescence intensities of the two labeling dyes. The dye bias can come from various sources including differences in dye labeling efficiencies, heat and light sensitivities, as well as scanner settings for scanning two channels. Some commonly used methods for calculating normalization factor include: (i) global normalization that uses all genes on the array, such as by log scale robust multi-array analysis (RMA); (ii) housekeeping genes normalization that uses constantly expressed housekeeping/invariant genes; and (iii) internal controls normalization that uses known amount of exogenous control genes added during hybridization (Quackenbush (2002) Nat. Genet. 32 (Suppl.), 496-501). In one embodiment, expression of the genes disclosed herein can be normalized to control housekeeping genes or by log scale robust multi-array analysis (RMA).

In various illustrative embodiments, the present invention provides, in part, a therapeutic composition comprising a population of cells for use in a transplant, for example, a bone marrow transplant. As used herein, the terms "population of cells" refers to a heterogeneous or homogenous population of cells comprising hematopoietic stem and/or progenitor cells. The population of cells comprising hematopoietic stem and/or progenitor cells may be bone marrow cells, umbilical cord blood cells, or mobilized peripheral blood cells, or a population of cells obtained from any suitable source, including bone marrow, mobilized peripheral blood, and umbilical cord blood among others. The term "collection of cells" also refers to a population of cells, and in some embodiments is synonymous with "population of cells." However, a collection of cells need not refer to the any particular population of cells.

Hematopoietic stem and/or progenitor cells, whether obtained from cord blood, bone marrow, peripheral blood, or other source, may be grown, treated or expanded in any suitable, commercially available or custom defined medium, with or without serum, as desired (see, *e.g.,* Hartshorn et al., Cell Technology for Cell Products, pages 221-224, R. Smith, Editor; Springer Netherlands, 2007, herein incorporated by reference in its entirety). For instance, in certain embodiments, serum free medium may utilize albumin and/or transferrin, which have been shown to be useful for the growth and expansion of CD34⁺ cells in serum free medium. Also, cytokines may be included, such as Flt-3 ligand, stem cell factor (SCF), and thrombopoietin (TPO), among others. HSCs may also be grown in vessels such as bioreactors *(see, e.g.,* Liu et al., Journal of Biotechnology 124:592-601, 2006, herein incorporated by reference in its entirety). A suitable medium for *ex vivo* expansion of HSCs may also comprise HSC supporting cells, such as stromal cells *(e.g.,* lymphoreticular stromal cells), which can be derived, for instance, from the disaggregation of lymphoid tissue, and which have been show to support the *in vitro, ex vivo,* and *in vivo* maintenance, growth, and differentiation of HSCs, as well as their progeny.

In particular embodiments, the population of cells is not expanded *ex vivo* or *in vitro* prior to administration to a subject. In particular embodiments, an unexpanded population of cells is obtained, the population of cells is treated *ex vivo* in accordance with the protocol provided herein, may be washed to remove the treatment agent, and administered to a patient without expansion of the cell population *ex vivo.* In some embodiments, cells are obtained from a donor, including cord blood, and are not expanded prior to or after treatment of the cells, or at any time prior to administration of the therapeutic composition to a patient. In one embodiment, an unexpanded population of cells is treated and is administered to a patient prior to any substantial *ex vivo* cell division of the cells in the population, or prior to the time required for any substantial cell division *ex vivo.* In other embodiments, an unexpanded population of cells is treated and is administered to a patient prior to any substantial *ex vivo* mitosis of the cells in the population, or prior to the time required for any substantial mitosis *ex vivo.* In some embodiments, an unexpanded population of cells is treated and is administered to a patient prior to the doubling time of the cells in the population. In some embodiments, an unexpanded population of cells is treated and is administered to a patient within 6, 12, or 24 hours of treatment of the cells. In other embodiments, an unexpanded population of cells is treated and is administered to a patient within 2 hours of treatment of the cells.

In various embodiments, the population of cells is not cultured prior to treatment with an agent *ex vivo* or at any time prior to administration to a patient. In some embodiments, the population of cells is cultured for less than about 24 hours. In other embodiments, the population of cells is cultured for less than about 12 hours, 10 hours, 8 hours, 6 hours, 4 hours, or two hours.

In various embodiments, the population of cells that is treated with an agent as described elsewhere herein and subsequently administered to a subject is a heterogeneous population of cells including, whole bone marrow, umbilical cord blood, mobilized peripheral blood, hematopoietic stem cells, hematopoietic progenitor cells, and the progeny of hematopoietic stem and progenitor cells, including granulocytes (*e.g.,* promyelocytes, myelocytes, metamyelocytes, neutrophils, eosinophils, basophils), erythrocytes (*e.g.,* reticulocytes, erythrocytes), thrombocytes (*e.g.,* megakaryoblasts, platelet producing megakaryocytes, platelets), and monocytes (e.g., monocytes, macrophages).

In one embodiment, the therapeutic composition comprises a cell population that is about 100% hematopoietic stem and progenitor cells. In some embodiments, the population of cells in the therapeutic composition is less than about 0.1%, 0.5%, 1%, 2%, 5%, 10%, 15%, 20%, 25%, or 30% hematopoietic stem and progenitor cells. The population of cells in some embodiments is less than about 0.1%, 0.5%, 1%, 2%, 5%, 10%, 15%, 20%, 25%, or 30% CD34⁺ cells. In other embodiments, the population of cells is about 0.1% to about 1%, about 1% to about 3%, about 3% to about 5%, about 10%- about 15%, about 15%-20%, about 20%-25%, about 25%-30%, about 30%-35%, about 35%-40%, about 40%-45%, about 45%-50%, about 60%-70%, about 70%-80%, about 80%-90%, about 90%-95%, or about 95% to about 100% hematopoietic stem and progenitor cells. In particular embodiments, the population of cells is about 0.1% to about 1%, about 1% to about 3%, about 3% to about 5%, about 10%- about 15%, about 15%-20%, about 20%-25%, about 25%-30%, about 30%-35%, about 35%-40%, about 40%-45%, about 45%-50%, about 60%-70%, about 70%-80%, about 80%-90%, about 90%-95%, or about 95% to about 100% CD34⁺ cells.

Cells in the therapeutic composition of the invention can be autologous/autogeneic ("self") or non-autologous ("non-self," *e.g.,* allogeneic, syngeneic or xenogeneic). "Autologous," as used herein, refers to cells from the same subject. "Allogeneic," as used herein, refers to cells of the same species that differ genetically to the cell in comparison. "Syngeneic," as used herein, refers to cells of a different subject that are genetically identical to the cell in comparison. "Xenogeneic," as used herein, refers to cells of a different species to the cell in comparison. In particular embodiments, the cells of the invention are allogeneic.

A "stem cell" refers to a cell which is an undifferentiated cell capable of (1) long term self -renewal, or the ability to generate at least one identical copy of the original cell, (2) differentiation at the single cell level into multiple, and in some instance only one, specialized cell type and (3) of *in vivo* functional regeneration of tissues. Stem cells are subclassified according to their developmental potential as totipotent, pluripotent, multipotent and oligo/unipotent. A "progenitor cell" also has the capacity to self-renew and to differentiate into more mature cells, but is committed to a lineage (e.g., hematopoietic progenitors are committed to the blood lineage; myeloid progenitors are committed to the myeloid lineage; lymphoid progenitors are committed to the lymphoid lineage), whereas stem cells are not necessarily so limited. "Self-renewal" refers a cell with a unique capacity to produce unaltered daughter cells and therefore replenish and maintain its population numbers, and to generate specialized cell types (potency). Self-renewal can be achieved in two ways. Asymmetric cell division produces one daughter cell that is identical to the parental cell and one daughter cell that is different from the parental cell and is a more committed progenitor or differentiated cell. Symmetric cell division produces two identical daughter cells. "Proliferation" or "expansion" of cells refers to symmetrically dividing cells.

Hematopoietic stem cells (HSCs) give rise to committed hematopoietic progenitor cells (HPCs) that are capable of generating the entire repertoire of mature blood cells over the lifetime of an organism. The term "hematopoietic stem cell" or "HSC" refers to multipotent stem cells that give rise to all the blood cell types of an organism, including myeloid (e.g., monocytes and macrophages, neutrophils, basophils, eosinophils, erythrocytes, megakaryocytes/platelets, dendritic cells), and lymphoid lineages (e.g., T-cells, B-cells, NK-cells), and others known in the art (*See* Fei, R., et al., U.S. Patent No. 5,635,387; McGlave, et al., U.S. Patent No. 5,460,964; Simmons, P., et al, U.S. Patent No. 5,677,136; Tsukamoto, et al., U.S. Patent No. 5,750,397; Schwartz, et al., U.S. Patent No. 5,759,793; DiGuisto, et al., U.S. Patent No. 5,681,599; Tsukamoto, et al., U.S. Patent No. 5,716,827). When transplanted into lethally irradiated animals or humans, hematopoietic stem cells can repopulate the erythroid, neutrophil-macrophage, megakaryocyte and lymphoid hematopoietic cell pool.

HSCs may be identified according to certain phenotypic or genotypic markers. For example, HSCs may be identified by their small size, lack of lineage (lin) markers, low staining (side population) with vital dyes such as rhodamine 123 (rhodamine^{DULL}, also called rho^{lo}) or Hoechst 33342, and presence of various antigenic markers on their surface, many of which belong to the cluster of differentiation series (*e.g.,* CD34, CD38, CD90, CD133, CD105, CD45, and c-kit, the receptor for stem cell factor). HSCs are mainly negative for the markers that are typically used to detect lineage commitment, and, thus, are often referred to as Lin(-) cells. Most human HSCs may be characterized as CD34⁺, CD59⁺, Thy1/CD90\CD38^{lo/-}, C-kit/CD117⁺, and Lin(-). However, not all stem cells are covered by these combinations, as certain HSCs are CD34⁻/CD38⁻. Also some studies suggest that earliest stem cells may lack c-kit on the cell surface. For human HSCs, CD133 may represent an early marker, as both CD34⁺ and CD34- HSCs have been shown to be CD133⁺. It is known in the art that CD34⁺ and Lin(-) cells also include hematopoietic progenitor cells.

Suitable sources of hematopoietic stem and progenitor cells for use in the methods of the present invention include, but are not limited to, cells isolated or obtained from an organ of the body containing cells of hematopoietic origin. By "isolated" is meant material that is removed from its original environment. For example, a cell is isolated if it is separated from some or all of the components that normally accompany it in its native state. For example, an "isolated population of cells," an "isolated source of cells," or "isolated hematopoietic stem and progenitor cells and the like, as used herein, refer to *in vitro* or *ex vivo* separation of one or more cells from their natural cellular environment, and from association with other components of the tissue or organ, *i.e.,* it is not significantly associated with *in vivo* substances.

Hematopoietic stem and progenitor cells for use in the methods of the present invention may be depleted of mature hematopoietic cells such as T cells, B cells, NK cells, dendritic cells, monocytes, granulocytes, erythroid cells, and their committed precursors from bone marrow aspirate, umbilical cord blood, or mobilized peripheral blood (mobilized leukapheresis product). Mature, lineage committed cells are depleted by immunodepletion, for example, by labeling solid substrates with antibodies that bind to a panel of so-called "lineage" antigens: CD2, CD3, CD11b, CD14, CD15, CD16, CD19, CD56, CD123, and CD235a. A subsequent step can be performed to further purify the population of cells, in which a substrate labeled with antibodies that bind to the CD34⁺ antigen are used to isolate primitive hematopoietic stem and progenitor cells. Kits are commercially available for purifying hematopoietic stem and progenitor cells from various cell sources and in particular embodiments, these kits are suitable for use with the methods of the present invention. Exemplary commercially available kits for purifying hematopoietic stem and progenitor cells include, but are not limited to Lineage (Lin) Depletion Kit (Miltenyi Biotec); CD34⁺ enrichment kit (Miltenyi Biotec); RosettaSep (Stem Cell Technologies).

The population of cells comprising the therapeutic composition of the invention, in some embodiments, comprises less than about 30%, 25%, 20%, 15%, 10% or 5% mesenchymal stem cells. In particular embodiments, the population of cells comprises no more than about 10% mesenchymal stem cells. Mesenchymal stem cells (MSCs) are multipotent stem cells that can differentiate readily into lineages including osteoblasts, myocytes, chondrocytes, and adipocytes (Pittenger, et al., Science, Vol. 284, pg. 143 (1999); Haynesworth, et al., Bone, Vol. 13, pg. 69 (1992); Prockop, Science, Vol. 276, pg. 71 (1997)).

In other embodiments, the population of cells comprising the therapeutic composition of the invention comprises less than about 30%, 25%, 20%, 15%, 10% or 5% endothelial progenitor cells. In other embodiments, the population of cells comprises less than about 10% endothelial progenitor cells. As used herein, "endothelial progenitor cell" refers to a multipotent or unipotent cell with the potential to differentiate into vascular endothelial cells.

In more particular embodiments, the population of cells comprises no more than about 10% mesenchymal stem cells or endothelial progenitor cells.

The population of cells as obtained from a donor, or as otherwise provided, may be substantially free of mesenchymal stem cells and/or endothelial progenitor cells, and in particular embodiments comprise less than about 10% mesenchymal stem cells and less than about 10% endothelial progenitor cells. The population of cells may alternatively be depleted of mesenchymal stem cells and/or endothelial progenitor cells using methods known in the art, for example, using immunomagnetic selection techniques, fluorescence activated cell sorting, or a combination therein. The depletion methods can further comprise the use of at least one antibody specific for at least one of the cell-surface markers described herein.

In some embodiments, the population of cells is depleted of endothelial progenitor cells, including cells positive for the CD14 cell surface marker and negative for CD45 (CD14+/CD45-) and/or cells positive for VWF (Von Willebrand Factor) (VWF+). In other embodiments, the cell population is depleted of cells positive for CD73 and/or CD140B cell surface markers. In particular embodiments of the invention, the population of cells comprises cells positive for the cell surface marker CD34, and comprises less than about 30%, 25%, 20%, 15%, 10% or 5% of cells positive for a cell surface marker selected from the group consisting of CD73, CD140B, CD14 and VWF.

In particular embodiments, the population of cells comprising the therapeutic composition of the invention comprises CD34⁺ cells and comprises less than about 30%, 25%, 20%, 15%, 10% or 5% CD14⁺/CD45⁻ cells. In other embodiments of the invention, the population of cells comprises CD34⁺ cells and comprises less than about 30%, 25%, 20%, 15%, 10% or 5% VWF⁺ cells. In other embodiments of the invention, the population of cells comprises CD34⁺ cells and comprises less than about 30%, 25%, 20%, 15%, 10% or 5% CD140B⁺ cells.

In more particular embodiments, the population of cells comprises CD34⁺ hematopoietic stem or progenitor cells and comprises less than about 30%, 25%, 20%, 15%, 10% or 5% of CD14⁺/CD45⁻ cells, VWF⁺ cells, CD73⁺ cells, and CD140B⁺ cells. In some embodiments, the population of cells is positive for the cell surface marker CD34 and is negative for at least one cell surface marker from the group consisting of CD14, VWF, CD73, and CD140B. In other embodiments, the population of cells is positive for the cell surface marker CD34 and is negative for the cell surface markers CD14, VWF, CD73, and CD140B.

Hematopoietic stem and progenitor cells can be obtained or isolated from unfractionated or fractioned bone marrow of adults, which includes femurs, hip, ribs, sternum, and other bones. Hematopoietic stem and progenitor cells can be obtained or isolated directly by removal from the hip using a needle and syringe, or from the blood, often following pre-treatment with cytokines, such as G-CSF (granulocyte colony-stimulating factors), that induce cells to be released or mobilized from the bone marrow compartment. Other sources of hematopoietic stem and progenitor cells include umbilical cord blood, placental blood, and mobilized peripheral blood. For experimental purposes, fetal liver, fetal spleen, kidney marrow, and AGM (Aorta-gonad-mesonephros) of animals are also useful sources of hematopoietic stem and progenitor cells.

In particular embodiments, the hematopoietic stem or progenitor cells are harvested from a hematopoietic source, e.g., bone marrow cells, umbilical cord blood, or mobilized peripheral blood cells. "Harvesting" hematopoietic stem and progenitor cells is defined as the dislodging or separation of cells from the matrix. This can be accomplished using a number of methods, such as enzymatic, non-enzymatic, centrifugal, electrical, or size-based methods, or preferably, by flushing the cells using media (e.g. media in which the cells are incubated). In particular embodiments, harvesting a sufficient quantity of cells for transplantation may require mobilizing the stem and progenitor cells in the donor.

"Hematopoietic stem cell mobilization" refers to the release of stem cells from the bone marrow into the peripheral blood circulation for the purpose of leukapheresis, prior to stem cell transplantation. Hematopoietic growth factors, e.g., granulocyte colony stimulating factor (G-CSF) or chemotherapeutic agents often are used to stimulate the mobilization. Commercial stem cell mobilization drugs exist and can be used in combination with G-CSF to mobilize sufficient quantities of hematopoietic stem and progenitor cells for transplantation into a subject. For example, G-CSF and Mozobil™ (Genzyme Corporation) can be administered to a donor in order to harvest a sufficient number of hematopoietic cells for transplantation.

By increasing the number of stem cells harvested from the donor, the number of stem cells available for transplantation back into a subject the outcome of the subject can be significantly improved, thereby potentially reducing the time to engraftment, and consequently leading to a decrease in the time during which the subject has insufficient neutrophils and platelets, thus preventing infections, bleeding, or other complications. Other methods of mobilizing hematopoietic stem and progenitor cells would be apparent to one having skill in the art.

In particular embodiments, hematopoietic stem or progenitor cells are obtained from umbilical cord blood. Cord blood can be harvested according to techniques known in the art (*see, e.g.,* U.S. Patent Nos. 7,147,626 and 7,131,958, herein incorporated by reference for such methodologies).

In one embodiment, hematopoietic stem and progenitor cells for use in the therapeutic compositions and methods of the invention can be obtained from pluripotent stem cell sources, e.g., induced pluripotent stem cells (iPSCs) and embryonic stem cells (ESCs). As used herein, the term "induced pluripotent stem cell" or "iPSC" refers to a non-pluripotent cell that has been reprogrammed to a pluripotent state. Once the cells of a subject have been reprogrammed to a pluripotent state, the cells can then be programmed to a desired cell type, such as a hematopoietic stem or progenitor cell. As used herein, the terms "reprogramming" refers to a method of increasing the potency of a cell to a less differentiated state. As used herein, the term "programming" refers to a method of decreasing the potency of a cell or differentiating the cell to a more differentiated state.

In various embodiments, the invention contemplates administration of the therapeutic composition to a human patient, or a subject in need of therapy. The amount of hematopoietic stem or progenitor cells contained in the therapeutic composition and administered to a patient will vary with the source of the cells, disease state, age, sex, and weight of the individual, and the ability of the hematopoietic stem and progenitor cells to elicit a desired response in the individual.

In one embodiment, the amount of hematopoietic stem or progenitor cells (e.g., CD34⁺, Lin(-) cells) in the therapeutic composition administered to a subject is the amount of hematopoietic stem or progenitor cells in a partial or single cord of blood, or at least 0.1 x 10⁵ cells, at least 0.5 x 10⁵ cells, at least 1 x 10⁵ cells, at least 5 x 10⁵ cells, at least 10 x 10⁵ cells, at least 0.5 x 10⁶ cells, at least 0.75 x 10⁶ cells, at least 1 x 10⁶ cells, at least 1.25 x 10⁶ cells, at least 1.5 x 10⁶ cells, at least 1.75 x 10⁶ cells, at least 2 x 10⁶ cells, at least 2.5 x 10⁶ cells, at least 3 x 10⁶ cells, at least 4 x 10⁶ cells, at least 5 x 10⁶ cells, at least 10 x 10⁶ cells, at least 15 x 10⁶ cells, at least 20 x 10⁶ cells, at least 25 x 10⁶ cells, or at least 30 x 10⁶ cells.

In a particular embodiment, the amount of hematopoietic stem or progenitor cells (e.g., CD34⁺, Lin(-) cells) in the therapeutic composition is the amount of hematopoietic stem or progenitor cells in a partial or single cord of blood, or about 0.1 x 10⁵ cells to about 10 x 10⁵ cells; about 0.5 x 10⁶ cells to about 5 x 10⁶ cells; about 1 x 10⁶ cells to about 3 x 10⁶ cells; about 1.5 x 10⁶ cells to about 2.5 x 10⁶ cells; or about 2 x 10⁶ cells to about 2.5 x 10⁶ cells.

In a particular embodiment, the amount of hematopoietic stem or progenitor cells in the therapeutic composition is the amount of hematopoietic stem or progenitor cells in a partial or single cord of blood, or about 1 x 10⁶ cells to about 3 x 10⁶ cells; about 1.0 x 10⁶ cells to about 5 x 10⁶ cells; about 1.0 x 10⁶ cells to about 10 x 10⁶ cells, about 10 x 10⁶ cells to about 20 x 10⁶ cells, about 10 x 10⁶ cells to about 30 x 10⁶ cells, or about 20 x 10⁶ cells to about 30 x 10⁶ cells.

In another embodiment, the amount of hematopoietic stem or progenitor cells in the therapeutic composition is the amount of hematopoietic stem or progenitor cells in a partial or single cord of blood, or about 1 x 10⁶ cells to about 30 x 10⁶ cells; about 1.0 x 10⁶ cells to about 20 x 10⁶ cells; about 1.0 x 10⁶ cells to about 10 x 10⁶ cells, about 2.0 x 10⁶ cells to about 30 x 10⁶ cells, about 2.0 x 10⁶ cells to about 20 x 10⁶ cells, or about 2.0 x 10⁶ cells to about 10 x 10⁶ cells.

In a particular embodiment, the amount of hematopoietic stem or progenitor cells in the therapeutic composition is about 1 x 10⁶ hematopoietic stem or progenitor cells, about 2 x 10⁶ cells, about 5 x 10⁶ cells, about 7 x 10⁶ cells, about 10 x 10⁶ cells, about 15 x 10⁶ cells, about 17 x 10⁶ cells, about 20 x 10⁶ cells about 25 x 10⁶ cells, or about 30 x 10⁶ cells.

In one embodiment, the amount of hematopoietic stem or progenitor cells) in the therapeutic composition administered to a subject is the amount of hematopoietic stem or progenitor cells in a partial or single cord of blood, or at least 0.1 x 10⁵ cells/kg of bodyweight, at least 0.5 x 10⁵ cells/kg of bodyweight, at least 1 x 10⁵ cells/kg of bodyweight, at least 5 x 10⁵ cells/kg of bodyweight, at least 10 x 10⁵ cells/kg of bodyweight, at least 0.5 x 10⁶ cells/kg of bodyweight, at least 0.75 x 10⁶ cells/kg of bodyweight, at least 1 x 10⁶ cells/kg of bodyweight, at least 1.25 x 10⁶ cells/kg of bodyweight, at least 1.5 x 10⁶ cells/kg of bodyweight, at least 1.75 x 10⁶ cells/kg of bodyweight, at least 2 x 10⁶ cells/kg of bodyweight, at least 2.5 x 10⁶ cells/kg of bodyweight, at least 3 x 10⁶ cells/kg of bodyweight, at least 4 x 10⁶ cells/kg of bodyweight, at least 5 x 10⁶ cells/kg of bodyweight, at least 10 x 10⁶ cells/kg of bodyweight, at least 15 x 10⁶ cells/kg of bodyweight, at least 20 x 10⁶ cells/kg of bodyweight, at least 25 x 10⁶ cells/kg of bodyweight, or at least 30 x 10⁶ cells/kg of bodyweight.

Without wishing to be bound to any particular theory, the present invention contemplates, in part, that one of the advantages of the present methods is that fewer hematopoietic stem and progenitor cells can be used in a transplant because the enhanced hematopoietic stem and progenitor cells in the therapeutic composition of the invention have increased engraftment potential, improved homing, and increased capacity for *in vivo* expansion compared to control treated cells and cells treated with an agent at 4°C, for example.

### C. Methods of the Invention

The present inventors analyzed several biological parameters of populations of hematopoietic stem and progenitor cells treated with agents that modify gene expression of the cells, including agents that stimulate the prostaglandin pathway and upregulate gene and cell-surface expression of CXCR4 in order to increase the effectiveness of hematopoietic stem and progenitor cells used in stem cell transplants. A cell population's effectiveness in reconstituting a subject's hematopoietic system upon transplantation depends on such properties as the cell population's ability to home to and engraft in the bone marrow, self-renew, and proliferate *in vivo.* The invention provides a method for modulating a cell population to improve such cell properties and provide resultant therapeutic improvements in hematopoietic reconstitution.

The "engraftment potential" refers to the ability of a cell to engraft. In particular embodiments, the engraftment potential of a hematopoietic stem or progenitor cell, such as a CD34⁺, Lin(-) cell, can be determined by measuring, for example, the activity of PGE₂R₂/R₄ cell signaling pathways, the expression in the cell of genes associated with homing or engraftment, cell viability, and the capacity of the cell to self-renew. Of course, the skilled artisan would appreciate other suitable assays that would also indicate an increased engraftment potential in a hematopoietic stem or progenitor cell. As used herein, the term "engraft" refers to the ability of a cell to integrate into a location, such as a tissue, and persist in the particular location over time, e.g., the ability of a hematopoietic stem or progenitor cell to integrate into and persist in the bone marrow. "Homing" refers to the ability of hematopoietic stem or progenitor cells to localize, i.e., travel, to a particular area or tissue, such as localization of transplanted stem cells to the bone marrow.

In various embodiments, the invention provides a therapeutic composition comprising human hematopoietic stem or progenitor cells contacted with one or more agents capable of increasing CXCR4 gene expression in the cells, including agents that stimulate the prostaglandin pathway, *e.g.,* the PGE₂R₂/R₄ cell signaling pathway. The therapeutic composition of treated cells offers numerous advantages over cells previously used in stem cell transplants, such as, for example, increased homing, engraftment and expansion of the cell population *in vivo.* As used herein, "agent" refers to an agent capable of increasing CXCR4 gene expression in the cells. Such agents include, for example and without limitation, PGE₂ or agents having dmPGE₂ activity, including without limitation, a PGE₂ analogue, a cAMP analogue or activator, and/or a Gα-s activator as described elsewhere herein. In particular embodiments, a population of cells comprising hematopoietic stem or progenitor cells can be contacted with 1, 2, 3, 4, 5 or more agents in any combination, simultaneously or sequentially.

Human hematopoietic stem or progenitor cells contacted with an agent capable of increasing CXCR4 gene expression in the cells, such as PGE₂ or an agent having dmPGE₂ activity, under conditions sufficient to increase engraftment and/or engraftment potential and/or expansion, can be characterized in multiple and various ways, such as by increased levels of intracellular cAMP signaling, e.g., CREB phosphorylation, or as determined by a biochemical assay; gene expression signatures indicating upregulation of genes implicated in the PGE₂R₂/R₄ cell signaling pathway, e.g., CREM, and genes that increase hematopoietic stem and progenitor cell homing and engraftment, *e.g.,* CXCR4, as determined by gene expression assays, *e.g.,* microarrays; no measurable decrease in hematopoietic stem and progenitor cell viability as determined by cell viability assays, e.g., 7-aminoactinomycinD (7-AAD) staining; and/or an increased capacity of hematopoietic stem cells to self-renew as determined by an *in vitro* colony forming units (CFU-C) assay, for example.

In one embodiment, hematopoietic stem or progenitor cells contacted with an agent capable of increasing CXCR4 gene expression in the cells, such as PGE₂ or an agent having dmPGE₂ activity, under conditions sufficient to increase engraftment and/or engraftment potential and/or expansion can be identified by examining the gene expression signature of the contacted (treated) cells compared to vehicle treated cells or cells treated with an agent at 4°C.

In particular embodiments, treated hematopoietic stem or progenitor cells that have increased engraftment and/or engraftment potential and/or increased *in vivo* expansion have increased expression of 1, 2, 3, 4, 5, or all of the following genes compared to vehicle treated cells or cells treated with an agent at 4°C: hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), Fos-related antigen 2 (FOSL2), and CXC chemokine receptor 4 (CXCR4). In specific embodiments of the invention, CXCR4 is upregulated by at least four fold in the hematopoietic stem or progenitor cells in the therapeutic composition as compared to the level of CXCR4 expression in non-treated cells.

In contrast to observations derived from pre-clinical studies, the present inventors discovered that hematopoietic stem and progenitor cells contacted with an agent that stimulates the prostaglandin pathway increases the expansion and engraftment potential of the cells under particular conditions described herein. These conditions optimize the desired biological response of treatment with an agent that stimulates the prostaglandin pathway, including stem cell homing, survival, proliferation, and engraftment.

Thus, the inventors have discovered that conditions believed to decrease hematopoietic stem and progenitor cell viability and decrease dmPGE₂ half-life unexpectedly result in hematopoietic stem o progenitor cells that display increased potential for engraftment and/or *in vivo* expansion because they preserve cell viability, increase homing and engraftment to the bone marrow (e.g., increased CXCR4 expression), and increase capacity for cell self-renewal.

Accordingly, the invention contemplates novel methods for conducting bone marrow, peripheral blood, and umbilical cord blood transplants, in part, by treating hematopoietic stem or progenitor cells populations with agents described herein that upregulate CXCR4 expression in the cells, including agents that stimulate the PGE₂R₂/R₄ cell signaling pathway, such as dmPGE₂, under conditions not expected to be favorable for increasing hematopoietic stem and progenitor cell engraftment or *in vivo* expansion of hematopoietic stem and progenitor cells.

As used herein, the terms "conditions sufficient," or "under conditions sufficient," refer to the incubation conditions for treating the source of transplant material, for example, bone marrow cells, peripheral blood cells, or cord blood cells, and/or other populations of cells comprising hematopoietic stem and/or progenitor cells, and/or enriched or selected populations of hematopoietic stem and progenitor cells, with an agent that increases CXCR4 gene expression in the cells. In one embodiment, the conditions are sufficient to increase engraftment of hematopoietic stem and progenitor cells administered to a subject. In one embodiment, the conditions are sufficient to increase the expansion of hematopoietic stem or progenitor cells administered to a subject. In another embodiment, the conditions are sufficient to increase engraftment and expansion of the population of hematopoietic stem or progenitor cells administered to a subject. Incubations conditions include, but are not limited to source of the cells, agent concentration, duration of incubation of the cells and the agent, and the temperature of the incubation. In particular embodiments, the agent is PGE₂ or an agent having dmPGE₂ activity. In one embodiment, the agent is 16,16-dimethyl PGE₂.

In various embodiments, conditions sufficient to increase engraftment and/or expansion of hematopoietic stem o progenitor cells include, incubation at a physiologically relevant temperature, such as a temperature range of about 39°C (about room temperature to about body temperature), including but not limited to temperatures of about 22°C, 23°C, 24°C, 25°C, 26°C, 27°C, 28°C, 29°C, 30°C, 31 °C, 32°C, 33°C, 34°C, 35°C, 36°C, 37°C, 38°C, and 39°C; at a final concentration of about 10 nM to about 120 µM 16,16-dimethyl PGE₂, including, but not limited to about 100 nM, about 500 nM, about 1 µM, about 10 µM, about 20 µM, about 30 µM, about 40 µM, about 50 µM, about 60 µM, about 70 µM, about 80 µM, about 90 µM, about 100 µM, about 110 µM, or about 120 µM, or any other intervening concentration of 16,16-dimethyl PGE₂ (e.g., .1 µM, 1 µM, 5 µM, 10 µM, 20 µM, 50 µM, 100 µM); and incubation for about 60 minutes to about 4 hours, including but not limited to incubation for a duration of about 60 minutes, about 70 minutes, about 80 minutes, about 90 minutes, about 100 minutes, about 110 minutes, about 2 hours, about 2.5 hours, about 3 hours, about 3.5 hours or about 4 hours or any other intervening duration of incubation (e.g., 111 minutes, 112 minutes, 113 minutes, 114 minutes, 115 minutes, 116 minutes, 117 minutes, 118 minutes, 119 minutes).

As used herein, the term "about" or "approximately" means a concentration, temperature, duration, quantity, level, value, number, frequency, percentage, dimension, size, amount, weight or length that varies by as much as 30, 25, 20, 25, 10, 9, 8, 7, 6, 5, 4, 3, 2 or 1 % to a reference concentration, temperature, duration quantity, level, value, number, frequency, percentage, dimension, size, amount, weight or length. For example, in preferred embodiment, the term about refers to a range of quantities centered about the specific quantity plus or minus 10%, *e.g.,* a temperature of about 37°C refers to a temperature range of 33°C to 41 °C. In another preferred embodiment, the term about refers to a range of quantities centered about the specific quantity plus or minus 5%. In another preferred embodiment, the term about refers to a range of quantities centered about the specific quantity plus or minus 1%.

In particular embodiments, conditions sufficient to increase engraftment and/or *in vivo* expansion of hematopoietic stem and progenitor cells include, incubation at a temperature range of about 35°C to about 39°C; at a final concentration of about 10 µM to about 25 µM 16,16-dimethyl PGE₂; and incubation for about 1 hour to about 4 hours, for about 2 hours to about 3 hours, for about 2 hours to about 4 hours, or for about 3 hours to about 4 hours.

In another embodiment, conditions sufficient to increase engraftment and/or *in vivo* expansion of hematopoietic stem or progenitor cells include, incubation at a temperature of about 37°C (about body temperature); at a final concentration of about 10 µM or more 16,16-dimethyl PGE₂; and incubation for about two hours.

In another embodiment, contacting human cord blood, bone marrow cells, or mobilized peripheral blood cells comprising hematopoietic stem or progenitor cells or a purified population of Lin(-)CD34⁺, hematopoietic stem or progenitor cells with a final concentration of 10 µM 16,16-dmPGE₂ (dmPGE₂) for 120 minutes or more at a temperature of 37°C increases the potential for hematopoietic stem or progenitor cell engraftment in the bone marrow of a subject. The contacted cells show no statistically significant decrease in cell viability, and show statistically significant increases in gene expression associated with hematopoietic stem or progenitor cell homing and engraftment, and the capacity to self-renew.

In another embodiment, contacting human cord blood, bone marrow cells, or mobilized peripheral blood cells comprising hematopoietic stem or progenitor cells or a purified population of Lin(-)CD34⁺, hematopoietic stem or progenitor cells with a final concentration of 10 µM 16,16-dmPGE₂ (dmPGE₂) for 120 minutes or more at a temperature of 37 °C increases the *in vivo* expansion of the hematopoietic stem or progenitor cell population administered to a subject.

In various embodiments, the invention provides, in part, methods for obtaining and preparing a population of cells for a hematopoietic stem and progenitor cell transplant, comprising contacting the population of cells with one or more agents that increase CXCR4 gene expression in the cells, including agents that stimulate the PGE₂R₂ and/or PGE₂R₄ cell signaling pathway, under conditions sufficient to increase engraftment potential and/or engraftment of the cells.

In particular embodiments, the invention provides, in part, methods for obtaining and preparing a population of cells for increasing the amount of hematopoietic stem and progenitor cells in a subject, comprising contacting the population of cells with one or more agents that increase CXCR4 gene expression in the cells, including agents that stimulate the PGE₂R₂ and/or PGE₂R₄ cell signaling pathway, under conditions sufficient to increase the expansion of the cell population *in vivo.*

In various other embodiments, the invention provides, in part, a method of increasing hematopoietic stem and progenitor cell engraftment in a subject comprising contacting a population of cells that comprises hematopoietic cells that express CD34 but that lack Lin expression (e.g., Lin(-)CD34⁺, cells) with one or more agents selected from the group consisting of: a prostaglandin E₂ (PGE₂) or an agent having dmPGE₂ activity, and administering the population of cells to a subject. The cells are contacted with the agent under conditions sufficient to increase the engraftment of the contacted hematopoietic stem and progenitor cells in the subject as described elsewhere herein.

In certain embodiments, the invention provides, in part, a method of expanding a hematopoietic stem and progenitor cell population in a subject, *in vivo,* comprising contacting a population of cells that comprises hematopoietic cells that express CD34 but that lack lin expression (e.g., Lin(-)CD34⁺, cells) with one or more agents selected from the group consisting of: a prostaglandin E₂ (PGE₂) or an agent having dmPGE₂ activity, and administering the population of cells to a subject. The cells are contacted with the agent under conditions sufficient to expand the contacted hematopoietic stem and progenitor cell population in the subject as described elsewhere herein.

The invention contemplates, in part, methods to increase stem cell engraftment in a subject in need thereof (e.g., a human) comprising contacting a population of cells that comprises hematopoietic stem and/or progenitor cells (e.g., bone marrow cells, peripheral blood cells, and/or umbilical cord blood cells) with PGE₂ or an analogue thereof, e.g., 16,16-dimethyl PGE₂ (dmPGE₂) or an agent having dmPGE₂ activity and administering the cells to the subject. In one embodiment, the source of cells comprising hematopoietic stem and/or progenitor cells is contacted with a PGE₂ analogue such as dmPGE₂. In various embodiments, the source of cells comprising hematopoietic stem and/or progenitor cells is contacted with an agent having dmPGE₂ activity such as dmPGE₂, a cAMP analogue or enhancer, or a Gα-s activator.

In a certain embodiment, the population of cells comprising hematopoietic stem and/or progenitor cells is contacted with a PGE₂ analogue such as dmPGE₂ and an agent having dmPGE₂ activity, e.g., a cAMP analogue or enhancer, or a Gα-s activator. In another embodiment, the source of cells comprising hematopoietic stem and/or progenitor cells is contacted with one or more PGE₂ analogues, one or more cAMP analogues or enhancers, and/or one or Gα-s activators.

In various other embodiments, the invention provides methods of treating a subject in need thereof that comprise identifying a subject in need, and administering to the subject a population of cells that comprises hematopoietic stem and/or progenitor cells contacted with one or more agents selected from the group consisting of: a prostaglandin E₂ (PGE₂), an agent having dmPGE₂ activity, e.g., a cAMP analogue or enhancer, and a Gα-s activator under conditions sufficient to increase the engraftment or *in vivo* expansion of the contacted hematopoietic stem or progenitor cells in the subject, thereby treating the subject in need.

By "enhance" or "promote," or "increase" or "activate" refers generally to the ability of PGE₂ or an agent having dmPGE₂ activity to produce or cause a greater physiological response *(i.e.,* downstream effects) in a cell, as compared to the response caused by either vehicle or a control molecule/composition, e.g., increased engraftment/engraftment potential of stem and/or progenitor cells and increased *in vivo* stem cell expansion. A measurable physiological response may include an increase in hematopoietic stem and/or progenitor cell engraftment, viability, homing, self-renewal, and/or expansion, among others apparent from the understanding in the art and the description herein. In one embodiment, the increase can be an increase in gene expression as a result of increased signaling through the PGE₂R₂ and/or PGE₂R₄ cell signaling pathways, including, but not limited to an increase in CREB phosphorylation, an increase in CREM expression, and an increase in CXCR4. Increases in hematopoietic stem and/or progenitor cell engraftment, viability, homing, self-renewal and/or *in vivo* expansion, can also be ascertained using methods known in the art, such as gene expression, CFU-C assays, CFU-S assays, CAFC assays, and cell surface protein expression, among others. An "increased" or "enhanced" amount is typically a "statistically significant" amount, and may include an increase that is 1.1, 1.2, 1.5, 2, 3, 4, 5, 6, 7, 8, 9, 10, 15, 20, 30 or more times (e.g., 500, 1000 times) (including all integers and decimal points in between and above 1, *e.g.,* 1.5, 1.6, 1.7. 1.8, etc.) the response produced by vehicle (the absence of an agent) or a control composition. For example, in particular embodiments, methods of the invention comprise contacting a population of cells comprising hematopoietic stem or progenitor cells with dmPGE₂ at about 37°C. These cells have an increased engraftment potential and expansion compared to cells contacted at about 4°C.

By "decrease" or "lower," or "lessen," or "reduce," or "abate" refers generally to the ability of a PGE₂ or an agent having dmPGE₂ activity to produce or cause a lesser physiological response *(i.e.,* downstream effects) in a cell, as compared to the response caused by either vehicle or a control molecule/composition, e.g., decreased apoptosis. In one embodiment, the decrease can be a decrease in gene expression or a decrease in cell signaling that normally is associated with a reduction of cell viability. An "decrease" or "reduced" amount is typically a "statistically significant" amount, and may include an decrease that is 1.1, 1.2, 1.5, 2, 3, 4, 5, 6, 7, 8, 9, 10, 15, 20, 30 or more times (e.g., 500, 1000 times) (including all integers and decimal points in between and above 1, *e.g.,* 1.5, 1.6, 1.7. 1.8, etc.) the response produced by vehicle (the absence of an agent) or a control composition. For example, in particular embodiments, methods of the invention comprise contacting a population of cells comprising hematopoietic stem or progenitor cells with dmPGE₂ at about 37°C. The contacted cells do not show a statistically significant decrease in cell viability compared to cells contacted with dmPGE₂ at about 4°C.

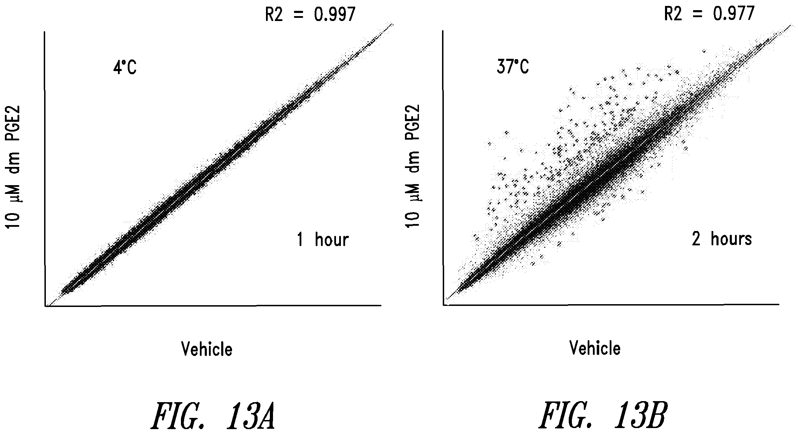

By "maintain," or "preserve," or "maintenance," or "no change," or "no substantial change," or "no substantial decrease" refers generally to the ability of a PGE₂ or an agent having dmPGE₂ activity to produce or cause a comparable physiological response *(i.e.,* downstream effects) in a cell, as compared to the response caused by either vehicle or a control molecule/composition (reference response). A comparable response is one that is not significantly different or measurably different from the reference response (see Figure 13A). In one embodiment, a population of cells comprising hematopoietic stem and progenitor cells is contacted with an agent that stimulates the PGE₂R₂ and/PGE₂R₄ cell signaling pathways, such as an agent having dmPGE₂ activity, e.g., dmPGE₂, a cAMP analogue or enhancer, and a Gα-s activator at about 37°C for about two hours. The treated cells do not show a statistically significant decrease in cell viability compared to cells contacted at about 4°C. In other words, the methods described herein maintain, do not substantially decrease, do not result in a statistically significant decrease in, do not cause a loss of, and/or do not substantially change hematopoietic stem and progenitor cell viability compared to cells contacted at about 4°C.

In particular embodiments, cells are treated with an agent, e.g., dmPGE₂ for a period of time. In related embodiments, the cells are washed after treatment in a cell culture medium so that they are substantially free of the agent. For example, in one embodiment, a population of cells comprising human hematopoietic stem or progenitor cells, e.g., bone marrow cells, mobilized peripheral blood cells, or umbilical cord blood cells is contacted with 16,16-dimethyl PGE₂ for a period of 120 minutes at about 37°C. After the incubation, but prior to infusion or subsequent treatment or storage, the cells are washed with a cell culture medium, such as low molecular weight dextran with 5% human serum albumin medium (LMD/5% HSA) or Stem Span medium (Stem Cells Technology Inc.).

In various illustrative embodiments, the invention provides, in part, *in vitro* or *ex vivo* treatment methods comprising contacting a population of cells comprising hematopoietic stem or progenitor cells with PGE₂ or an agent having dmPGE₂ activity that maintains stem/progenitor cell viability, and increases engraftment, homing, self-renewal, and expansion *in vivo.*

The term *"ex vivo"* refers generally to activities that take place outside an organism, such as experimentation or measurements done in or on living tissue in an artificial environment outside the organism, preferably with minimum alteration of the natural conditions. In particular embodiments, "*ex vivo"* procedures involve living cells or tissues taken from an organism and cultured in a laboratory apparatus, usually under sterile conditions, and typically for a few hours or up to about 24 hours, but including up to 48 or 72 hours, depending on the circumstances. In certain embodiments, such tissues or cells can be collected and frozen, and later thawed for *ex vivo* treatment. Tissue culture experiments or procedures lasting longer than a few days using living cells or tissue are typically considered to be *"in vitro*," though in certain embodiments, this term can be used interchangeably with *ex vivo.*

The recitations *"ex vivo* administration," *"ex vivo* treatment," or "*ex vivo* therapeutic use," relate generally to medical procedures in which one or more organs, cells, or tissues are obtained from a living or recently deceased subject, optionally purified/enriched, exposed to a treatment or procedure (e.g., an *ex vivo* administration step that involves incubating the cells with a composition or agent of the present invention to enhance expansion of desirable cells, such as hematopoietic stem or progenitor cells). Cells treated *ex vivo* may be administered to the same or different living subject.

Such *ex vivo* therapeutic applications may also include an optional *in vivo* treatment or procedural step, such as by administering contacted cells of the invention one or more times to the living subject. Both local and systemic administration is contemplated for these embodiments, according to well-known techniques in the art and as described elsewhere herein. The amount of cells administered to a subject will depend on the characteristics of that subject, such as general health, age, sex, body weight, and tolerance to drugs, as well as the degree, severity, and type of reaction to the drug and/or cell transplant.

The term *"in vivo"* refers generally to activities that take place inside an organism, such as cell engraftment, cell homing, self-renewal of cells, and expansion of cells. In one embodiment, the term *"in vivo* expansion" refers to the ability of a cell population to increase in number *in vivo.* In particular embodiments, the *in vivo* expansion include self-renewal and/or proliferation of stem cells.

In one embodiment, the invention provides, in part, a method of preparing a population of cells, e.g., bone marrow cells, mobilized peripheral blood cells, umbilical cord blood cells, for a transplant, e.g., bone marrow transplant that comprises contacting the cells *ex vivo,* with dmPGE₂ or an agent having dmPGE₂ activity at a temperature and for a time sufficient to increase the engraftment and/or *in vivo* expansion of the contacted cells when administered to a subject.

In a particular embodiment, the invention provides a method of treating a subject in need of hematopoietic reconstitution or reconstitution of the hematopoietic system comprising identifying a subject in need of hematopoietic reconstitution, and administering to the subject an amount of hematopoietic stem and/or progenitor cells contacted with an agent capable of increasing CXCR4 gene expression, such as dmPGE₂, under conditions sufficient to increase the engraftment of the contacted hematopoietic stem and progenitor cells in the subject, thereby treating the subject in need of hematopoietic reconstitution.

In another particular embodiment, the invention provides a method of treating a subject in need of hematopoietic reconstitution, reconstitution of the hematopoietic system, an increased number of hematopoietic stem or progenitor cells, and/or *in vivo* expansion of hematopoietic stem or progenitor cells comprising identifying a subject in need of hematopoietic reconstitution, and administering to the subject an amount of hematopoietic stem or progenitor cells contacted with an agent that increases CXCR4 gene expression, such as dmPGE₂ under conditions sufficient to increase the *in vivo* expansion of the contacted hematopoietic stem or progenitor cells in the subject, thereby treating the subject in need of hematopoietic reconstitution.

A "subject," as used herein, includes any animal that exhibits a symptom that can be treated with an agent or composition or device of the invention, or can be treated with HSCs or cord blood that have been treated *ex vivo* with an agent or composition of the invention. "Subjects in need" of hematopoietic reconstitution, reconstitution of the hematopoietic system, an increased number of hematopoietic stem or progenitor cells, and/or *in vivo* expansion of hematopoietic stem or progenitor cells include, but are not limited to subjects that have or that have been diagnosed with various types of leukemias, anemias, lymphomas, myelomas, immune deficiency disorders, and solid tumors as discussed elsewhere herein. A "subject" also includes a human who is a candidate for stem cell transplant or bone marrow transplantation, such as during the course of treatment for a malignant disease or a component of gene therapy. Subjects may also include individuals or animals that donate stem cells or bone marrow for allogeneic transplantation. In certain embodiments, a subject may have undergone irradiation therapy or chemotherapy, such as during various cancer treatments. Suitable subjects *(e.g.,* patients) include laboratory animals *(e.g.,* mouse, rat, rabbit, or guinea pig), farm animals, and domestic animals or pets (*e.g*., a cat or dog). Non-human primates and, preferably, human patients, are included. Typical subjects include animals that exhibit aberrant amounts (lower or higher amounts than a "normal" or "healthy" subject) of one or more physiological activities that can be modulated by an agent or a stem cell or marrow transplant.

Suitable methods for administering populations of cells used in the methods described herein include parenteral administration, including, but not limited to methods of intravascular administration, such as intravenous and intraarterial administration. Additional illustrative methods for administering cells of the invention include intramuscular, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

Administration of an "amount" of hematopoietic stem and progenitor cells to a subject refers to administration of "an amount effective," to achieve the desired therapeutic or prophylactic result, including without limitation treatment of the subject. A "therapeutically effective amount" of hematopoietic stem or progenitor cells for purposes herein is thus determined by such considerations as are known in the art, and may vary according to factors such as the disease state, age, sex, and weight of the individual, and the ability of the hematopoietic stem and progenitor cells to elicit a desired response in the individual. The term "therapeutically effective amount" includes an amount that is effective to "treat" a subject (e.g., a patient). A therapeutically effective amount is also one in which any toxic or detrimental effects of the hematopoietic stem or progenitor cells are outweighed by the therapeutically beneficial effects.

A "prophylactically effective amount" refers to an amount of hematopoietic stem or progenitor cells effective to achieve the desired prophylactic result. Typically but not necessarily, since a prophylactic dose is used in subjects prior to or at an earlier stage of disease, the prophylactically effective amount is less than the therapeutically effective amount.

In another particular embodiment, the invention contemplates, a method of treating a subject in need of a hematopoietic stem/progenitor cell transplant that comprises: selecting the subject in need of a hematopoietic stem/progenitor cell transplant and administering to a subject, a population of cells contacted *ex vivo* with dmPGE₂ or an agent having dmPGE₂ activity at a temperature and for a time sufficient to increase the engraftment and/or *in vivo* expansion of the contacted cells in a subject compared to non-contacted cells. In particular embodiments, the subject is in need of hematopoietic reconstitution.

As used herein, the terms "treatment," "treating," and the like, refer to obtaining a desired pharmacologic and/or physiologic effect, including without limitation achieving an improvement or elimination of symptoms of a disease. The effect may be prophylactic in terms of completely or partially preventing a disease or symptom thereof and/or may be therapeutic in terms of achieving an improvement or elimination of symptoms, or providing a partial or complete cure for a disease and/or adverse affect attributable to the disease. "Treatment," as used herein, covers any treatment of a disease in a mammal, particularly in a human, and includes: (a) preventing the disease from occurring in a subject which may be predisposed to the disease but has not yet been diagnosed as having it; (b) inhibiting the disease, *i.e.,* arresting its development; (c) relieving the disease, *e.g.,* causing regression of the disease, *e.g.,* to completely or partially eliminate symptoms of the disease; and (d) restoring the individual to a pre-disease state, e.g., reconstituting the hematopoietic system.

"Treatment" or "treating," as used herein, includes any desirable effect on the symptoms or pathology of a disease or pathological condition, and may include even minimal reductions in one or more measurable markers of the disease or condition being treated. "Treatment" does not necessarily indicate complete eradication or cure of the disease or condition, or associated symptoms thereof. In particular methods of the invention, treatment or treating provides improved engraftment of a cell population in a subject, improved hematopoietic reconstitution in a subject, or improved survival in a subject.

Subjects in need of this type of treatment include subjects suffering from (*e.g*., afflicted with) non malignant blood disorders, particularly immunodeficiencies (*e.g*. SCID, Fanconi's anemia, severe aplastic anemia, or congenital hemoglobinopathies, or metabolic storage diseases, such as Hurler's disease, Hunter's disease, mannosidosis, among others) or cancer, particularly hematological malignancies, such as acute leukemia, chronic leukemia (myeloid or lymphoid), lymphoma (Hodgkin's or non-Hodgkin's), multiple myeloma, myelodysplastic syndrome, or non-hematological cancers such as breast carcinoma, colon carcinoma, neuroblastoma, or renal cell carcinoma.

The methods of the invention can be used to treat any disease or disorder in which it is desirable to increase the amount of hematopoietic stem or progenitor cells in the bone marrow or mobilize hematopoietic stem or progenitor cells to the bone marrow. For example, methods of the invention can be used to treat patients requiring a bone marrow transplant or a hematopoietic stem or progenitor cell transplant, such as cancer patients undergoing chemo and/or radiation therapy. Methods of the present invention are particularly useful in the treatment of patients undergoing chemotherapy or radiation therapy for cancer, including patients suffering from myeloma, non-Hodgkin's lymphoma, Hodgkin's lymphoma, leukemia, and solid tumors (breast cancer, ovarian cancer, brain cancer, prostate cancer, lung cancer, colon cancer, skin cancer, liver cancer, or pancreatic cancer). Methods of the present invention can also be used in the treatment of patients suffering from aplastic anemia, an immune disorder (severe combined immune deficiency syndrome or lupus), myelodysplasia, thalassemaia, sickle-cell disease or Wiskott-Aldrich syndrome. Disorders treated by methods of the invention can be the result of an undesired side effect or complication of another primary treatment, such as radiation therapy, chemotherapy, or treatment with a bone marrow suppressive drug, such as zidovadine, chloramphenical or gangciclovir. Such disorders include neutropenias, anemias, thrombocytopenia, and immune dysfunction. In addition, methods of the invention can be used to treat damage to the bone marrow caused by unintentional exposure to toxic agents or radiation.

The disorder to be treated can also be the result of an infection (e.g., viral infection, bacterial infection or fungal infection) causing damage to stem or progenitor cells of the bone marrow.

In addition to the above, further conditions which can benefit from treatment using methods of the invention include, but are not limited to, lymphocytopenia, lymphorrhea, lymphostasis, erythrocytopenia, erthrodegenerative disorders, erythroblastopenia, leukoerythroblastosis; erythroclasis, thalassemia, myelofibrosis, thrombocytopenia, disseminated intravascular coagulation (DIC), immune (autoimmune) thrombocytopenic purpura (ITP), HIV inducted ITP, myelodysplasia; thrombocytotic disease, thrombocytosis, congenital neutropenias (such as Kostmann's syndrome and Schwachman-Diamond syndrome), neoplastic associated - neutropenias, childhood and adult cyclic neutropaenia; post-infective neutropaenia; myelodysplastic syndrome; neutropaenia associated with chemotherapy and radiotherapy; chronic granulomatous disease; mucopolysaccharidoses; Diamond Blackfan; Sickle cell disease; or Beta thalassemia major.

In a particular embodiment, the patient in need of a transplant is a bone marrow donor who has donated bone marrow, is a bone marrow donor who has yet to donate bone marrow, is a bone marrow donor transplant recipient, has hematopoietic progenitor cells under environmental stress, has anemia, has a reduced level of immune cell function compared to a normal subject, or has an immune system deficiency.

In a certain embodiment, the patient in need of a transplant has myeloma, non-Hodgkin's lymphoma, Hodgkin's lymphoma, chronic myeloid leukemia, chronic myelogenous leukemia, chronic granulocytic leukemia, acute lymphoblastic leukemia, acute nonlymphoblastic leukemia, or pre-leukemia.

### D. Agents Used in the Methods of the Invention

In various embodiments, the invention contemplates a therapeutic composition of a population of cells comprising hematopoietic stem or progenitor cells contacted with one or more agents that increases CXCR4 gene expression in the cells, such as agents that stimulate the PGE₂R₂ and/or PGE₂R₄ cell signaling pathways.

Using cGMP practices, agents useful in preparing the therapeutic composition of the invention can be formulated in an organic solvent, such as methyl acetate, for use in contacting the cells of the invention, and may be supplied in an endotoxin free vessel. Agents contemplated by the invention are suitable for *ex vivo* administration to mammalian cells, as described herein. In certain embodiments, the solvent is typically a suitable organic solvent, as described herein (e.g., DMSO, DMF, DME, etc., including combinations or mixtures thereof). One or more solvents may be combined at certain ratios. For instance, a mixture of two solvents may be combined at a ratio of 9.5:0.5, 9:1, 8:2, 7:3, 6:4, 5:5, etc., including all integers and decimal points.

The recitation "organic solvent" or "suitable organic solvent" relates generally to carbon containing liquids or gases that dissolve a solid, liquid, or gaseous solute, resulting in a solution. A "suitable" organic solvent is one that is appropriate for *ex vivo* administration to, or incubation with, mammalian cells, and may also be appropriate for *in vivo* administration to a subject, such as by having minimal toxicity or other inhibitory effects under *ex vivo* conditions *(e.g.,* cell culture) or *in vivo* at a selected concentration for the time of incubation or administration. A suitable organic solvent should also be appropriate for storage stability and handling of the agents described herein. Examples of suitable organic solvents include, but are not limited to, dimethyl sulfoxide (DMSO), N,N-dimethylformamide (DMF), dimethoxyethane (DME), and dimethylacetamide, including mixtures or combinations thereof. In certain embodiments, a composition or organic solvent is "substantially free" of methyl acetate, meaning that there should be no more than trace amounts of methyl acetate in the composition or solvent, and preferably undetectable amounts (e.g., as measured by high pressure liquid chromatography (HPLC), gas chromatography (GC), etc.).

As used herein, the term "endotoxin free" refers to vessels and/or compositions that contain at most trace amounts *(i.e.,* amounts having no adverse physiological effects to a subject) of endotoxin, and preferably undetectable amounts of endotoxin. By "substantially free of endotoxin" is meant that there is less endotoxin per dose of cells than is allowed by the FDA for a biologic, which is a total endotoxin of 5 EU/kg body weight per day, which for an average 70 kg person is 350 EU per total dose of cells. In one embodiment, the term "endotoxin free" refers to a vessel and/or compositions that is at least 95%, at least 96%, at least 97%, at least 98%, at least 99%, or 100% endotoxin free. Endotoxins are toxins associated with certain bacteria, typically gram-negative bacteria, although endotoxins may be found in gram-positive bacteria, such as *Listeria monocytogenes.* The most prevalent endotoxins are lipopolysaccharides (LPS) or lipooligosaccharides (LOS) found in the outer membrane of various Gram-negative bacteria, and which represent a central pathogenic feature in the ability of these bacteria to cause disease. Small amounts of endotoxin in humans can produce fever, a lowering of the blood pressure, and activation of inflammation and coagulation, among other adverse physiological effects. Therefore, it is often desirable to remove most or all traces of endotoxin from drug product containers, because even small amounts may cause adverse effects in humans. Endotoxins can be removed from vessels using methods known in the art, for example, vessels can be cleaned in HEPA filtered washing equipment with endotoxin-free water, depyrogenated at 250°C, and clean-packaged in HEPA filtered workstations located inside a class 100/10 clean room (*e.g*., a class 100 clean room, contains no more than 100 particles bigger than half a micron in a cubic foot of air).

As used herein, the term "good manufacturing practice (GMP)" refers to the control and management of manufacturing, and quality control testing, of foods, pharmaceutical products, and medical devices. GMP does not necessarily rely on sampling, but instead relies on documentation of every aspect of the process, activities, and operations involved with drug and medical device manufacture. If the documentation showing how the product was made and tested (which enables traceability and, in the event of future problems, recall from the market) is not correct and in order, then the product does not meet the required specification and is considered contaminated (*i.e.,* adulterated in the US). Additionally, GMP typically requires that all manufacturing and testing equipment has been qualified as suitable for use, and that all operational methodologies and procedures (e.g., manufacturing, cleaning, and analytical testing) utilized in the drug manufacturing process have been validated according to predetermined specifications to demonstrate that they can perform their purported function(s). In the US, the phrase "current good manufacturing practice" appears in 501(B) of the 1938 Food, Drug, and Cosmetic Act (21 U.S.C. § 351).

Agents which may be used in preparing a therapeutic composition of the invention are agents capable of enhancing a human hematopoietic stem or progenitor cell population's homing and engraftment potential. Such agents include agents that increase CXCR4 gene expression in the cells, including agents that stimulate the PGE₂R₂ and/or PGE₂R₄ cell signaling pathways. Useful agents include, but are not limited to PGE₂ and agents that have dmPGE₂ activity, e.g., PGE₂ analogues, cAMP analogues or enhancers, and Gα-s activators. In certain embodiments, PGE₂R₄ specific analogues are of particular interest, and in some embodiments, the agent preferentially binds and activates a PGE₂ E₄ receptor.

As used herein, the terms "prostaglandin E₂" or "PGE₂" include, without limitation, any naturally-occurring or chemically synthesized PGE₂ molecule, as well as "analogues" thereof. As used herein, the term "analogue" or relates to a chemical molecule that is similar to another chemical substance, e.g., PGE₂, in structure and function, often differing structurally by a single element or group, but may differ by modification of more than one group (e.g., 2, 3, or 4 groups) if it retains the same function as the parental chemical. Such modifications are routine to persons skilled in the art, and include, for example, additional or substituted chemical moieties, such as esters or amides of an acid, protecting groups such as a benzyl group for an alcohol or thiol, and tert-butoxylcarbonyl groups for an amine. Also included are modifications to alkyl side chains, such as alkyl substitutions (e.g., methyl, dimethyl, ethyl, etc.), modifications to the level of saturation or unsaturation of side chains, and the addition of modified groups such as substituted phenyl and phenoxy. Analogues can also include conjugates, such as biotin or avidin moieties, enzymes such as horseradish peroxidase and the like, and including radiolabeled, bioluminescent, chemoluminescent, or fluorescent moieties. Also, moieties may be added to the agents described herein to alter their pharmacokinetic properties, such as to increase half-life *in vivo* or *ex vivo,* or to increase their cell penetration properties, among other desirable properties. Also included are prodrugs, which are known to enhance numerous desirable qualities of pharmaceuticals (e.g., solubility, bioavailability, manufacturing, etc.) (*see, e.g.,* WO/2006/047476 for exemplary EP agonist prodrugs, which is incorporated by reference for its disclosure of such agonists).

Illustrative examples of PGE₂ "analogues" and agents that have dmPGE₂ activity include, without limitation, 16,16-dimethyl PGE₂ (dmPGE₂), 16,16-dimethyl PGE₂ p-(p-acetamidobenzamido) phenyl ester, 11-deoxy-16,16-dimethyl PGE₂, 9-deoxy-9-methylene-16, 16-dimethyl PGE₂, 9-deoxy-9-methylene PGE₂, 9-keto Fluprostenol, 5-trans PGE₂, 17-phenyl-omega-trinor PGE₂, PGE₂ serinol amide, PGE₂ methyl ester, 16-phenyl tetranor PGE₂, 15(S)-15-methyl PGE₂, 15(R)-15-methyl PGE₂, 8-iso-15-keto PGE₂, 8-iso PGE₂ isopropyl ester, 20-hydroxy PGE₂, 11-deoxy PGE1, nocloprost, sulprostone, butaprost, 15-keto PGE₂, and 19 (R) hydroxyy PGE₂. Also included are PG analogues or derivatives having a similar structure to PGE₂ that are substituted with halogen at the 9-position (see, e.g., WO 2001/12596, herein incorporated by reference in its entirety), as well as 2-decarboxy-2-phosphinico prostaglandin derivatives, such as those described in U.S. Publication No. 2006/0247214, herein incorporated by reference in its entirety).

PGE1 analogues, including without limitation alprostadil, can also be used to activate the PGE₂R₂ (EP₂) and PGE₂R₄ (EP₄) cell signaling pathways, and are contemplated as agents useful in the methods of the invention.

Stimulation/activation of the PGE₂R₂ (EP₂) and PGE₂R₄ (EP₄) cell signaling pathways are contemplated to underlie the physiological responses in hematopoietic stem and progenitor cells that increase engraftment, maintain cell viability, and increase homing and proliferation of the cells. Accordingly, in one embodiment, a "non-PGE₂-based ligand" that binds to and stimulates PGE₂R₂ and PGE₂R₄ receptors *(i.e.,* a PGE₂R₂/PGE₂R₄ agonist) is contemplated for use in the methods of the present invention.

Illustrative examples of non-PGE₂-based EP₂ receptor agonists include CAY10399, ONO_8815Ly, ONO-AE1-259, CP-533,536 and carbazoles and fluorenes disclosed in WO 2007/071456.

Illustrative examples of non-PGE₂-based EP₄ agonists include ONO-4819, APS-999 Na, AH23848, ONO-AE1-329, and other non-PGE₂-based EP₄ agonists disclosed in WO/2000/038663; U.S. Patent No. 6,747,037; and U.S. Patent No. 6,610,719).

Agents selective for the PGE₂ EP₄ receptor preferentially bind to PGE₂ EP₄ receptors. Such agents have a higher affinity for the EP₄ receptor than for any of the other three EP receptors namely EP₁, EP₂ and EP₃. Agents that selectively bind the PGE EP₄ receptor include, but are not limited to, agents selected from the group consisting of: 5-[(IE,3R)-4,4-difluoro-3-hydroxy-4-phenyl-I-buten-I-yl]-I-[6-(2H-tetrazol-5R-yl)hexyl]-2-pyrrolidinone; 2-[3-[(IR,2S,3R)-3-hydroxy-2-[(E,3S)-3-hydroxy-5-[2-(methoxymethyl)phenyl]pent-1-enyl]-5-oxocyclopentyljsulfanylpropylsulfanyl] acetic acid; methyl 4-[2-[(IR,2R,3R)-3-hydroxy-2- [(E,3 S)-3 -hydroxy-4- [3 - (methoxymethyl)phenyl]but- 1 -enyl]-5 - oxocyclopentyl]ethylsulfanyl]butanoate; 16-(3-Methoxymethyl)phenyl-ro-tetranor-5-thiaPGE; 5- {3-[(2S)-2- {(3R)-3-hydroxy-4-[3-(trifluoromethyl)phenyl]butyl} -5-oxopyrrolidin- 1 - yl]propyl]thiophene-2-carboxylate; [4'-[3-butyl-5-oxo-I-(2-trifluoromethylphenyl)-1,5-dihydro-[1,2,4]triazol-4-ylmethyl]-biphenyl-2-sulfonic acid(3-methyl-thiophene-2-carbonyl)-amide]; and ((Z)-7-{(IR,4S,5R)-5-[(E)-5-(3-chloro-benzo[b]thiophene-2-yl)-3-hydroxy-pent-I-enyl]-4-hydroxy-3,3-dimethyl-2-oxo-cyclopentyl}-hept-5-enoic acid), and pharmaceutically acceptable salts of any of these agents.

A "cyclic AMP (cAMP) enhancer," refers to a molecule that produces or causes a greater amount of cAMP in a cell, or a greater amount of cAMP activity in a cell, or any other relevant component of a cAMP related signal transduction pathway, or a measurable downstream physiological response or effect of a cAMP signaling pathway, as compared to no agent or a control molecule/composition. In a particular embodiment, the agent having dmPGE₂ activity is a cAMP analogue or enhancer.

The cAMP enhancers of the present invention typically increases or maintains the intracellular levels and/or activity of cAMP. Most generally, cyclic adenosine monophosphate (cAMP, cyclic AMP or 3'-5'-cyclic adenosine monophosphate) acts as an important secondary messenger in many biological processes. Secondary messenger systems relate to methods of cellular signaling, whereby a diffusible signaling molecule is rapidly produced/secreted upon a certain activation signal, which can then activate effector proteins within the cell to exert a cellular response. For instance, among other responses, cAMP signaling transfers the effects of prostaglandins, which otherwise cannot pass through the cell membrane. cAMP also regulates the passage of Ca²⁺ through ion channels.

Measurable downstream effects may include greater stem cell viability, proliferation or expansion, and self-renewal and engraftment, among others apparent from the understanding in the art and the description herein. cAMP enhancers may include "agonists," which typically bind to a receptor or other molecule of a cell and trigger a response by the cell, and "antagonists," which typically act against and block/inhibit an action, such as by blocking the degradation of cAMP (*e.g*., blocking a phosphodiesterase). Also contemplated are cAMP analogues.

cAMP activity can also be negatively regulated by a variety of mechanisms. For instance, the Gα-s subunit slowly catalyzes the hydrolysis of GTP to GDP, which in turn deactivates the Gₛ protein, thereby shutting off the cAMP pathway. The cAMP pathway may also be deactivated downstream by directly inhibiting adenylyl cyclase or by dephosphorylating the proteins phosphorylated by PKA. Adenylyl cyclase, and thus cAMP production, may be inhibited by agonists of adenylyl cyclase inhibitory G (Gᵢ)-protein coupled receptors. cAMP decomposition into AMP is catalyzed by the enzyme phosphodiesterase, which may also act as a negative regulator of cAMP signaling.

Illustrative examples of molecules that inhibit cAMP pathway include, for example cAMP phosphodiesterase, which dephosphorylates cAMP into AMP, reducing the cAMP levels; Gᵢ protein, which inhibits adenylyl cyclase, thereby reducing cAMP levels; and pertussis toxin, which decrease cAMP levels.

The cAMP enhancers of the invention are typically capable of activating the cAMP pathway at any of the stages in that pathway, or may prevent the negative regulation (e.g., degradation) of cAMP, and include chemicals, polypeptides, antibodies, and other molecules having such functional effects. Exemplary molecules or agents that activate cAMP pathway may include, for instance, cholera toxin, which increases cAMP levels; forskolin, a diterpine natural product that activates adenylyl cyclase; and caffeine and theophylline, which inhibit cAMP phosphodiesterase, leading to an activation of G proteins that then activate the cAMP pathway.

Illustrative examples of cAMP enhancers include, but are not limited to phorbol ester, forskolin, sclareline, 8-bromo-cAMP, cholera toxin (CTx), aminophylline, 2,4 dinitrophenol (DNP), norepinephrine, epinephrine, isoproterenol, isobutylmethylxanthine (IBMX), caffeine, theophylline (dimethylxanthine), dopamine, rolipram, iloprost, prostaglandin E₁, prostaglandin E₂, pituitary adenylate cyclase activating polypeptide (PACAP), and vasoactive intestinal polypeptide (VIP), among others known in the art. As exemplified above, examples of cAMP enhancers also include cAMP and analogues of cAMP, such sp-5,6-DCI-BIMPS (BIMPS) and dibutyryl cAMP (dbcAMP), among others.

cAMP is implicated in the growth and/or survival of hematopoietic stem cells in culture (*see*, *e.g.,* Negrotto et al., Experimental Hematology 34:1420-1428, 2006, herein incorporated by reference in its entirety). For instance, it was observed that two different cAMP analogues, such as dibutyryl-cAMP and BIMPS, promote survival of human umbilical cord-derived CD34⁺ cells by suppressing apoptosis induced by either nitric oxide (NO) or serum deprivation. Involvement of PKA and PI3K pathway was demonstrated by the ability of their specific inhibitors Rp-cAMP and Wortmannin or LY294002, respectively, to reverse the antiapoptotic effect of BIMPS. While thrombopoietin (TPO), granulocyte colony-stimulating factor (G-CSF), or stem cell factor (SCF) did not increase cAMP levels, the antiapoptotic activity exerted by these growth factors was blocked by inhibition of the adenylate cyclase and synergized by BIMPS. Thus, cyclic AMP analogues suppress the decreased colony formation in cells exposed to NO or serum deprivation, showing that cAMP appears to be not only a key pathway controlling CD34⁺ survival, but also a mediator of TPO, G-CSF, and SCF-mediated cytoprotection.

Likewise, activation of cAMP, such as by injection of isoproterenol (which stimulates adenylyl cyclase) or dibutyryl cyclic adenosine 3',5'-monophosphate shortly after marrow cell graft, almost immediately triggers the transplanted stem cells into entering S phase by inducing DNA synthesis (*see*, *e.g.,* Necas et al., Cell Proliferation, 9:223-230, 2008, herein incorporated by reference in its entirety).

A "Gα-s activator or activating agent" or "G-protein alpha-s activator or activating agent" includes any molecule capable of activating the alpha subunit of the stimulatory G-protein ("Gα-s") or variants of Gα-s. Illustrative examples of Gα-s activators include PGE₂ and agonists and derivatives thereof, and cholera toxin. In a particular embodiment, the agent having dmPGE₂ activity is a Gα-s activator.

Therefore, compositions of the invention that comprise PGE₂ and agents that have dmPGE₂ activity, e.g., dmPGE analogues, cAMP analogues or enhancers, and/or Gα-s activators can be utilized to preserve or maintain cell viability, and increase the engraftment, homing, self-renewal and/or expansion of hematopoietic stem cells *in vivo.*

Accordingly, in particular embodiments, engraftment/engraftment potential/ and or *in vivo* expansion of hematopoietic stem/progenitor cells is increased by contacting the cells *ex vivo* or *in vitro* with PGE₂ and analogues thereof (e.g., dmPGE₂), and agents that have dmPGE₂ activity in any particular combination, without limitation.

In particular embodiments, a population of cells is treated (e.g., contacted) with one or more agents, each at a final concentration of about 1 µM to about 100 µM. In certain embodiments, a population of cells is treated with one or more pharmaceutical agents, each at a final concentration of about 1 x 10⁻¹⁴ M to about 1 x 10⁻³ M, about 1 x 10⁻¹³ M to about 1 x 10⁻⁴ M, about 1 x 10⁻¹² M to about 1 x 10⁻⁵ M, about 1 x 10⁻¹¹ M to about 1 x 10⁻⁴ M, about 1 x 10⁻¹¹ M to about 1 x 10⁻⁵ M, about 1 x 10⁻¹⁰ M to about 1 x 10⁻⁴ M, about 1 x 10⁻¹⁰ M to about 1 x 10⁻⁵ M, about 1 x 10⁻⁹ M to about 1 x 10⁻⁴ M, about 1 x 10⁻⁹ M to about 1 x 10⁻⁵ M, about 1 x 10⁻⁸ M to about 1 x 10⁻⁴ M, about 1 x 10⁻⁷ M to about 1 x 10⁻⁴ M, about 1 x 10⁻⁶ M to about 1 x 10⁻⁴ M, or any intervening ranges of final concentrations.

In another particular embodiment, a population of cells is treated with one or more agents, each at a final concentration of about 1 x 10⁻¹⁴ M, about 1 x 10⁻¹³ M, about 1 x 10⁻¹² M, about 1 x 10⁻¹⁰ M, about 1 x 10⁻⁹ M, about 1 x 10⁻⁸ M, about 1 x 10⁻⁷ M to about 1 x 10⁻⁶ M, about 1 x 10⁻⁵ M, about 1 x 10⁻⁴ M, about 1 x 10⁻³ M, or any intervening final concentration. In treatments comprising one or more agents, the agonists can be at different concentrations from each other or at the same concentration.

In particular embodiments, a population of cells is treated (e.g., contacted with one or more agents) 1, 2, 3, 4, 5, 6, 7, 8, 9 or 10 or more times. A population of cells can be intermittently, episodically, or sequentially contacted with one or more agents within the same vessel (e.g., contacting the population of cells with one drug for a period of time, exchanging the culture medium and/or washing the population of cells, then repeating the cycle with the same or a different combination of pharmaceutical agents for the same predetermined period of time or a different predetermined period of time).

In preferred embodiments, a population of cells is treated with a PGE₂R₂ or PGE₂R₄ agonist, e.g., 16,16-dimethyl PGE₂, at a final concentration of about 10 µM for 2 hours at about 37°C.

Exemplary treatment durations generally include a treatment time of about 1 hour, about 2, hours, or about 3 hours.

In particular embodiments, agents useful in the invention can be transferred from a first vessel to a second vessel, wherein the second vessel is 2 ml vial with a teflon cap that is endotoxin free and is suitable for storage or *ex vivo* administration of the agent, wherein the agent is 16,16-dimethyl PGE₂ at a stock concentration of about 10mM, provided in dimethyl sulfoxide (DMSO) that is substantially free of methyl acetate, and wherein there is an air overlay in the vial. Preferably, the entire composition, including the vessel and the solvent, is sterile and endotoxin-free.

In certain embodiments, the second or other vessel may comprise cells including hematopoietic stem or progenitor cells, such as bone marrow cells, peripheral blood cells, or umbilical cord cells. Accordingly, these and other embodiments may involve transferring the composition from the first or initial vessel to a second vessel that is suitable for *ex vivo* treatment conditions and that comprises hematopoietic stem or progenitor cells in a suitable medium. Alternatively, the population of human cells may be transferred to the first or second vessel that already contains the composition, such as a PE bag or tube, and which is already suitable for *ex vivo* treatment or incubation conditions.

### E. Administration-Ready Compositions of the Invention

The therapeutic compositions of the invention are sterile, and are suitable and ready for administration (*i.e.,* can be administered without any further processing) to human patients. In some embodiments, the therapeutic composition is ready for infusion into a patient. As used herein, the terms "administration-ready," "ready for administration" or "ready for infusion" refer to a cell based composition of the invention that does not require any further treatment or manipulations prior to transplant or administration to a subject.

The sterile, therapeutically acceptable compositions suitable for administration to a patient may comprise one or more pharmaceutically acceptable carriers (additives) and/or diluents (*e.g*., pharmaceutically acceptable medium, for example, cell culture medium), or other pharmaceutically acceptable components. Pharmaceutically acceptable carriers and/or diluents are determined in part by the particular composition being administered, as well as by the particular method used to administer the therapeutic composition. Accordingly, there is a wide variety of suitable formulations of therapeutic compositions of the present invention (see*, e.g.,* Remington's Pharmaceutical Sciences, 17th ed. 1985)).

In particular embodiments, therapeutic cell compositions comprising hematopoietic stem and/or progenitor cells comprise a pharmaceutically acceptable cell culture medium. A therapeutic composition comprising a cell-based composition of the present invention can be administered separately by enteral or parenteral administration methods or in combination with other suitable compounds to effect the desired treatment goals.

The pharmaceutically acceptable carrier and/or diluent must be of sufficiently high purity and of sufficiently low toxicity to render it suitable for administration to the human subject being treated. It further should maintain or increase the stability of the therapeutic composition. The pharmaceutically acceptable carrier can be liquid or solid and is selected, with the planned manner of administration in mind, to provide for the desired bulk, consistency, etc., when combined with other components of the therapeutic composition of the invention. For example, the pharmaceutically acceptable carrier can be, without limitation, a binding agent (*e.g.*, pregelatinized maize starch, polyvinylpyrrolidone or hydroxypropyl methylcellulose, etc.), a filler (e.g., lactose and other sugars, microcrystalline cellulose, pectin, gelatin, calcium sulfate, ethyl cellulose, polyacrylates, calcium hydrogen phosphate, etc.), a lubricant (*e.g*., magnesium stearate, talc, silica, colloidal silicon dioxide, stearic acid, metallic stearates, hydrogenated vegetable oils, corn starch, polyethylene glycols, sodium benzoate, sodium acetate, etc.), a disintegrant (*e.g*., starch, sodium starch glycolate, etc.), or a wetting agent (*e.g*., sodium lauryl sulfate, etc.). Other suitable pharmaceutically acceptable carriers for the compositions of the present invention include, but are not limited to, water, salt solutions, alcohols, polyethylene glycols, gelatins, amyloses, magnesium stearates, talcs, silicic acids, viscous paraffins, hydroxymethylcelluloses, polyvinylpyrrolidones and the like.

Such carrier solutions also can contain buffers, diluents and other suitable additives. The term "buffer" as used herein refers to a solution or liquid whose chemical makeup neutralizes acids or bases without a significant change in pH. Examples of buffers envisioned by the invention include, but are not limited to, Dulbecco's phosphate buffered saline (PBS), Ringer's solution, 5% dextrose in water (D5W), normal/physiologic saline (0.9% NaCl).

These pharmaceutically acceptable carriers and/or diluents may be present in amounts sufficient to maintain a pH of the therapeutic composition of between about 3 and about 10. As such, the buffering agent may be as much as about 5% on a weight to weight basis of the total composition. Electrolytes such as, but not limited to, sodium chloride and potassium chloride may also be included in the therapeutic composition.

In one aspect, the pH of the therapeutic composition is in the range from about 4 to about 10. Alternatively, the pH of the therapeutic composition is in the range from about 5 to about 9, from about 6 to about 9, or from about 6.5 to about 8. In another embodiment, the therapeutic composition comprises a buffer having a pH in one of said pH ranges. In another embodiment, the therapeutic composition has a pH of about 7. Alternatively, the therapeutic composition has a pH in a range from about 6.8 to about 7.4. In still another embodiment, the therapeutic composition has a pH of about 7.4.

The sterile composition of the invention may be a sterile solution or suspension in a nontoxic pharmaceutically acceptable medium. The term "suspension" as used herein may refer to non-adherent conditions in which cells are not attached to a solid support. For example, cells maintained in suspension may be stirred and are not adhered to a support, such as a culture dish.

A suspension is a dispersion (mixture) in which a finely-divided species is combined with another species, with the former being so finely divided and mixed that it doesn't rapidly settle out. A suspension may be prepared using a vehicle such as a liquid medium, including a solution. In particular embodiments, the therapeutic composition of the invention is a suspension, where the hematopoietic stem and/or progenitor cells are dispersed within an acceptable liquid medium or solution, e.g., saline or serum-free medium, and are not attached to a solid support. In everyday life, the most common suspensions are those of solids in liquid water. Among the acceptable diluents, e.g., vehicles and solvents, that may be employed are water, Ringer's solution, isotonic sodium chloride (saline) solution, and serum-free cell culture medium. In some embodiments, hypertonic solutions are employed in making suspensions. In addition, sterile, fixed oils are conventionally employed as a solvent or suspending medium. For parenteral application, particularly suitable vehicles consist of solutions, preferably oily or aqueous solutions, as well as suspensions, emulsions, or implants. Aqueous suspensions may contain substances which increase the viscosity of the suspension and include, for example, sodium carboxymethyl cellulose, sorbitol and/or dextran. In some embodiments, the infusion solution is isotonic to subject tissues. In some embodiments, the infusion solution is hypertonic to subject tissues.

The pharmaceutically acceptable carrier, diluents, and other components comprising the administration-ready therapeutic composition of the invention are derived from U.S. Pharmaceutical grade reagents that will permit the therapeutic composition to be used in clinical regimens. Typically, these finished reagents, including any medium, solution, or other pharmaceutically acceptable carriers and/or diluents, are sterilized in a manner conventional in the art, such as filter sterilized, and are tested for various undesired contaminants, such as mycoplasma, endotoxin, or virus contamination, prior to use. The pharmaceutically acceptable carrier in one embodiment is substantially free of natural proteins of human or animal origin, and suitable for storing the population of cells of the therapeutic composition, including hematopoietic stem and progenitor cells. The therapeutic composition is intended to be administered into a human patient, and thus is substantially free of cell culture components such as bovine serum albumin, horse serum, and fetal bovine serum.

The invention also contemplates, in part, the use of a pharmaceutically acceptable cell culture medium in particular compositions and/or cultures of the present invention. Such compositions are suitable for administration to human subjects. Generally speaking, any medium that supports the maintenance, growth, and/or health of the desired reprogrammed and/or programmed cells of the invention are suitable for use as a pharmaceutical cell culture medium. In particular embodiments, the pharmaceutically acceptable cell culture medium is a serum free medium.

The therapeutic composition may comprise serum-free medium suitable for storing the population of cells comprising the composition. Serum-free medium has several advantages over serum containing medium, including a simplified and better defined composition, a reduced degree of contaminants, elimination of a potential source of infectious agents, and lower cost. In various embodiments, the serum-free medium is animal-free, and may optionally be protein-free. Optionally, the medium may contain biopharmaceutically acceptable recombinant proteins. "Animal-free" medium refers to medium wherein the components are derived from non-animal sources. Recombinant proteins replace native animal proteins in animal-free medium and the nutrients are obtained from synthetic, plant or microbial sources. Protein-free medium, in contrast, is defined as substantially free of protein.

The serum-free medium employed in the present invention is a formulation suitable for use in human therapeutic protocols and products. One serum-free media is QBSF-60 (Quality Biological, Inc.), as described in U.S. Pat. No. 5,945,337. QBSF-60 isoptimized with U.S. Pharmaceutical grade components and is composed of the basal medium IMDM plus 2 mM L-glutamine, 100 U/ml penicillin, 100 µg/ml streptomycin, human injectable grade serum albumin (4 mg/ml) (Alpha Therapeutic Corporation), partially iron saturated human transferrin (300 µg/ml) (Sigma Chemical Corporation or Bayer Corporation) and human recombinant sodium insulin (0.48 U/ml) (Sigma). Other serum-free media known in the art include, but are not limited to: Life Technologies Catalogue StemPro-34 serum free culture media; Capmany, et al., Short-term, serum-free, static culture of cord blood-derived CD34+ cells: effects of FLT3-L and MIP-1α on in vitro expansion of hematopoietic progenitor cells, Haematologica 84:675-682 (1999); Daley, J P, et al., Ex vivo expansion of human hematopoietic progenitor cells in serum-free StemProTM-34 Medium, Focus 18(3):62-67; Life Technologies Catalogue information on AIM V serum free culture media; BioWhittaker Catalogue information on X-VIVO 10 serum free culture media; 5,397,706 entitled Serum-free basal and culture medium for hematopoietic and leukemia cells; no cell proliferation; Kurtzberg *et al.,* 18:153-4 (2000); Kurtzberg et al., Exp Hematol 26(4):288-98 (April 1998).

One having ordinary skill in the art would appreciate that the above example of medium is illustrative and in no way limits the formulation of media suitable for use in the present invention and that there are many such media known and available to those in the art.

In various embodiments, the therapeutic composition of the invention comprises a sterile solution of human serum albumin (HSA), such as 5% HSA, and low molecular weight (LMW) dextran.

The therapeutic composition is substantially free of mycoplasm, endotoxin, and microbial contamination. In particular embodiments, the therapeutic composition contains less than about 10, 5, 4, 3, 2, 1, 0.1, 0.05 µg/ml bovine serum albumin.

By "substantially free" with respect to endotoxin is meant that there is less endotoxin per dose of cells than is allowed by the FDA for a biologic, which is a total endotoxin of 5 EU/kg body weight per day, which for an average 70 kg person is 350 EU per total dose of cells.

With respect to mycoplasma and microbial contamination, "substantially free" as used herein means a negative reading for the generally accepted tests known to those skilled in the art. For example, mycoplasm contamination is determined by subculturing a sample of the therapeutic composition in broth medium and distributed over agar plates on day 1, 3, 7, and 14 at 37°C with appropriate positive and negative controls. The sample appearance is compared microscopically, at 100×, to that of the positive and negative control. Additionally, inoculation of an indicator cell culture is incubated for 3 and 5 days and examined at 600× for the presence of mycoplasmas by epifluorescence microscopy using a DNA-binding fluorochrome. The sample is considered satisfactory if the agar and/or the broth media procedure and the indicator cell culture procedure show no evidence of mycoplasma contamination.

The therapeutic compositions of the invention are HLA typed and may be matched or partially matched to a specific patient for transplantation. HLA-type refers to the unique set of proteins called human leukocyte antigens. These proteins are present on each individual's cells and allow the immune system to recognize 'self from 'foreign'. Administration of cells or tissues that are recognized as foreign can lead to compatibility problems such as immuno-rejection or graft versus host disease (GVHD). Accordingly, HLA type and matching is particularly important in organ and tissue transplantation.

There are six major HLAs (HLA-A, HLA-B, HLA-C, HLA-DR, HLA-DP, and HLA-DQ). Each HLA antigen has multiple isoforms in the human population, and each individual can have two different isoforms for each HLA due to the diploid nature of our genome. Therefore, a complete match would match twelve out of twelve isoforms. A cell or tissue donated from the same individual as, or an identical twin of, the intended recipient would have a perfect HLA-type and is referred to as syngeneic or autologous. It is also understood that certain factors including but not limited to ethnic background and race correlate with certain HLA-types.

Many major and minor HLA isoforms exist and it is understood that a suitable match may include a match between a subset of the major HLAs, all the major HLAs, some or all major and minor HLAs or any combination known to the art that mitigates immuno-rejection or GVDH. It is also understood that specific guidelines for what constitutes a good HLA-type match depends on many factors. Therefore, judgment must be made by one skilled in the art to assess the suitability of a given cell or tissue sample for transplant into a given individual.

HLA-type can be determined using so-called low resolution methods, for example by sero-typing, or using antibody based methods. Sero-typing is based on antibody recognition of HLA-types. Sero-typing can distinguish between 28 different HLA-A genes, 59 HLA-B genes and 21 HLA-C genes. A perfect match by sero-typing methods would be a so-called six out of six match referring to the two alleles for each HLA (A,B, and C) present in each individual. In certain cases, a five out of six match or less may be considered a good match as determined by one skilled in the art.

Other low or medium resolution methods to determine HLA-type examine the HLA isoforms of the individual, but do not rely on determining the actual sequence of an individual's HLA alleles. Often, the donor is related to the individual receiving the sample, in this case sero-typing alone or in combination with other low or medium resolution methods may be sufficient to determine if a sample is suitable for transplantation. In other cases a five out of six or lower match is readily found, but a perfect match is not. In such cases it may be advantageous to use cells or tissues with a lower match rather than expend time and effort to find a better HLA-type match.

High resolution methods involve examining the specific sequence of the HLA genes or gene expression products (protein or RNA). High resolution methods can distinguish between thousands of different isoforms.

At a minimum, HLA typing of the therapeutic composition is performed for six HLA loci, HLA-A, -B, and -DR, for example, at low resolution/split antigen level.

DNA-based testing methods can be utilized for HLA-DR typing. DNA-based testing may be used for HLA-A and -B. Transplant center guidelines for typing of patient, family and to confirm the HLA types of potential unrelated donors include, typing HLA-A, B, and -DR loci using primarily DNA-based testing methods at allele level resolution for DRBI and low resolution/split antigen level for HLA-A and -B. The typing of a patient and the selected donor can be performed using the same set of reagents, methodology, and interpretation criteria with fresh tissue samples to ensure HLA identity. Quality assurance and quality control for HLA testing are complied with.

In various embodiments, the population of cells comprises haplotyped hematopoietic stem or progenitor cells. In some embodiments, the population of cells comprising the therapeutic composition is HLA typed based on HLA-A, HLA-B, HLA-C, and HLA-DRB1. In particular embodiments, the population of cells is HLA typed based on the group consisting of HLA-DRB3/4/5, HLA-DQB1, and DPB1. In some embodiments, the population of cells comprising the therapeutic composition is matched with a specific human patient. In some embodiments, the population of HLA haplotyped cells has 4 out of 6 HLA matches with a specific human subject. HLA matching may be based on alleles or antigens, and combinations thereof. In some embodiments, the population of HLA haplotyped cells is a partial mismatch with a specific human subject, such as the subject to which the therapeutic composition is administered.

The therapeutic composition of the invention is capable of obtaining product licensure from the FDA *(i.e.,* FDA approval) and other health authorities in other countries and regulatory territories, as well as product labeling with characterizing information regarding product indication, product efficacy, safety and purity. FDA licensure is likely to be based on cell dose and HLA mismatch. The therapeutic composition of the invention, in some embodiments, is processed and cryopreserved according to accredited standards, sterile, and labeled for, e.g., RH and ABO typing, HLA typing and the A, B, and DR-beta-1 loci, and post-processing counts, CD34⁺ counts, CFU-GM counts, infectious disease screening, family history and evidence of maternal consent for donation. The therapeutic composition to be used for transplant would include cells that match a minimum of 4/6 antigens or 3/6 alleles, and a cell dose as described herein.

### F. Smart Bio-Vessels

In various embodiments, the invention contemplates, in part, methods of cell therapy, *e.g*., hematopoietic stem/progenitor cell transplants, that comprise contacting a population of cells with one or more pharmaceutical agents in an endotoxin-free vessel as described herein, under conditions sufficient to increase the engraftment and/or *in vivo* expansion of the contacted cells in a subject and administering the contacted cells to the subject.

The invention further contemplates, in part, methods to increase stem cell engraftment in a subject (e.g., a human) that comprise contacting a population of cells that comprises hematopoietic stem and/or progenitor cells (e.g., bone marrow cells, peripheral blood cells, and/or umbilical cord blood cells) with agents that increase CXCR4 expression in the hematopoietic stem and/or progenitor cells, such as PGE₂, dmPGE₂, or agents that have dmPGE₂ activity, in an endotoxin-free vessel as described herein, under conditions sufficient to increase the engraftment of the contacted cells in a subject and administering the contacted cells to the subject.

The invention further contemplates, in part, methods to increase the number of hematopoietic stem or progenitor cells in a subject (e.g., a human) that comprise contacting a population of cells that comprises hematopoietic stem and/or progenitor cells (*e.g*., bone marrow cells, peripheral blood cells, and/or umbilical cord blood cells) with agents that increase CXCR4 expression in the hematopoietic stem and/or progenitor cells, such as PGE₂ or an analogue thereof, *e.g*., 16,16-dimethyl PGE₂ (dmPGE₂) or an agent having dmPGE₂ activity in an endotoxin-free vessel as described herein, under conditions sufficient to increase the *in vivo* expansion of the contacted cells in a subject and administering the contacted cells to the subject.

In various illustrative embodiments, the invention provides, in part, a method of contacting hematopoietic stem or progenitor the cells with PGE₂ or an analogue thereof, e.g., 16,16-dimethyl PGE₂ (dmPGE₂) or an agent having dmPGE₂ activity in an endotoxin-free vessel as described herein, under conditions sufficient to maintain stem/progenitor cell viability and increase engraftment, homing, and expansion *in vivo.*

In one embodiment, the invention provides, in part, a method of preparing a population of cells, e.g., bone marrow cells, mobilized peripheral blood cells, umbilical cord blood cells, for a transplant, *e.g*., bone marrow transplant, that comprises contacting the cells with dmPGE2 or an agent having dmPGE₂ activity in an endotoxin-free vessel as described herein, under conditions sufficient to increase the engraftment and/or *in vivo* expansion of the contacted cells in a subject compared to non-contacted cells.

In a particular embodiment, the invention contemplates, a method of increasing hematopoietic stem or progenitor cell engraftment in a subject that comprises administering to the subject, a source or population of cells contacted with dmPGE₂ or an agent having dmPGE₂ activity in an endotoxin-free vessel as described herein, under conditions sufficient to increase the engraftment of the contacted cells in a subject compared to non-contacted cells.

In another particular embodiment, the invention contemplates, a method of treating a subject in need of a hematopoietic stem/progenitor cell transplant that comprises: selecting the subject in need of a hematopoietic stem/progenitor cell transplant and administering to a subject, a population of cells contacted with dmPGE₂ or an agent having dmPGE₂ activity in an endotoxin-free vessel as described herein, under conditions sufficient to increase the engraftment and/or *in vivo* expansion of the contacted cells in a subject compared to non-contacted cells.

As used herein, the term "vessel" relates generally to any item capable of being used for purposes of culturing, handling, manipulating, storing, analyzing, incubating, administering and otherwise establishing, supporting, harvesting, and populations of cells comprising hematopoietic stem or progenitor cells and by-products thereof *ex vivo* or *in vitro* or otherwise for a variety of purposes as set forth and as contemplated herein.

Illustrative embodiments of vessels include, but are not limited to: bags *(e.g.,* intravenous (IV) bags; cell culture bags, *e.g.,* VueLife™, KryoSure™, KryoVue™, Lifecell®, PermaLife™, X-Fold™, Si-Culture™, VectraCell™), bioreactors, cell or tissue culture devices, pouches, capsules, culture vials, apparatuses, cell factories, containers, culture tubes (*e.g.*, microcentrifuge tubes, EPPENDORF TUBES®, FALCON® conical tubes, etc.), culture dishes (*e.g*., Petri dishes), culture flasks, spinner flasks, roller bottles, multi-well plates (*e.g.*, 2-well, 4-well, 6-well, 12-well, 24-well, 48 well, 96-well, and 384-well plates), micro-incubators, micro-carriers, microplates, microslide and chamber slides, and implant devices (*e.g*., collagen sponges). The vessel may be used multiple times or may be a single-use vessel.

Preferably, vessels of the present invention are endotoxin free, and manufactured according to GMP practices as discussed elsewhere herein.

In particular embodiments, vessels can be fabricated from materials that comprise one or more of the following characteristics: gas permeability (materials have suitable gas transfer rates for oxygen, carbon dioxide and nitrogen); negligible water loss rates (materials are practically impermeable to water); chemically and biologically inert (materials do not react with the vessel contents), and retention of flexibility and strength in various conditions (materials enable vessel to be microwaved, treated with UV irradiation, centrifuged, or used within a broad range of temperatures, e.g., from -100°C to +100°C).

Those skilled in the relevant art can select appropriate polymeric materials with the desired permeability, bioreactive and biocompatible properties, temperature resistance, flexibility, heat conductivity, and strength.

Illustrative materials that are suitable for fabricating vessels of the present invention include, but are not limited to: glass, ceramics, metals, thermoset and elastomer monomers and polymers, and monomeric and polymeric thermoplastics. Exemplary thermoplastic materials suitable for fabricating vessels of the present invention include, without limitation: acetal resins, delrin, fluorocarbons, polyesters, polyester elastomers, metallocenes, polyamides, nylon, polyvinyl chloride, polybutadienes, silicone resins, ABS (an acronym for "acrylonitrile, butadiene, styrene"), polycarbonate (also referred to in the plastics industry as "PC") polypropylene, polyethylene, polystyrene, liquid crystal polymers, alloys and combinations and mixtures and composites thereof, and reinforced alloys and combinations and mixtures and composites thereof.

In particular embodiments, vessels can be fabricated from one or more materials selected from the group consisting of: diethylhexyl phthalate, polyvinylchloride, polyethylene, polypropylene, and fluorinated ethylene propylene.

In various illustrative embodiments, a vessel can be fabricated to have a cross-sectional wall thickness that is based upon and that is an implicit function of the selected materials and the intended applications. Exemplary cross-sectional wall thicknesses include, without limitation, thicknesses between about 0.25 mm and about 2.0 mm, between about .5 mm and about 1.5 mm, and between about .75 mm and about 1.25 mm In particular embodiments, the cross-sectional wall thickness of a vessels is at least .2 mm, .3 mm, .4 mm, .5 mm, .6 mm, .7 mm, .8 mm, .9 mm, 1.0 mm, 1.1 mm, 1.2 mm, 1.3 mm, 1.4 mm, 1.5 mm, 1.6 mm, 1.7 mm, 1.8 mm, 1.9 mm, or 2.0 mm or any intervening thickness.

In particular illustrative embodiments, vessels of the present invention are designed to accommodate specific volumes. Exemplary volumes of the vessels of the present invention include, without limitation, volumes of about 10 mL, about 25 mL, about 50 mL, about 75 mL, about 100 mL, about 150 mL, about 250 mL, about 500 mL, about 750 mL, about 1000 mL, about 1250 mL, about 1500 mL, about 1750 mL, about 2000 mL, or more, including any intervening volume. For example, intervening volumes between 10 mL and 25 mL, include 11 mL, 12 mL, 13 mL, 14 mL, 15 mL, 16 mL, 17 mL, 18 mL, 19 mL, 20 mL, 21 mL, 22 mL, 23 mL, and 24 mL.

In certain embodiments, a vessel contemplated herein comprises 1, 2, 3, 4, or 5 compartments. The compartments can be the same size or different sizes and may have the same or different porosities. In one embodiment, the porosity of adjacent compartments is such that small molecules, nutrients, polypeptides, and/or growth factors may freely be exchanged between compartments, but wherein the cells of each compartment are restricted to their respective compartments.

One having skill in the relevant art can fabricate a vessel having the desired thickness, volume, and number of compartments based on knowledge of the fabrication materials and the intended uses of the vessel.

In particular embodiments, vessels can be fabricated from materials that accommodate particular coatings, films, or other agents, e.g., a PGE₂ or an agent having dmPGE₂ activity, or suitable combination thereof.

The interior surface of the vessel can be designed to accommodate coating with various hydrophobic, hydrophilic, or amphipathic molecules using methods known to those in the relevant art. For example, commonly used hydrophobic materials such as for example, thermoset materials, elastomers, rubbers, and thermoplastics such as polystyrenes, polycarbonates, ABSs, and other polymeric materials disclosed herein accommodate binding of hydrophobic molecules (e.g., proteins having one or more hydrophobic regions). Furthermore, specific experimental, industrial, or clinical applications may require, preclude, or be indifferent to the binding of various types of molecules such as, for example, nucleic acids, proteins, carbohydrates, or the like. It may also be desirable to prevent, minimize, and or maximize binding molecules to the substrate depending upon the objectives of a particular application.

In particular illustrative embodiments, the vessels contemplated herein comprise one or more input and/or output ports for introducing, exchanging, or removing compounds, cells, cell culture medium, and the like. The ports can provide needle-less access or can include access points for needles, as appropriate. Each port may contain one or more adapters (e.g., luer adapters), valves and/or filters.

The present invention contemplates, in part, vessels comprising one or more devices in any suitable combination and configuration that indicate, for example, the temperature of the vessel contents, the time the vessel has been at any given temperature, and various environmental conditions (*e.g*., pH, oxygen concentration, carbon dioxide concentration, glucose concentration). The invention contemplates devices in the form of cards, strips, disks, stickers, labels, probes, sensors, and small electronic devices, and the like. The contemplated devices can be integrated into the material of the vessel or alternatively, can be manufactured separately from the vessel and subsequently, permanently or non-permanently affixed or adhered to the outer surface of the vessel.

In one embodiment, a vessel comprises a temperature indicating device. In a preferred embodiment, a vessel comprises both a temperature indicating device and an elapsed time indicating device, either separately as individual devices or combined as a single device. In further embodiments, the vessel comprises a temperature indicating device, an elapsed time indicating device and one or more devices that indicate an environmental condition. The plurality of devices may be fabricated separately as individual devices or fabricated as a single device.

As used herein, the term "temperature indicating device" refers to a device that senses, measures, and/or indicates a temperature of the vessel and/or vessel contents and that comprises one or a plurality of "temperature indicators." As used herein, the term "temperature indicator" refers to an indicator that produces a signal that indicates a temperature. The temperature indicator can produce a signal that corresponds to the real-time temperature or exposure to a particular temperature for any predetermined length of time. In particular embodiments, the temperature indicator is reversible. In other embodiments, the one or more temperature indicators irreversibly indicate that the vessel and/or vessel contents have reached a predetermined temperature or have experienced a predetermined temperature for a particular length of time. In further embodiments, a temperature indicating device comprises one or more reversible and irreversible temperature indicators in any number or combination.

The temperature indicating device can be user-activated, or activated by exposure to a predetermined temperature. As used herein, the term "predetermined temperature" refers to a temperature or temperature range selected for monitoring by the user. In various embodiments, the predetermined temperature is a "target temperature" that indicates the desired temperature or temperature range for which the vessel is contemplated for use. In various embodiments, a temperature indicating device provides temperature indicators that indicate one or more predetermined temperatures, *i.e.,* temperatures of the vessel and/or vessel contents at, above, or below a target temperature, or above, below or within a target temperature range. The present invention contemplates a variety of target temperatures and temperature ranges, without limitation.

Various types of signals are contemplated for use in temperature indicators of the present invention. For example, temperature indicators can indicate temperature by producing a visual signal (*e.g*., a digital display, a color change, a graph), an audible signal, an infrared signal, a radio signal, an analogue signal, a digital signal, or combinations thereof. For example, the temperature indicating device can include one or more of: a liquid crystal display (LCD) to indicate temperature; a voice or speech synthesizer to indicate temperature or to specify that an item is below or exceeds a predetermined target temperature; an analogue, infrared, or radio signal that indicates the temperature via sound, ultra-sonic or other waves; and/or a digital signal that indicates the temperature electronically.

Temperature indicators that produce visual indications of temperature can produce a signal that comprises a color that corresponds to a temperature of the vessel and/or vessel contents that are at, above, or below a target temperature, or above, below or within a target temperature range. The present invention contemplates that any color combinations can be used with any number and combination of temperature indicators, without limitation. One having skill in the art could employ any number of chemicals which reflect certain colors at certain temperatures and could select such chemical substances to reflect a desired color to correspond to a particular temperature.

In additional illustrative embodiments, temperature indicating devices can comprise one or a plurality of temperature scales each comprising one or more temperature indicators that indicate a predetermined temperature or temperature range. Each temperature indicator within a temperature scale can produce a signal (*e.g*., visual signal, digital signal).

Temperature scales illustratively include temperature indicators in increments of 1°C, 2°C, 3°C, 4°C, 5°C, 6°C, 7°C, 8°C, 9°C, 10°C, over ranges of 5°C, 10°C, 15°C, 20°C, 25°C, 30°C, 35°C, 40°C, 45°C, 50°C, 55°C, 60°C, 65°C, 70°C, 75°C, 80°C, 85°C, 90°C, 95°C, 100°C, 110°C, 125°C, 130°C, 140°C, 150°C, 160°C, 170°C, 180°C, 190°C, or 200°C or more.

Illustrative temperature ranges include, without limitation, ranges of about 4°C to about 65°C, about 4°C to about 50°C, about 4°C to about 42°C, about 4°C to about 37°C or any intervening ranges of temperatures.

The present invention contemplates, without limitation, that a temperature indicating device can comprise one or more temperature scales, each scale comprising any number and combination of temperature indicators, in any increments over any temperature range to indicate a predetermined temperature or temperature range.

In one embodiment, the temperature indicating device comprises a plurality of reversible temperature indicators each associated with a specific temperature range and one or more irreversible temperature indicators that indicate when one or a plurality of predetermined temperatures have been reached or experienced for any predetermined length of time. The reversible indicators individually provide visual indications of the temperature in real time. The visual indications of temperature correspond to the temperature of the vessel and/or vessels contents that are at, above, or below a target temperature, or above, below or within a target temperature range. An irreversible indicator maintains a visual indication once the predetermined temperature has been reached.

The present invention further contemplates a vessels comprising one or more temperature indicating devices and one or more elapsed time indicating devices, or devices that indicate various environmental conditions.

As used herein, the term "elapsed time indicating device" refers to a device comprising one or a plurality of "elapsed time indicators." As used herein, the term "elapsed time indicator" refers to an indicator that measure, monitors, and indicates when a predetermined length of time has elapsed. Each elapsed time indicator can be independently user-activated or activated by exposure to a particular predetermined temperature or temperature range.

Once activated, an elapsed time indicator indicates the time from activation. In one embodiment, a given elapsed time indicator measures a predetermined amount of time and then produces a signal that indicates when the predetermined amount of time has elapsed. In various illustrative embodiments, an elapsed time indicating device comprises 1, 2, 3, 4, 5, or more elapsed time indicators, each being individually capable of independent activation by a user or by exposure to the same or a different predetermined temperature or temperature range.

In various embodiments, the present invention contemplates, in part, a vessel comprising an elapsed time indicator that indicates the time the vessel and/or contents have been exposed to, experienced, or maintained at one or a plurality of predetermined temperatures or temperature ranges. In a related embodiment, the elapsed time indicator indicates a predetermined amount of time the vessels and/or contents have been exposed to, experienced, or maintained at one or a plurality of predetermined temperatures or temperature ranges. The elapsed time indicator can produce a continuous signal or one or a plurality of signals at points at which a predetermined percentage of the total predetermined time has elapsed, e.g., produces a signal at regular intervals of the total elapsed time to be measured.

In one embodiment, an elapsed time indicator produces a signal at regular intervals of the total elapsed time to be measured. For example, if the elapsed time indicator is designed to measure and indicate a total elapsed time of one hour, the indicator can produce a signal to indicate the point at which 15 minutes, 30 minutes, 45 minutes, and one hour have elapsed. Exemplary regular intervals include 1 minute intervals, 2 minute intervals, 5 minute intervals, 10 minute intervals, 15 minute intervals, 20 minute intervals, 30 minute intervals, 45 minute intervals, 60 minute intervals, 90 minute intervals, 120 minute intervals or more. Exemplary numbers of regular intervals within any total elapsed time period include 1, 2, 3, 4, 5, 6, 7, 8, 9, 10 or more intervals.

In another embodiment, an elapsed time indicator produces a continuous signal indicating the amount of time that has elapsed or that has yet to elapse (*i.e.,* the time remaining). For example, an elapsed time indicator that is designed to measure and indicate a total elapsed time of one hour may be in the form of a progress bar, pie chart, or clock, wherein a signal is produced upon activation and increases linearly compared to the fraction of total elapsed time that has elapsed. In one embodiment, once the total time has elapsed the elapsed time indicator can discontinue producing the signal or produce a different signal to indicate that the total time has elapsed.

In a particular illustrative embodiment, the elapsed time indicating device comprises one or a plurality of any combination of elapsed time indicators. Each elapsed time indicator can be independently activated by a user or activated by exposure to a different predetermined temperature. In addition, each elapsed time indicator can measure and produce a signal for different predetermined lengths of time.

Merely for purposes of illustration, a single elapsed time indicating device may comprise: an elapsed time indicator that is user-activated and measures and indicates an elapsed time of 1 hour; an elapsed time indicator that is user-activated and measures and indicates an elapsed time of 2 hours; an elapsed time indicator that is user-activated and measures and indicates an elapsed time of 10 minutes; an elapsed time indicator that is activated by exposure to a predetermined temperature of 4°C and measures and indicates an elapsed time of 1 hour, 2 hours, or more since activation; and/or an elapsed time indicator that is activated by exposure to a predetermined temperature in the range of 30°C-40°C, preferably 37°C and measures and indicates an elapsed time of 1 hours, 2 hours, or more since activation.

Exemplary types of signals produced by elapsed time indicators include, but are not limited to visual signals, audible signals, infrared signals, radio signals, digital signals, analogue signals, and combinations thereof.

The present invention further contemplates, in part, vessels comprising an indicating device comprising one or more environmental indicators that measure, monitor, and indicate, for example, the pH, carbon dioxide concentration, oxygen concentration, osmolarity, or glucose concentration of the vessel's contents. In one embodiment, an environmental indicator continuously monitors and produces a signal that indicates the environmental condition. In another embodiment, an environmental indicator monitors and produces a signal at one or more predetermined times and/or at regular intervals. The signal is preferably a visual signal, more preferably an alphanumeric signal produced on an LED, OLED, or LCD.

In various embodiments, environmental indicators comprise digital sensors that measure, monitor, and indicate the environmental conditions in the vessel. In addition, it is contemplated that the sensors can be calibrated to measure any range of environmental conditions, without limitation. Thus, a normal range for each environmental condition can be pre-programmed into each environmental indicating device. The range can include one or more of a minimum threshold, optimal condition, or maximum threshold. In a particular embodiment, when the environmental condition being measured or monitored is outside of the minimum or maximum threshold values, the environmental indicator will produce an audible signal to indicate that the environmental condition is not within an acceptable range.

Other exemplary environmental indicators include, but are not limited to, Ca⁺⁺, potassium, sodium, magnesium, manganese, sulfate, phosphate, and chloride concentration.

The present invention, contemplates, in part, vessels comprising a combination of indicating devices that indicate, for example, the temperature of the vessel contents, the time the vessel has been at any given temperature, and optionally, one or more various environmental conditions (e.g., pH, oxygen concentration, glucose concentration). In preferred embodiments, the combination devices can include and of the features of individual devices and indicators disclosed herein. One having skill in the art would appreciate that the environmental indicating devices are preferably in contact with the contents of the vessel. The contemplated devices can be integrated into the material of the vessel at a point where the permeability of the vessel is such that the particular environmental sensing device is in contact with the vessel lumen or contents of the vessel. In another embodiment, the environmental indicating devices can be manufactured separately from the vessel and subsequently, permanently or non-permanently affixed or adhered to the outer surface of the vessel. In one related embodiment, the affixed or adhered device perforates the vessel such that the particular environmental sensing device is in contacts with the vessel lumen or contents of the vessel. In another related embodiment, the affixed or adhered device is applied to a portion of the device that is permeable, such that the particular environmental sensing device is in contact with the vessel lumen or contents of the vessel.

In a preferred embodiment, the vessel comprises a combination of a temperature indicating device and an elapsed time indicating device, either separately as individual devices or as a single device. In further embodiments, the vessel comprises a combination of a temperature indicating device, an elapsed time indicating device and one or more environmental condition indicating devices either separately as individual devices or as a single device.

The combination indicating device can be fabricated integral with the vessel, can be permanently or non-permanently attached to the vessel exterior surface, can be laminated to the vessel exterior surface or can be encased with the vessel within a vessel liner. The device can be a small electronic device, a probe, a sensor, or a combination thereof.

The combination indicating device may be of any size or shape. Exemplary shapes of the combination indicating device, include, but are not limited to: cards, strips, disks, stickers, labels, probes, or combinations thereof. For example a temperature indicating device in the form of a card or strip may comprise one or a plurality of temperature indicating disks or vice versa.

As will be clear to one skilled in the art, various types of graphic design may be employed for visualizing the elapsed time and temperature of the vessel including, but not limited to, various lines, curves, ellipses, rectangles or points. Similarly, indicia showing the progress of the liquid-migration front may also be employed, including but not limited to various arrows, curves, lines and points of different sizes. For example, the signal can be viewed in a continuous window as progress on a progress bar or in a plurality of windows visible at particular intervals of elapsed time for particular temperatures or a range of temperatures. Furthermore, each embodiment may be adapted to include numerous additional indicia to show the status of the time indicator or temperature of the vessel. Such indicia include graphic symbols showing the gradual advance to the total elapsed time versus temperature.

In a more preferred embodiment, the vessel comprises a combination of a temperature indicating device and an elapsed time indicating device.

As used herein, the singular forms "a," "an" and "the" include plural references unless the content clearly dictates otherwise.

Throughout this specification, unless the context requires otherwise, the words "comprise", "comprises" and "comprising" will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that no other elements are optional and may or may not be present depending upon whether or not they affect the activity or action of the listed elements.

Reference throughout this specification to "one embodiment" or "an embodiment" means that a particular feature, structure or characteristic described in connection with the embodiment is included in at least one embodiment of the present invention. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

### EXAMPLES

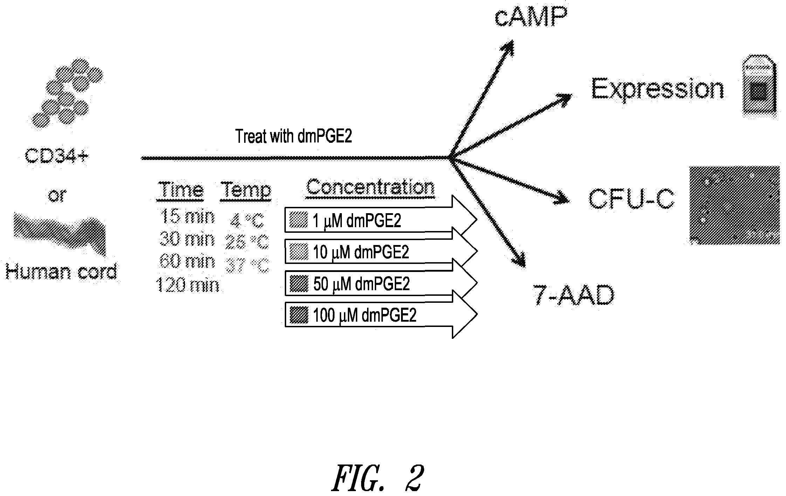

The analysis of several biological parameters of populations of hematopoietic stem and progenitor cells treated with agents that stimulate the prostaglandin pathway in order to develop clinical methods to increase the engraftment potential and expansion of the cells was conducted (See Figure 2). The results of these experiments and the results for Phase1b clinical trials are described below.

### EXAMPLE 1

### COMPETITIVE CAMP ASSAYS

The cAMP assay was performed on CD34⁺ cells using the LANCE® cAMP detection kit (Perkin Elmer Inc., Waltham, MA) according to the manufacturer's instructions. Briefly, 3,000 CD34⁺ cells (Stem Cell Technologies, Vancouver, Canada) were aliquoted in each well of a 384-well opaque white plate in the recommended stimulation buffer. Assays were performed in triplicate for all conditions.

CD34⁺ cells were placed on ice prior to being stimulated with DMSO or 16,16-dimethyl PGE₂ at 4°C. For assays conducted at other temperatures, *e.g*., 25°C or 37°C, DMSO or 16,16-dimethyl PGE₂ was added to CD34⁺ cells at room temperature and then the plates were incubated with DMSO or 16,16-dmPGE₂ at the experimental temperature (25°C or 37°C).

CD34⁺ cells were incubated for periods of 5, 15, 30, 60 or 120 minutes. Following the incubation period, detection buffer was added to the stimulated cells and cells were incubated for an additional 1 hour at room temperature in detection buffer, regardless of stimulation temperature. The assay plates were analyzed using an EnVision® 2104 Multilabel Reader (Perkin Elmer) according to the manufacturer's instructions.

A competitive cAMP assay was performed using CD34⁺ cells to determine the effects of time (incubation for 15, 30, 60, or 120 minutes), temperature (incubation at 4°C, 25°C, or 37°C), and final 16,16-dimethyl PGE₂ concentration (1 µM, 10 µM, 50 µM, or 100 µM) on cAMP production in the cells.

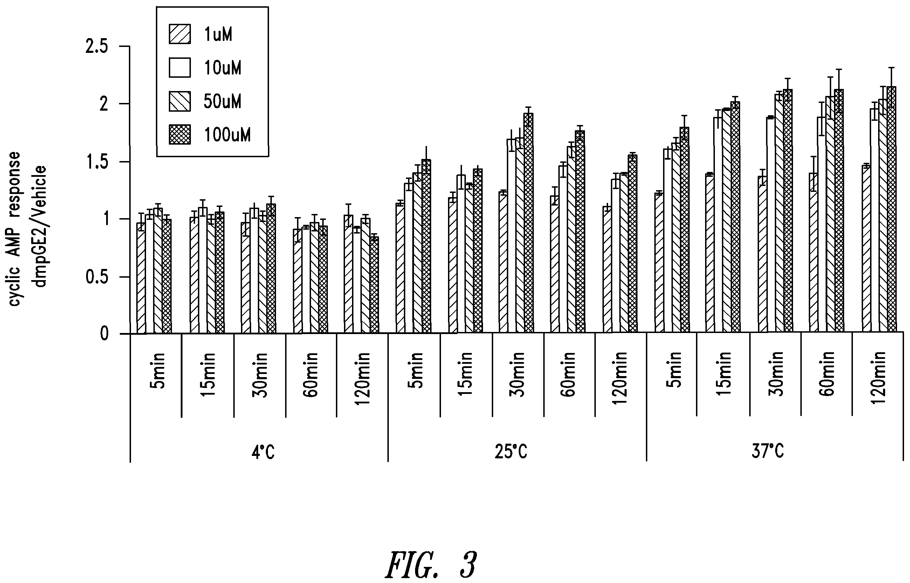

The results showed that the maximal cAMP response occurred around 30-60 minutes of incubation; that higher incubation temperatures resulted in more robust cAMP activity; and that the cAMP response was relatively dose-insensitive above a threshold concentration of 10 µM (See Figure 3). A statistically significant increase in cAMP activity was observed when CD34⁺ cells were incubated with 16,16-dimethyl PGE₂ at 37°C and 25°C compared to DMSO control, for durations as short as 5 min and up to 2 hours, at all concentrations of 16,16-dimethyl PGE₂ tested (1, 10, 50 and 100µM) (p<0.001). No statistically significant increase in cAMP production was observed in CD34⁺ cells incubated 16,16-dimethyl PGE₂ at 4°C compared to DMSO controls, under similar conditions of time and across all concentrations tested (1, 10, 50 and 100µM) (p>0.05 for all samples).

Incubation at higher temperatures for a longer duration of time, which conditions were previously believed to be associated with a decrease in CD34⁺ cell viability and 16,16-dimethyl PGE₂ half-life, showed superior stimulation of cAMP without negatively affecting cell viability. Further, as described herein, treatment of cells with 16,16-dimethyl PGE₂ at 4°C for shorter durations of time resulted in increased production of cAMP but did not result in increased expression of genes believed to be important in regulating stem cell homing, survival, proliferation, and engraftment. Upregulation of such genes, and thus achievement of the most robust biological response, required treatment of cells with 16,16-dimethyl PGE₂ at physiologically relevant temperatures, such as 37°C, for increased durations of time of at least about one hour.

### EXAMPLE 2

### GENE EXPRESSION

### Whole Genome Expression Arrays

Human umbilical cord blood and pre-isolated CD34⁺ cells from human umbilical cord blood were purchased from Stem Cell Technologies Inc. (Vancouver, BC, Canada). Cells were incubated in either low molecular weight dextran with 5% human serum albumin media (LMD/5% HSA) or Stem Span media (Stem Cells Technology Inc.) for *ex vivo* treatment with 16,16-dimethyl PGE₂. Total RNA was isolated from incubated cells using a Pico Pure RNA Isolation Kit (Molecular Devices, Sunnyvale, CA).

Biotinylated amplified RNA (aRNA) was prepared using the standard protocol for MessageAmp II aRNA Amplification Kit (Applied Biosystems/Ambion, Austin, TX) and the optional Second Round Amplification; the copy RNA (cRNA) was transcribed into biotin labeled aRNA using MessageAmp II Biotin Enhanced Kit (Applied Biosystems/Ambion, Austin, TX) according to the manufacturer's instructions. Biotin labeled aRNA was purified and fragmented according to the Applied Biosystems protocols. 20µg of fragmented aRNA was hybridized to Human Genome U133 Plus 2.0 GeneChips (Affymetrix Inc., Santa Clara, CA) for 16 hrs at 45°C.

The GeneChips were washed and stained in the Affymetrix Fluidics Station 450 and scanned using the Affymetrix GeneChip Scanner 3000 7G. The image data were analyzed using Affymetrix Expression Console software and default analysis settings. GeneChip expression data were normalized by log scale robust multi-array analysis (RMA) and visualized in Spotfire for Genomics 3.1 (Tibco Spotfire, Palo Alto, CA). Pathway analysis was performed in MetaCore. (GeneGo, St. Joseph, MI).

GeneChip technology was used to determine the effects of time (incubation for 5, 15, 20, 30, 40, 60, 80, 100, 120, 180, or 240 minutes), temperature (incubation at 4°C, 25°C, or 37°C), and final 16,16-dimethyl PGE₂ concentration (.1 µm, 1 µM, 10 µM, 50 µM, or 100 µM) on cellular gene expression.

### Microfluidic qPCR using the Fluidigm platform

Real-time PCR transcript quantitation from 16,16-dimethyl PGE₂ *ex vivo* treated CD34+ cell samples was performed using the BioMark Dynamic Array microfluidics system (Fluidigm Corporation, South San Francisco, CA, USA). Total RNA was isolated from treated cells using Pico Pure RNA Isolation Kit (Molecular Devices, Sunnyvale, CA, USA). Complimentary DNA (cDNA) was reverse transcribed from 50 ng of isolated total RNA using the High-Capacity cDNA Reverse Transcription Kit (Life Technologies Corporation, Carlsbad, CA, USA).

cDNA was pre-amplified for specific target genes (96) using a 200 nM mixture of 96 gene specific primer pairs (see Table 1), including 6 reference control genes using the TaqMan PreAmp Master Mix Kit (Life Technologies) protocol. Specific target amplification (STA) from cDNA was performed using 14 cycles of amplification with the standard cycling conditions using the manufacturer's protocol. EvaGreen dye (Biotium, Inc. Hayward, CA, USA) was added according to Fluidigm's EvaGreen protocol to detect amplified products. For samples, the reaction mix contained 3.0 µL Gene Expression Master Mix (Life Tech.), 0.3 µL Sample Loading Buffer (Fluidigm), 0.3 µL 20X EvaGreen dye (Biotium, Inc.), 1.5 µL diluted (1:5 sterile nH2O) STA cDNA, and 0.9 µL sterile diH2O for loading into the sample inlets of the 96.96 Dynamic Array (Fluidigm).

Samples were run in replicates, from 5 to 9 wells. For primer pairs, the reaction mix contained 2.5 µL Gene Specific Primer pairs (20µM) and 2.5 µL Assay Loading Buffer (Fluidigm) for loading into the assay inlets on the 96.96 Dynamic Array (Fluidigm). 96.96 Dynamic arrays were loaded using a NanoFlex IFC Controller HX (Fluidigm) and real-time reactions were performed using a BioMark Real-Time PCR System (Fluidigm).

Results were analyzed using BioMark Real-Time PCR Analysis software. Average Cts were calculated from the sample replicates and delta-delta Cts (ΔΔCt) were calculated using the mean of 6 reference genes (ACTB, GAPDH, HPRT1, QARS, ARPC2 and LRIG2) against a vehicle only sample. Cts above 28 or amplified products with inappropriate melting curve properties were excluded from the calculations. Results are displayed in Spotfire for Genomics 3.1 (Tibco Spotfire, Palo Alto, CA, USA) in heat map format or as Excel graphic plots (Microsoft Corp., Redmond, WA, USA).

**Table 1: Primer Pairs**

| **Well Position** | **Name** | **Sequence** | **Name** | **Sequence** |
|---|---|---|---|---|
| A1 | AREG-F | CGGCTCAGGCCATTA TGC | AREG-R | GGTCCCCAGAAAATGGTT CA |
| A2 | AREGB-F | TCTCCACTCGCTCTTC CAACAC | AREGB-R | ATCAAGAGCGACAGCAC CACTG |
| A3 | ATP6V0 A4-F | TGGACGACCATGGAG AAGAGTTC | ATP6V0A4 -R | ACTCGATGGTGTGGATG GCTTG |
| A4 | AKAP12-F | CAGAAACAAGAGAGA GAATCTGCAA | AKAP12-R | TGTCTTCACATTCTGGTCT TCCA |
| A5 | ADCY7-F | GCACTGGAGAACTTG GGAAAAT | ADCY7-R | GCATTCACAAGAGTACCC GAGG |
| A6 | CCND1-F | CTTCCTGTCCTACTAC CGCCTC | CCND1-R | CTTGACTCCAGCAGGGCT TC |
| A7 | C6orf17 6-F | TCGGACACACACACA CACACAC | C6orf176-R | AGCAACTTCGGACTCAGA CCTC |
| A8 | CA2-F | GATGACTCTCAGGAC AAAGCAGTG | CA2-R | AACCTTGTCCATCAAGTG AACCC |
| A9 | CA4-F | AAGGTCGTCTGGACT GTGTTCC | CA4-R | CTGAGAGAATGCCAGGA TCTGTTC |
| A10 | CREB5-F | AAGACTGCCCAATAA CAGCCAT | CREB5-R | GACAGGACTAGCAGGAG GGCTA |
| A11 | COL1A1-F | TGCGATGACGTGATC TGTGACG | COL1A1-R | TTTCTTGGTCGGTGGGTG ACTCTG |
| A12 | CREM-F | AAGAAGCAACACGCA AACGA | CREM-R | TTCTTTCTTCTTCCTGCGA CACT |
| B1 | CXCL1-F | CGGAAAGCTTGCCTC AATCCTG | CXCL1-R | CAGTTGGATTTGTCACTG TTCAGC |
| B2 | CXCL2-F | AAACCGAAGTCATAG CCACACTC | CXCL2-R | AGCCACCAATAAGCTTCC TCCTTC |
| B3 | CXCL5-F | AGACCACGCAAGGAG TTCATCC | CXCL5-R | TCTTCAGGGAGGCTACCA CTTC |
| B4 | CXCL6-F | AGCTTGAGTTTCCTGC CAGTCG | CXCL6-R | TTTCCTCGTGCCTTCTGCA CTC |
| B5 | CXCR4-F | TCCTGGCTTTCTTCGC CTGTTG | CXCR4-R | TGAAGGAGTCGATGCTG ATCCC |
| B6 | DUSP2-F | AACCAGATGGTGGAG ATCAGTGC | DUSP2-R | CCGCTGTTCTTCACCCAG TCAATG |
| B7 | DUSP4-F | TGCATCCCAGTGGAA GATAACCAC | DUSP4-R | GCATCGATGTACTCTATG GCTTCC |
| B8 | ECEL1-F | GACTTCCTGCTGAAAC CCGATG | ECEL1-R | GGTCTTCTCATGGACCTC AAACTC |
| B9 | EDN1-F | ATTTGGGTCAACACTC CCGAGCAC | EDN1-R | TCACGGTCTGTTGCCTTT GTGG |
| B10 | ETV3-F | ATGAAAGCCGGCTGT AGCATCG | ETV3-R | CAGGAAACTGATACCCTC CACCTC |
| B11 | GULP1-F | GCAGCAGATTTCCCTC CAGATA | GULP1-R | TGTCTAACGGGTCGAGA CAAAA |
| B12 | FGF9-F | TCGGTGTGGGCATTG TCTCTTG | FGF9-R | ACTGGATGCAAACCCATG AGCTG |
| C1 | FGFR1-F | ATACCAGCTGGATGT CGTGGAG | FGFR1-R | ACATGAACTCCACGTTGC TACCC |
| C2 | FU2735 2-F | TCACCGGCTTTCTTGC CATCTG | FU27352-R | TTCTTATCCCGGTTGCGG TCTG |
| C3 | FOS-F | TACACTCCAAGCGGA GACAGAC | FOS-R | GTTGGCAATCTCGGTCTG CAAAG |
| C4 | FOSL2-F | GCAGTTGGGTTTCTG GCTTGAG | FOSL2-R | TCCTGCTACTCCTGGCTC ATTC |
| C5 | FOXA1-F | TCCTCAGGAATTGCCC TCAAGAAC | FOXA1-R | ATGACATGACCATGGCAC TCTGC |
| C6 | GEM-F | GCCGAGAAGTGTCTG TATCAGAAG | GEM-R | CTGTCGCACAATGCCCTC AAAC |
| C7 | GNAL-F | AGAATCGACAGCGTC AGCTTGG | GNAL-R | TGGCCACCAACATCAAAC ATGTGG |
| C8 | HAS1-F | GCCTGGTACAACCAG AAGTTCC | HAS1-R | ACCTGGAGGTGTACTTG GTAGC |
| C9 | HOMER 1-F | AGAAGCTGCTCGACT AGCAAAGG | HOMER1-R | GCGGATTCCTGTGAAGG TGTACTG |
| C10 | HR-F | AGGACCAAGAGCATC AAAGAGGAG | HR-R | TGTATTCGCTCATGGCCC AAGC |
| C11 | IL11-F | AGCGGACAGGGAAG GGTTAAAG | IL11-R | GGCGGCAAACACAGTTC ATGTC |
| C12 | INHBA-F | TCACGTTTGCCGAGTC AGGAAC | INHBA-R | TGACAGGTCACTGCCTTC CTTG |
| D1 | JAG1-F | TGGGCCCGACTGCAG AATAAAC | JAG1-R | ATCCACACAGGTCGCTCC AAAG |
| D2 | JOSD1-F | TCCAGGACAGCAATG CCTTCAC | JOSD1-R | CATGGTGTTTGGAGACA ACCTCTG |
| D3 | KCTD20-F | TCTAGGTCCCAGGAA TGAAGACC | KCTD20-R | GGAACTGAAGATTTGCT GGCTGAG |
| D4 | KIAA119 9-F | TTGGCCTCCTTGTCAA GTCTGG | KIAA1199-R | ATCCAGAAGGTGGACAC AGCATTG |
| D5 | LGALS12 -F | CGGGAATGAGGAAGT GAAGGTGAG | LGALS12-R | AGCGTGTCCACATGAGA CAGTG |
| D6 | LIF-F | TGCTTCATCCGGCTTA GCTTGG | LIF-R | AGTTTGTCTTTCTCGAAG CCCATC |
| D7 | LONRF2-F | AGGGTCACAGCCACA TGAATGC | LONRF2-R | AATTCTCCGAGCTCCCTG CATC |
| D8 | LXN-F | ACAAGTTAAGGTGAA CTGCACAGC | LXN-R | TGCAGTTTCTTGTCCCGT TGAAGG |
| D9 | MALT1-F | TGGTCACAGCTGGAT GTTTGCG | MALT1-R | TCAGAGACGCCATCAACA CTTCTC |
| D10 | MPPE1-F | TGGCTGACACCCATTT GCTTGG | MPPE1-R | TCTCCATCTGCCATTCCCT TCG |
| D11 | MYOM2 -F | TGACCATCATGGAAG GGAAGACC | MYOM2-R | CTGGATGTCCTGGTCGTT CTTG |
| D12 | NPTX1-F | ATCAGCGAGCTCGAG AAAGGTC | NPTX1-R | TAGTTGGTCCGCAGTGG GAATG |
| E1 | NR4A2-F | GCTGTTGGGATGGTC AAAGAAGTG | NR4A2-R | TCTTCGGTTTCGAGGGCA AACG |
| E2 | NR4A3-F | TGCGTCCAAGCCCAA TATAGCC | NR4A3-R | TGTATGTCTGCGCCGCAT AACTG |
| E3 | PLAT(2)-F | GTGTGCTGGAGACAC TCGGA | PLAT(2)-R | CATCGTTCAGACACACCA GGG |
| E4 | NTRK1-F | AGCACCGACTATTACC GTGTGG | NTRK1-R | TCGGTGGTGAACTTACG GTACAGG |
| E5 | OSM-F | TGCCTGTCGGTTGCTT GGATTC | OSM-R | TGCACCACCTGTCCTGAT TTACAG |
| E6 | PCDH8-F | TCATCAACCACATGCA GAGTGGAC | PCDH8-R | AAGGTTGACATCTGGGCT GGTG |
| E7 | PDE4A-F | TCCACAACATTCCTGG ACAAACAG | PDE4A-R | TTCCTTCATCGTGGGTGA TGGG |
| E8 | PDE4B-F | ACAAGTTCAGGCGTT CTTCTCC | PDE4B-R | CCATGTTGCGAAGGACCT GAATG |
| E9 | PDE4D-F | TTGTGACTCCATTTGC TCAGGTC | PDE4D-R | GATGGATGGTTGGTTGC ACATGG |
| E10 | PDLIM3-F | ACAAATGTGGGAGTG GCATAGTCG | PDLIM3-R | ACTCAGGGTGCCGGTACT TATC |
| E11 | PLAT-F | TCTCAGATTTCGTGTG CCAGTGC | PLAT-R | GCACGTGGCCCTGGTATC TATTTC |
| E12 | PLAUR-F | ATCGTGCGCTTGTGG GAAGAAG | PLAUR-R | ACCCACACACAACCTCGG TAAG |
| F1 | PLK2-F | TGGAGGAGAACCTCA TGGATGG | PLK2-R | TTAGCCACTGAAGGAGG TAGAGC |
| F2 | PPARD-F | TCCTTCCAGCAGCTAC ACAGAC | PPARD-R | ATCTGCAGTTGGTCCAGC AGTG |
| F3 | RASD1-F | AGGCTTCAAGAAACC GTCATGC | RASD1-R | ACAGCAACCCGGAATCA CAGAC |
| F4 | PTGER2-F | TCCTGGCTATCATGAC CATCAC | PTGER2-R | TTCGGGAAGAGGTTTCAT TCAT |
| F5 | REN-F | TGTACCTTTGGTCTCC CGACAG | REN-R | GGGCATTCTCTTGAGGAA GATCCG |
| F6 | RGS1-F | TGCTGCTGAAGTAAT GCAATGGTC | RGS1-R | TGACCAGTTTGGTTGGCA AGAAG |
| F7 | RGS2-F | CAAACAGCAAGCTTT CATCAAGCC | RGS2-R | AAGCCCTGAATGCAGCA AGACC |
| F8 | S1PR1-F | ACGTAGGCTGTGGGA AGATGAAG | S1PR1-R | TGGAAACTTTGGCCTCAG CGAAG |
| F9 | SC5DL-F | AAGCGCCTACATAAA CCTCACC | SC5DL-R | AGCATGACTTGCAAATG GAGTAGG |
| F10 | SGIP1-F | AAGGAGCAGACCCAA GCAAATG | SGIP1-R | GCCAGCAGGAAATGACA ACACC |
| F11 | SGK1-F | AGGAGCCTGAGCTTA TGAATGCC | SGK1-R | TGATTTGCTGAGAAGGA CTTGGTG |
| F12 | SHISA2-F | ACTATCACCCGCTGCT TCTCTG | SHISA2-R | CGCCAAACCATAACCACA AGGC |
| G1 | SIK1-F | ACTCACCGCGCCATGT ATAGTC | SIK1-R | ACAACTGTCAGAGCTGGT TCCC |
| G2 | SSTR1-F | ATGGTGACAGGTGTG AGTCTGG | SSTR1-R | TTGAGTGCTGCTTGCACT CCTG |
| G3 | SV2C-F | TGTCTGCTCTGCTGAT GGACAG | SV2C-R | AAGCACCATAGAGCCAC CTAGC |
| G4 | SYT4-F | CACCAGCCGGGAAGA ATTTGATG | SYT4-R | GTGAAGACCAGGCCAAA TGCAC |
| G5 | SYTL3-F | GAATGAACGACCGCT TGCTTGG | SYTL3-R | CCAACAGCTGTGTCTCCC TTTG |
| G6 | TAC1-F | TACGACAGCGACCAG ATCAAGGAG | TAC1-R | TCCAAAGAACTGCTGAG GCTTGG |
| G7 | THBS1-F | GGCAGACACAGACAA CAATGGG | THBS1-R | TGTCCCGTTCATTGAGGA TACCG |
| G8 | PTGER4-F | TCTTACTCATTGCCAC CTCCCT | PTGER4-R | TGGCTGATATAACTGGTT GACGA |
| G9 | TMCC3-F | TTCAGCCGGTGAGGC TGTTATC | TMCC3-R | GGCAAGGCAATAAACAC AGAGTGG |
| G10 | TNFRSF1 B-F | TGTCCACACGATCCCA ACACAC | TNFRSF1B -R | TGTCACACCCACAATCAG TCCAAC |
| G11 | ULBP2-F | GCTCTCCTTCCATCAA GTCTCTCC | ULBP2-R | GCACAGAAGGATCTTGG TAGCG |
| G12 | VPS37B-F | ATGGTGCAGAAGATG GAGGAGAC | VPS37B-R | GGTCAAGCGTGCTTTCAA CGTG |
| H1 | WT1-F | TGTCCCACTTACAGAT GCACAGC | WT1-R | ACACTGGAATGGTTTCAC ACCTG |
| H2 | YPEL4-F | TCACCGCACTTACAGC TGTGTC | YPEL4-R | ATGGCTCCCTTGGAAGG ACTTG |
| H3 | PDE3B-F | TGATGAAGACGGTGA AGAATTAGA | PDE3B-R | AGGTGGTGCATTAGCTG ACAAA |
| H4 | ZNF331-F | AACAATGGCCCAGGG TTTGGTG | ZNF331-R | TACAGGTCCCTCTGAGCA GAGTTC |
| H5 | TGFB2-F | AGCATGCCCGTATTTA TGGAGT | TGFB2-R | GCAGATGCTTCTGGATTT ATGG |
| H6 | TCF4-F | ATCGAATCACATGGG ACAGATG | TCF4-R | GCTGTTAAGGAAGTGGT CTCTTG |
| H7 | ACTB-F | TGGCCGAGGACTTTG ATTGCAC | ACTB-R | GGACTTCCTGTAACAACG CATCTC |
| H8 | ARPC2-F | AGGTGAACAACCGCA TCATCGAG | ARPC2-R | TACTGCTTCCGGTTTGTTT CCG |
| H9 | GAPDH-F | AGCTCATTTCCTGGTA TGACAACG | GAPDH-R | CTCTTCCTCTTGTGCTCTT GCTG |
| H10 | HPRT1-F | TGCAGACTTTGCTTTC CTTGGTC | HPRT1-R | CAAGCTTGCGACCTTGAC CATC |
| H11 | LRIG2-F | TGGCAACAGCTGACA GAAATGGG | LRIG2-R | ACAAGCAGATGCACACC AGAGC |
| H12 | QARS-F | AGGTTCCCTTTGCACC CATTGTC | QARS-R | TTAAATCCTGGCTCTGGC TCCTC |

### 16,16-dimethyl PGE ₂ Gene Expression Signature

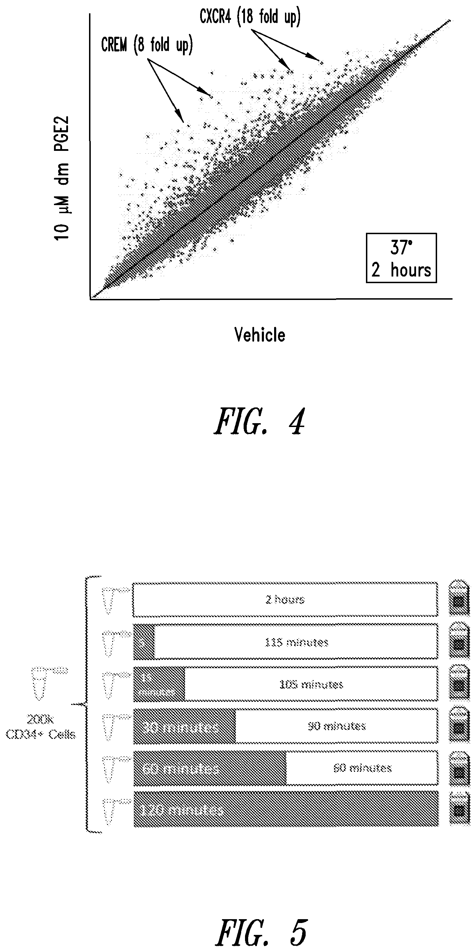

A baseline gene expression signature of CD34⁺ cells treated with 10 µM 16,16-dimethyl PGE₂ for 120 minutes at 37°C compared to CD34⁺ cells treated with vehicle was obtained (see Figure 4). A total of 608 genes were modulated at a statistically significant level (365 upregulated or 243 downregulated) in CD34⁺ cells treated under these conditions. CXCR4, a known mediator of HSC homing to the bone marrow niche through its interaction with SDF-1α was upregulated by 18-fold relative to the DMSO treated control. Also upregulated was CREM, one of many known cAMP-responsive genes. Modulation of gene expression associated with PGE₂ signaling pathways, cell adhesion, and chemokine signaling were also observed. However, no increase in gene expression associated with apoptosis or cell death was observed

The gene expression profile for CD34⁺ cells treated with 10 µM 16,16-dimethyl PGE₂ for 120 minutes at 37°C compared to the DMSO control, distinguish the cells of the invention from other known cells. The inventive methods produce cells which display enhanced or increased engraftment potential/engraftment and increased *in vivo* expansion compared to untreated, control, or cells treated at 4°C. A table of genes in the gene expression signature of the cells of the invention that show high amplitude regulation appears below.

**Table 2: Highly regulated genes in a dmPGE₂ Gene Expression Signature**

| Gene Symbol | Description | Fold change (increases) |
|---|---|---|
| HAS1 | Hyaluronan synthase 1 | 55.83 |
| GEM | GTP-binding protein GEM | 28.18 |
| DUSP4 | Dual specificity protein phosphatase 4 | 25.75 |
| AREG | Amphiregulin | 23.32 |
| NR4A2 | Nuclear receptor related 1 protein | 22.30 |
| REN | Renin | 19.50 |
| CREM | cAMP-responsive element modulator | 12.90 |
| COL1A1 | collagen, type I, alpha 1 | 10.50 |
| FOSL2 | Fos-related antigen 2 | 8.11 |
| CXCR4 | CXC chemokine Receptor 4 | 7.33 |

Accordingly, contrary to pre-clinical protocols, incubation at 37°C, which was previously thought to be associated with a decrease in CD34⁺ cell viability and 16,16-dimethyl PGE₂ half-life, unexpectedly showed an increase in gene expression associated with PGE₂R₂/R4 cell signaling pathways, cell homing, and proliferation. Moreover, no gene expression changes that would indicate a decrease in cell viability were observed.

Further experiments were performed to determine if CD34⁺ cells responded to 16,16-dimethyl PGE₂ when cells were treated in the context of a whole cord blood clinical treatment protocol. Human umbilical cord blood cells were incubated for 120 minutes at 37°C with vehicle or 10 µM 16,16-dimethyl PGE₂ (See Figure 17). After the incubation, Lin(-) CD34⁺ cells were isolated using Miltenyi magnetic sorting. Biotin-labeled aRNA was prepared from the cells, and gene expression profiles were analyzed. Consistent with the results for CD34⁺ cord blood cells incubated with different concentrations of 16,16-dimethyl PGE₂, Lin(-) CD34⁺ cells isolated from human cord blood reproduced the 16,16-dimethyl PGE₂ gene expression signature. aRNA from treated whole cord blood, from Lin(+) CD34⁺ cells, Lin(-) CD34⁺ CD38⁺ cells, and from Lin(-) CD34⁺ CD38- CD90⁺ cells were also prepared. Figure 17 shows that whole cord blood (more than 99% Lineage+ cells) did not respond to 16,16-dimethyl PGE₂ in a similar manner as Lin(-) CD34⁺ cells isolated from whole cord blood or from Lin(+) CD34⁺ cells.

### Time Parameters

Gene expression profiles were analyzed for CD34⁺ + cells incubated with 10 µM 16,16-dimethyl PGE₂ at 37°C for 5, 15, 30, 60, or 120 minutes. For incubation (e.g., treatment) periods less than 120 minutes, the cells were washed in media to remove the 16,16-dimethyl PGE₂ and then the cells were incubated for the remaining period in media to allow time for changes in gene expression to take place (See Figure 5).

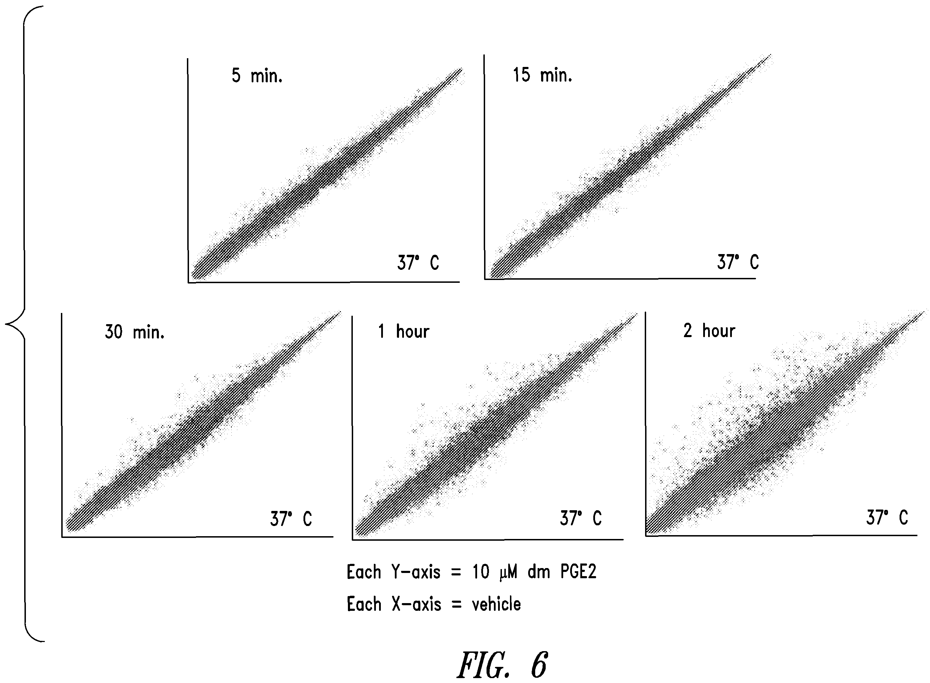

The results showed that longer exposures to 16,16-dimethyl PGE₂ yielded larger magnitudes of gene expression in the 16,16-dimethyl PGE₂ expression signature (See Figure 6). In contrast, very short incubation times (5-15 minutes) resulted in minimal gene expression changes in genes associated with the 16,16-dimethyl PGE₂ expression signature. Gene expression changes were apparent after 30 minutes of incubation with 16,16-dimethyl PGE₂ but continued to increase up to 120 minutes of incubation, despite the fact that all experiments were assessed after a total of 120 minutes of elapsed time.

This is in contrast to previous observations which suggested that short incubation times should be sufficient to load the EP₂/4 receptors with drug prior to transplantation, where it was believed that the downstream biology would take place. The current results demonstrate that longer incubation times (greater than 30 minutes) are required at physiologically relevant temperatures, such as 37°C, to achieve a biological benefit.

A microfluidic qPCR platform was also used to measure expression changes of a 16,16-dimethyl PGE₂ gene expression signature of the genes listed in Table 3 in human CD34⁺ cells at different incubation times. The gene expression analysis includes a 96 well format to detect the genes listed in Table 3.

**Table 3: Signature Genes for Fluidigm assay**

| | | | | | | | |
|---|---|---|---|---|---|---|---|
| ADCY7 | CXCL 1 | FGFR1 | INHBA | MYO M2 | PLAT (1) | SC5D L | THBS1 |
| AKAP1 2 | COL1 A1 | FLJ273 52 | JAG1 | NPTX 1 | PLAT (2) | SGIP1 | TMCC3 |
| AREG | CXCL 2 | FOS | JOSD1 | NR4A 2 | PLAUR | SGK1 | TNFRSF 1B |
| AREGB | CXCL 5 | FOSL2 | KCTD2 0 | NR4A 3 | PLK2 | SHIS A2 | ULBP2 |
| ARPC2 | CXCL 6 | FOXA1 | KIAA11 99 | NTRK 1 | PPAR D | SIK1 | VPS37B |
| ATB6V 0A4 | CXCR 4 | GEM | LGALS 12 | OSM | PTGE R2 | SSTR 1 | WT1 |
| C6orf17 6 | DUSP 2 | GNAL | LIF | PCDH 8 | PTGE R4 | SV2C | YPEL4 |
| CA2 | DUSP 4 | GULP1 | LONRF 2 | PDE3 B | RASD1 | SYT4 | ZNF331 |
| CA4 | ECEL1 | HAS1 | LRIG2 | PDE4 A | REN | SYTL 3 | ACTB |
| CCND1 | EDN1 | HOME R1 | LXN | PDE4 B | RGS1 | TAC1 | GAPDH |
| CREB5 | ETV3 | HR | MALT1 | PDE4 D | RGS2 | TCF4 | HPRT1 |
| CREM | FGF9 | IL11 | MPPE1 | PDLIM 3 | S1PR1 | TGFB 2 | QARS |

Figure 14A shows that suitable gene expression signatures were detectable after at least about 60 minutes of constant exposure to 16,16-dimethyl PGE₂ at 37°C up to at least about 4 hours of constant exposure to 16,16-dimethyl PGE₂ at 37°C. However, maximal gene expression response was observed after at least about two hours of constant exposure to 16,16-dimethyl PGE₂ at 37°C. Figure 14B shows the average gene expression for the signature genes listed in Table 3 and that maximal gene expression changes were observed after at least about two hours at 37°C. Figure 14C shows the expression data for CXCR4, which is responsible for homing to the bone marrow niche. It is interesting to note that the kinetics of the gene expression response are much slower compared to the cAMP response which reached maximal levels in only 15 minutes at 37°C. For the data shown in Figure 14, the gene expression detection reactions for the following group of genes failed and were excluded from this analysis: ARPC2, SSTR1, CXCL5, SYT4, CXCL6, TMCC3, FGF9, GNAL, GULP1, LRIG2, PDE4D, PLAT (1), and PLAT (2). For the data shown in Figure 14, the following control housekeeping genes were used: ACTB, GAPDH, HPRT1, and QARS.

Further, gene expression signatures were measured on cells that received short pulse treatments with 16,16-dimethyl PGE₂ followed by a recovery period in the absence of the drug. Human CD34⁺ cells were incubated for different times in the presence of 16,16-dimethyl PGE₂ followed by a wash and recovery period in media designed to reflect the *in vivo* setting. The experimental design matched the *ex vivo* treatment paradigm where cells are treated, washed to remove the drug and then administered to the patient. CD34⁺ cells were incubated with 16,16-dimethyl PGE₂ at 37°C for 5, 15, 30, 60, or 120 minutes and the gene expression signatures were analyzed. For incubation periods less than 120 minutes, the cells were washed in media to remove the 16,16-dimethyl PGE₂ and then the cells were incubated for the remaining period in media to allow time for gene expression to take place.

Figure 15 shows that short pulse treatments with 16,16-dimethyl PGE₂ (5-15 minutes) are not sufficient to generate a "full" gene expression response. Gene expression changes and recognizable gene expression signatures were only observed after about 30 minutes of incubation with 16,16-dimethyl PGE₂, and were maximal after about 2 hours, which is in contrast to the rapid cAMP response (see Fig. 3). Thus, in particular clinical embodiments, cord blood treated with 16,16-dimethyl PGE₂ under physiological conditions (e.g., 37°C) for 120 minutes achieve a "robust" gene expression signature indicative of improved clinical efficacy of the treated cells.

For the data shown in Figure 15, the gene expression detection reactions for the following group of genes failed and were excluded from this analysis: ADCY7, CCND1, CREB5, GULP1, MPPE1, PDE3B, PTGER2, RGS2, and YPEL4. For the data shown in Figure 15, the following control housekeeping genes were used: ACTB, ARPC2, GAPDH, HPRT1, LRIG2, and QARS.

### 16,16-dimethyl PGE₂ Concentration

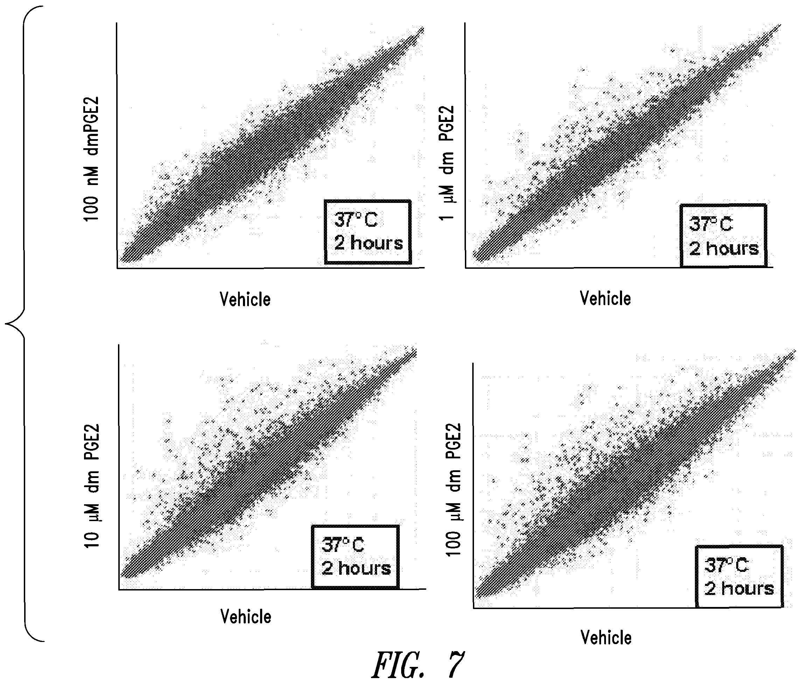

Gene expression profiles were analyzed for CD34⁺ cells incubated for 120 minutes at 37°C with different concentrations of 16,16-dimethyl PGE₂ (vehicle, 100 nM, 1 µM, 10 µM, or 100 µM; see Figure 7). The results showed that the full 16,16-dimethyl PGE₂ gene expression signature was reproduced at 10 µM but that no further substantial changes in the gene expression signature occurred above 10 µM 16,16-dimethyl PGE₂, suggesting that 10 µM is the optimal treatment dose.

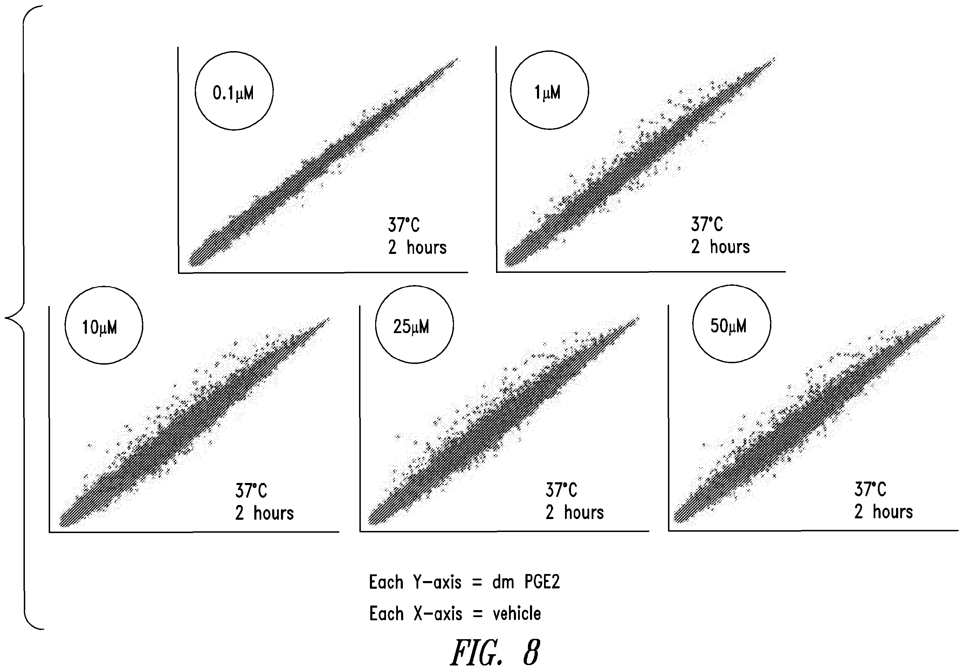

These experiments were repeated using human whole cord blood cells to determine if the CD34⁺ expression results can be translated to the clinical setting. Treating whole cords takes into account (1) increased cellular complexity present in whole cord blood, (2) reduced drug levels due to protein binding of drug, and (3) possible paracrine effects. Human umbilical cord blood cells were incubated for 120 minutes at 37°C with vehicle, 100 nM, 1 µM, 10 µM, 25 µM, or 50 µM 16,16-dimethyl PGE₂ (See Figure 8). After the incubation, Lin(-)CD34⁺ cells were isolated, labeled aRNA was prepared from the cells, and gene expression profiles were analyzed. Consistent with the results for CD34⁺ cells incubated with different concentrations of 16,16-dimethyl PGE₂, Lin(-)CD34⁺ cells isolated from human cord blood reproduced the 16,16-dimethyl PGE₂ gene expression signature at 10 µM, with 10 µM giving the maximal signature and no substantial improvement observed with increased concentration.

A microfluidic qPCR platform was also used to measure expression changes of a 16,16-dimethyl PGE₂ gene expression signature (see Table 3 for genes) in human CD34⁺ cells treated with different concentrations of 16,16-dimethyl PGE₂ (vehicle, 100nM, 1µM, 10µM, 50µM, or 100µM), at 37°C for 2 hours. Figure 16 shows that the 16,16-dimethyl PGE₂ gene expression signature was maximal at 10 µM but that no further substantial changes in the gene expression signature occurred above 10 µM 16,16-dimethyl PGE₂.

For the data shown in Figure 16, the gene expression detection reactions for the following group of genes failed and were excluded from this analysis: ADCY7, CCND1, CREB5, GULP1, FGFR1, FLJ27352, MPPE1, PDE4D, PTGER2, PDE3B, and YPEL4. For the data shown in Figure 16, the following control housekeeping genes were used: ACTB, ARPC2, GAPDH, HPRT1, LRIG2, and QARS.

### Incubation Temperature

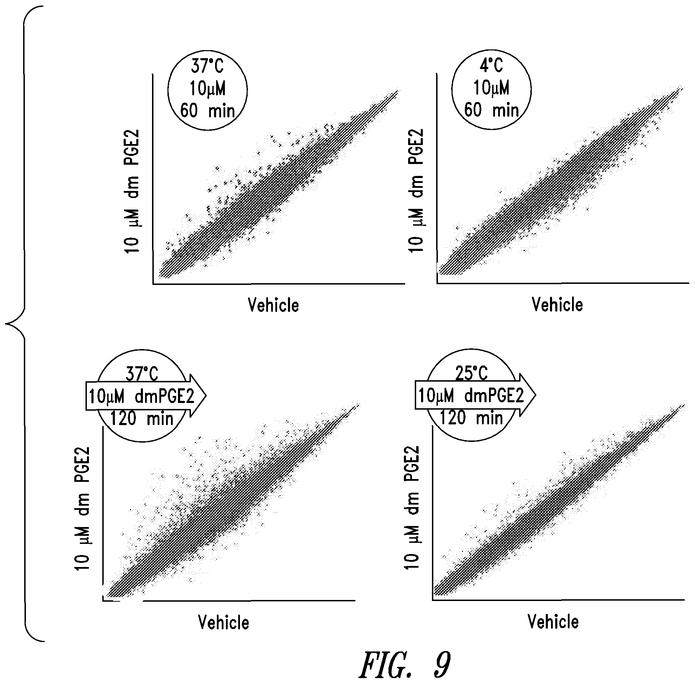

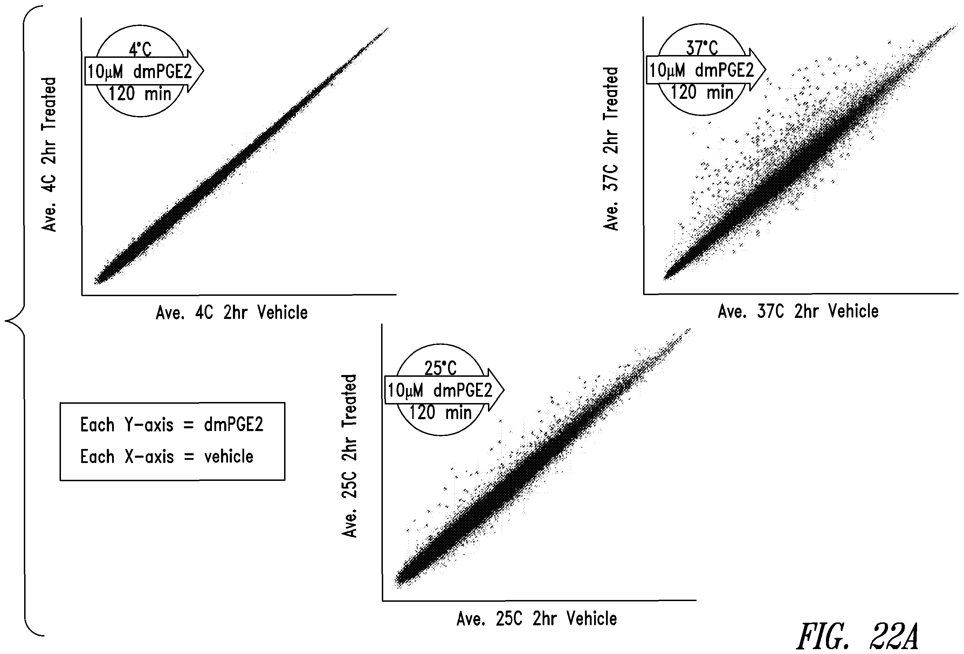

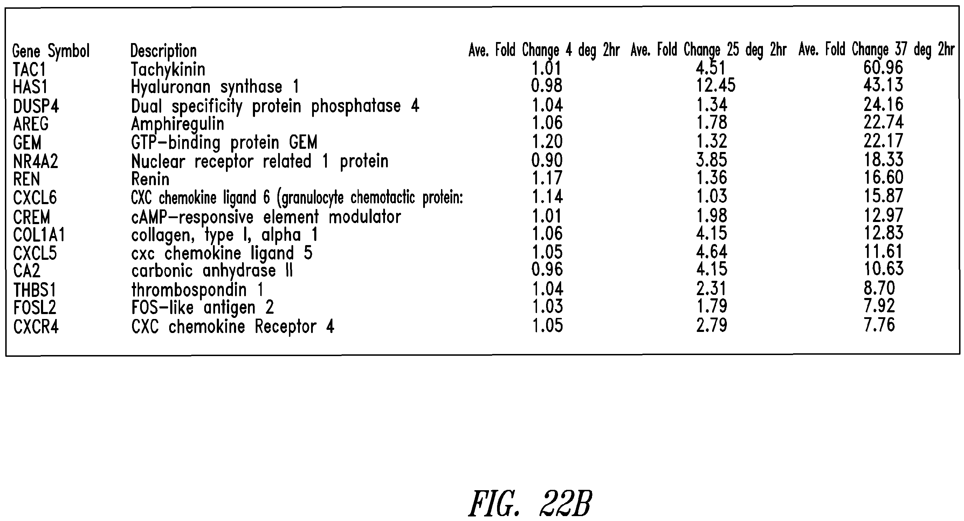

Gene expression profiles were analyzed for CD34⁺ cells incubated with 16,16-dimethyl PGE₂ at 4°C, 25°C, or 37°C (See Figures 9 and 22). These results showed that gene expression changes associated with the 16,16-dimethyl PGE₂ gene expression signature occurred in incubation at 37°C for 60-120 minutes, with the gene expression changes most robust at 120 minutes. In addition, concentration and temperature were covaried, and it was determined that lower temperatures and higher concentrations of 16,16-dimethyl PGE₂ could not be used to replicate the effects of a higher temperature (data not shown). Thus, treatment at 100 µM 16,16-dimethyl PGE₂ at 4 °C and 25 °C yielded smaller gene expression changes than 10 µM 16,16-dimethyl PGE₂ at 37 °C.

Accordingly, the results showed that gene expression in isolated CD34⁺ + cells or Lin(-)CD34⁺ cells in human cord blood resulted in the upregulation of genes involved in HSC homing and engraftment, e.g., CXCR4; cAMP responsive genes, e.g., CREM; and modulation of gene expression associated with PGE₂ signaling pathways, cell adhesion, and chemokine signaling. Significantly more genes had at least a 2-fold increase or decrease in gene expression (t-test p < 0.05 for each gene/probe) after incubation with 10 µM 16,16-dimethyl PGE₂ at 37°C compared to incubation at 25°C or 4°C. In particular, there were statistically significant changes in gene expression in genes associated with hematopoietic stem and progenitor cell homing, e.g., CXCR4 (p = 0.00014), and genes associated with increased PGE₂R₂/R4 cell signaling pathways, e.g., CREM (p = 0.0012), at 37°C compared to incubation at 25°C or 4°C.

Moreover, contrary to pre-clinical expectations, no increase in gene expression of apopotosis or cell death associated genes was observed in cells incubated at 37°C, which was previously thought to be associated with decreased CD34⁺ cell viability and 16,16-dimethyl PGE₂ half-life.

### EXAMPLE 3

### CELL VIABILITY ASSAYS

Whole cord blood cells or CD34⁺ cells obtained from Stem Cell Technologies (Vancouver, Canada) were aliquoted equally in Eppendorf tubes and treated *ex vivo* at a range of 16,16-dimethyl PGE₂ concentrations (10 µM to 100 µM) or DMSO control; temperatures (4°C, 22°C, or 37°C) for 60 or 120 minutes in LMD/5%HSA media. After treatment, an aliquot of the incubated cells were assayed using 7-Amino-Actinomycin D (7-AAD) staining as an indicator of cell death. One million whole cord blood were stained with 5 µL of 7AAD staining solution (BD Bioscience, San Jose, CA), or 200,000 CD34⁺ cord blood cells were stained with 1 µL 7-AAD solution. Cells were analyzed on a Guava EasyCyte 8HT System (Millipore) and with the FlowJo software package (Tree Star Inc., Ashland, OR). A separate aliquot of the same cells were also taken for assessment of proliferation potential using CFU-C assays (discussed below in Example 4).

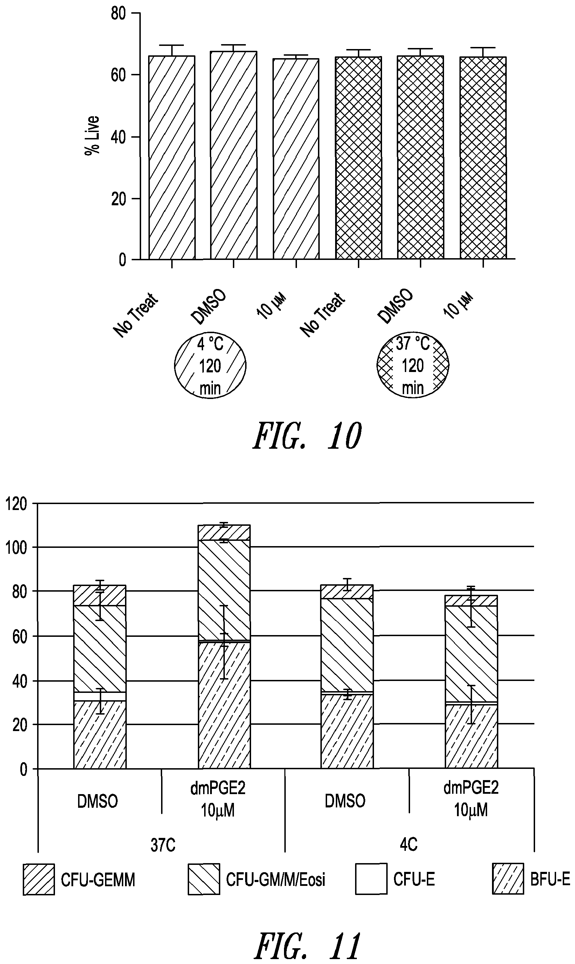

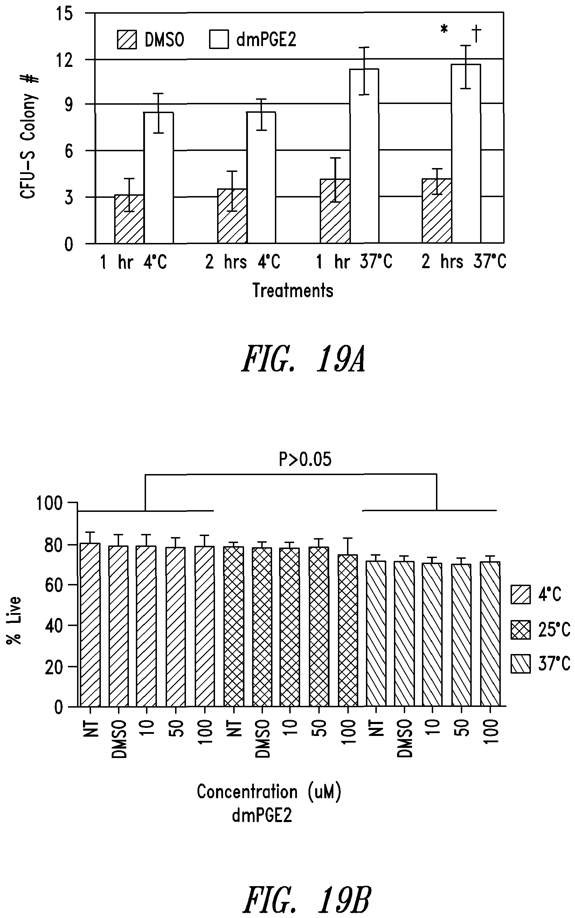

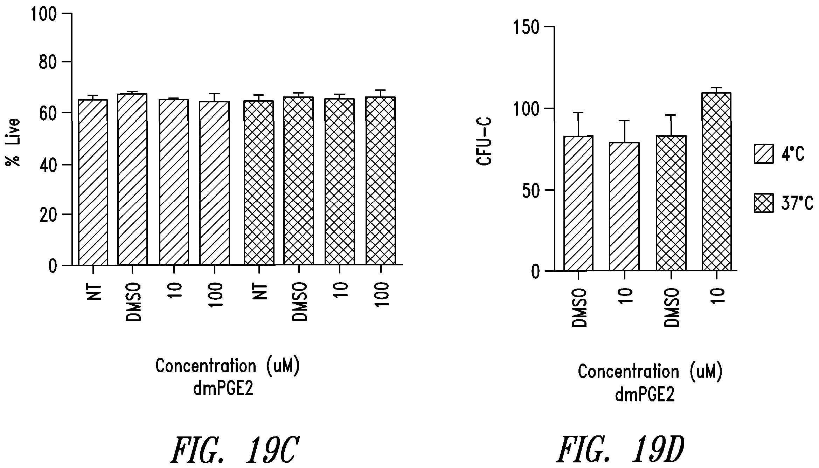

The results showed that whole cord blood cells or CD34⁺ cells incubated with 16,16-dimethyl PGE₂ at high temperatures for relatively long incubation periods did not decrease cell viability compared to cells incubated in other conditions, in contrast to what was expected from previous pre-clinical experiments. Thus, there was no statistically significant decrease in 7-AAD-assessed live cells from 4°C to 37°C (See Figures 10 and 19B-C).

### EXAMPLE 4

### CELL PROLIFERATION ASSAYS

Colony Forming Unit-Cell assays (CFU-C) were performed using a methyl cellulose assay kit, MethoCult® GF H4034 (Stem Cell Technologies, Vancouver, CA). Whole cord blood cells or CD34⁺ cells obtained from Stem Cell Technologies (Vancouver, Canada) were aliquoted equally in Eppendorf tubes and treated *ex vivo* at a range of 16,16-dimethyl PGE₂ concentrations (10 µM to 100 µM) or DMSO control; temperatures (4°C, 22°C, or 37°C) for 120 minutes in LMD/5%HSA media. After treatment, the cells were washed in LMD/5%HSA media and resuspended in Iscove's Media containing 2% Fetal Bovine Serum (FBS) (Stem Cell Technologies). Cells were then loaded into a MethoCult® assay tube, mixed, and plated in 35mm tissue culture plates according to the manufacturer's instructions. Unless otherwise mentioned, the equivalent of 10,000 WCB cells and 250 CD34⁺ cells were loaded into each plate.

The results showed that whole cord blood cells or CD34⁺ cells incubated with 16,16-dimethyl PGE₂ at high temperatures for relatively long incubation periods had a higher proliferative potential (*e.g*., capacity for self-renewal) than cells incubated in other conditions, in contrast to expectations based on previous pre-clinical experiments. Thus, there was a statistically significant increase in cellular proliferative potential from 4°C to 37°C (See Figure 11).

### EXAMPLE 5

### CXCR4 CELL SURFACE EXPRESSION IN 16,16-DIMETHYL PGE2 TREATED CELLS

### Flow Cytometry

CD34⁺ cord blood cells (Stem Cell Technologies or All Cells) were treated for 2 hours at 37°C in 10µM 16,16-dimethyl PGE₂ or DMSO as control in LMD/5%HSA. After treatment, cells were washed with LMD/5%HSA and centrifuged at 650 x g for 10 minutes. The cells were then sorted to isolate the long-term, short-term and multipotent progenitor cells according to a protocol published elsewhere (Park et al., 2008) The cells were then resuspended in staining media (mentioned above), and antibody stain was added. All antibodies were from BD Biosciences unless noted otherwise. Lineage depletion antibody panels included CD2, CD3, CD4, CD7, CD8, CD10, CD11b, CD14, CD19, CD20, CD56, CD235 and were all directly conjugated to FITC. Other antibodies used were CD34(8G12)-APC, CD38-PerCPCy5.5, CD45RA-V450, and CD90-PE. Cells were stained with the recommended amount of antibodies for 20 minutes on ice and then washed twice with staining media. The cells were then resuspended at 2 million cells/ml and sorted on a FACS Aria II using DiVa software (BD Biosciences). The cells were collected in StemSpan media and kept at 4°C until RNA extraction was performed.

### CXCR4 Surface Expression Analysis

CD34⁺ cord blood cells were treated with 10µM 16,16-dimethyl PGE₂ or DMSO in StemSpan media for 2 hours at 37°C or for 1 hour at 4°C. After treatment, the cells were washed in StemSpan media, centrifuged for 10 minutes at 300 x g and resuspended in StemSpan media containing cytokines (e.g., CC100) to promote CD34⁺ cell survival and incubated at 37°C for 1, 6 and 24 hours. After the 1, 6 or 24 hour incubation, cells were centrifuged and resuspended in staining media containing the Lineage cocktail, 1-FITC, CD34-APC, CXCR4(CD184)-PE, and incubated on ice for 15 minutes. Fresh staining media was then added to the cells, and the cells were centrifuged at 300g for 10 minutes, twice. The stained cells were acquired on a Guava EasyCyte 8HT and analysis was performed using FloJo Software Package (Treestar).

An increase in CXCR4 RNA expression was observed in whole cord blood (WCB) or CD34⁺ cells treated with 10µM 16,16-dimethyl PGE₂ for 2 hours at 37°C when compared to DMSO treated cells or cells treated with 10µM for 1 hour at 4°C. CXCR4 cell-surface expression is important for stem cell homing to the bone marrow hematopoietic niche. Figure 18 shows that CXCR4 protein surface expression increased in the presence of 16,16-dimethyl PGE₂ under certain conditions. Cells were treated for 2 hours at 37°C or for 1 hour at 4°C with either 10 µM 16,16-dimethyl PGE₂ or DMSO control in StemSpan media. CXCR4 cell-surface protein expression was assessed 1, 6 and 24 hours after the end of treatment by flow cytometry.

At 1 hour after treatment, CXCR4 expression was detectable in 48% of the CD34⁺ cord blood cells treated with 10µM 16,16-dimethyl PGE₂ compared to 3.5% of DMSO treated cells. At 6 hours post-treatment, 34.7% of CD34⁺ cord blood cells treated with 10µM 16,16-dimethyl PGE₂ expressed detectable levels of CXCR4 compared to 1.7% DMSO treated cells. At 24 hours post-treatment, the level of CXCR4 present on CD34⁺ cord blood cells treated with 10µM 16,16-dimethyl PGE₂ was substantially the same compared to DMSO treated cells.

In contrast, CD34⁺ cord blood cells treated with 10µM 16,16-dimethyl PGE₂ at 4°C, did not result in an increase in CXCR4 expression. Thus, CXCR4 mRNA expression in cells treated with 10µM 16,16-dimethyl PGE₂ faithfully represents the CXCR4 cell-surface protein expression in the cells. Accordingly, in particular clinical embodiments, to obtain the maximal activation of HSC to effect HSC homing and function, treating cells with 16,16-dimethyl PGE₂ at 37°C is preferred compared to treatment at 4°C.

### EXAMPLE 6

### COLONY FORMING UNIT SPLEEN ASSAYS

Colony forming Unit Spleen at day 12 (CFU-S12) assays were performed as described in North et al., Nature. 2007 Jun 21;447(7147):1007-11. Whole bone marrow from 8-week old C57BI/6 donor mice was isolated and treated with 10µM 16,16-dimethyl PGE₂ or DMSO at 4°C for 1 hour or at 37°C for 2 hours or in PBS. After treatment, the cells were washed by centrifugation and resuspended in PBS for tail-vein injection into C57BI/6 recipients (2 x 5/treatment) that had been previously lethally irradiated at 10.5Gy. 50,000 cells were injected per recipient.

The increase in the 16,16-dimethyl PGE₂ gene expression signature observed after treatment with 16,16-dimethyl PGE₂ at 37°C for 2 hours compared to treatment with 16,16-dimethyl PGE₂ at 4°C for 1 hour was further corroborated in a CFU-S assay.

Murine bone marrow was exposed to 10µM treatment with 16,16-dimethyl PGE₂ or DMSO for either 1 hour at 4°C or 2 hours at 37°C and administered to irradiated mice. Fourteen days later, spleens were excised and colonies counted. The results showed that the 4°C treatment did not elicit a cAMP response or an increase in gene expression signal, but resulted in a small increase in CFU-S number compared to DMSO treated cells. However, this increase is statistically significantly lower than when the cells were incubated with 10µM 16,16-dimethyl PGE₂ at 37°C.

Murine whole bone marrow (WBM) cells were exposed to 10µM 16,16-dimethyl PGE₂ or DMSO for 1 or 2 hours at 4°C or 37°C. A statistically significant increase in CFU-S12 number was observed when cells were treated with 10µM 16,16-dimethyl PGE₂ regardless of temperature when compared to DMSO treated cells (Figure 19A). Exposure to 10µM 16,16-dimethyl PGE₂ at 37°C for 2 hours resulted in the formation of 11.5±1.4 colonies which was significantly higher than WBM exposed to 10µM treatment with 16,16-dimethyl PGE₂ at 4°C for 1 hour, 8.5±1.3 colonies (p<0.005) or to DMSO at 37°C for 2 hours, 4.0±0.8 colonies (p<0.001). Further, WBM treated with 10µM 16,16-dimethyl PGE₂ at 37°C for 1 hour (11.2±1.5) or 2 hours (11.5±1.4) gave similar results. The same was observed with 16,16-dimethyl PGE₂ treatment of WBM at 4°C, where 1 or 2 hours exposure gave similar results, 8.5±1.3 and 8.4±1.0 colonies, respectively.

### EXAMPLE 7

### ENHANCED CHEMOTAXIS OF 16,16-DIMETHYL PGE2 TREATED CELLS

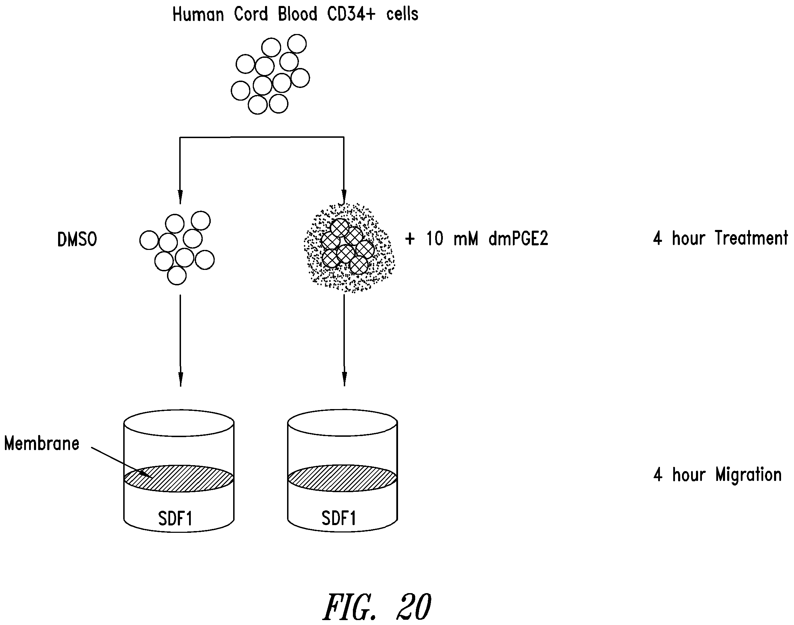

Chemotaxis assays were performed using 96-well chemotaxis chambers, 5µM pore size polycarbonate membrane (Corning Inc., Corning, NY) in accordance with manufacturer's instructions. Briefly, human CD34⁺ cord blood (hCD34⁺ CB) cells were obtained from All Cells and were thawed according to manufacturer's instruction. The cells were then treated for 4 hours at 37°C with 16,16-dimethyl PGE₂ or DMSO control at a concentration of 10µM in StemSpan media (Stem Cell Technology, Vancouver, Canada). The cells were then washed by centrifugation (300g for 10 minutes) and resuspended in transwell assay buffer (Phenol Red Free RPMI media (Mediatech), 0.5% lipid free BSA (Sigma-Aldrich) at a concentration of 40,000-60,000 cells/75ul. Seventy-five µl of cell suspension was added to the upper chamber of the plate, while 235µl of transwell assay media containing 0 or 50ng/ml SDF1α (R&D system, Minneapolis, MN) was added to the bottom well. Total cell number in the lower well was obtained by flow cytometry, using 7AAD (BD Biosciences) to exclude dead cells, after 4 hours of incubation at 37°C, 5% CO₂. Figure 20 shows a flowchart of the chemotaxis *in vitro* functional assay used in these experiments. Percent migration was calculated by dividing the number of the cells in the lower well by the total cell input multiplied by 100. Samples were analyzed in triplicate, the data was then averaged for statistical analysis.

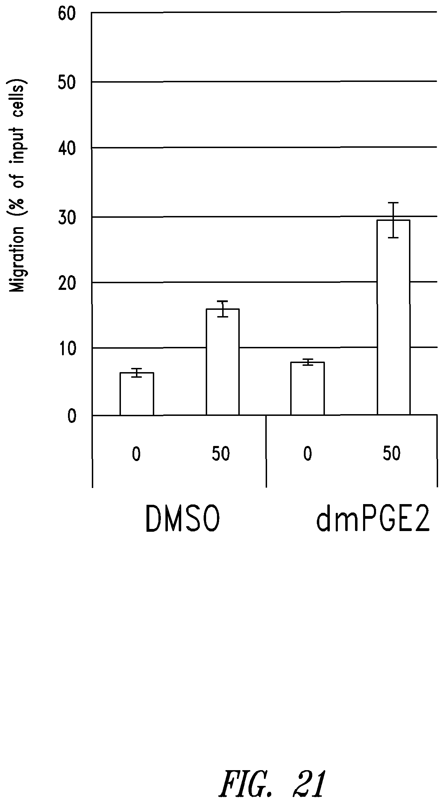

Figure 21 shows that the number of migrating CD34⁺ cells incubated with 16,16-dimethyl PGE is significantly increased when exposed to 50ng/ml SDF1α compared to the number of migrating CD34⁺ cells incubated with DMSO or negative controls (0ng/ml SDF1α). Thus, CD34⁺ cells treated with 16,16-dimethyl PGE have increased stem cell homing properties when compared to DMSO or non-treated control cells.

### EXAMPLE 8

### PHASE 1B CLINICAL STUDY

### Summary

Preclinical data generated by the present inventors supported the use of 16,16-dimethyl PGE₂ as a promoter of HSC homing, proliferation, survival, and differentiation. Based on the preclinical data, a Phase Ib clinical trial was initiated in adults with hematologic malignancies undergoing double (cord blood) CB transplantation after a reduced-intensity conditioning regimen. One primary objective of the study was to determine the safety of 16,16-dimethyl PGE₂ treated-UCB based upon engraftment by Day 42 with > 5% chimerism of the 16,16-dimethyl PGE₂ treated-UCB unit. Secondary objectives included time to engraftment, the rates of non-hematologic toxicity, graft failure, acute and chronic GVHD, relapse, treatment related mortality (TRM), fractional chimerism, and relapse-free and overall survival. The competitive engraftment dynamic of double UCB transplantation permits determination whether dmPGE₂-modified HSCs are able to out-compete unmodulated HSCs.

### Methods

The criteria for cord blood selection consisted of a minimum 4/6 HLA match of each cord blood unit to the subject as well as to the other cord blood unit. Patients without a sibling or matched-unrelated donor were conditioned with fludarabine (30 mg/m2/day IV Day -8 to -3), melphalan (100 mg/m2/day IV Day -2), and rabbit ATG (1 mg/kg/day Days -7, -5, -3 and -1). The immunosuppression regimen included sirolimus (target 3-12 ng/mL) and tacrolimus (target 5-10 ng/mL). On day 0, patients received two umbilical cord blood (UCB) units: the first UCB unit (16,16-dimethyl PGE₂-treated UCB), was thawed in a warm water bath and washed using a solution of 5% human serum albumin (HSA) and low molecular weight (LMW) dextran. The cells were incubated with 16,16-dimethyl PGE₂ for 2 hours at 37°C. After a final wash to remove residual 16,16-dimethyl PGE₂, the 16,16-dimethyl PGE₂-treated UCB was administered to the patient by infusion without further manipulation of the cells. The second untreated UCB unit was thawed, washed and infused 2-6 hours later without modulation or manipulation of the cells.

### Results

A total of 12 subjects were enrolled and received 16,16-dimethyl PGE₂-treated-UCB units, of which 11 subjects were evaluable. The median age was 57.5 years (range 19-66) and 67% were male. Diagnoses included: AML(5), MDS(4) and NHL/CLL(3). All 16,16-dimethyl PGE₂-treated-UCBs were treated and infused on Day 0. The median precryopreservation UCB sizes were 16,16-dimethyl PGE₂-treated-UCB: 2.7x10⁷ TNC/kg (range 2.0-5.1) and 1.3x10⁵ CD34/kg (range 0.3-6.3); untreated UCB: 2.0x10⁷ TNC/kg (range 1.8-5) and 1.1 x 10⁵ CD34/kg (range 0.5-3.4) with a median combined cell dose of 4.7x10⁷ TNC/kg (range 3.9-10.1) and 2.1x10⁵ CD34/kg range (1.4-9.7).

Treatment of UCB with 16,16-dimethyl PGE₂ did not result in significant cell loss, with a mean viable CD34⁺ cell recovery of 90%. The adverse events attributed to 16,16-dimethyl PGE₂ treated-UCB included five Grade 1 infusion-related events in four subjects, consisting of chills, flushing, abdominal pain, or cough. One additional subject with known coronary artery disease experienced transient Grade 4 ST-elevation with infusion and evidence of myocardial ischemia by cardiac troponin assay.

The median time to neutrophil recovery (> 500 cells/µL) was 17 days (range 15-27 days), which compares favorably to a median of 21 days for a historic control group of similarly treated patients at the same institution (n=53; p= 0.025). These data also compare favorably with a previous cohort of 9 patients, who also received an untreated UCB unit in combination with a 16,16-dimethyl PGE₂ treated-UCB that was prepared using an alternate incubation regimen: treatment with 16,16-dimethyl PGE₂ for 60 minutes on ice (a temperature of 4°C). These patients had a median time to neutrophil recovery of 22 days.

The median time to an unsupported platelet count of 20,000/µL was 42 days (n=9 evaluable). There were no instances of primary or secondary graft failure. The 16,16-dimethyl PGE₂ treated-UCB was the dominant source of hematopoiesis in 9 of the 11 evaluable subjects, and the median total chimerism of 16,16-dimethyl PGE₂ treated-UCB at day 14 was 90%, with long-term dominance again by the 16,16-dimethyl PGE₂ treated-UCB unit. In comparison, in the previous experience of 9 patients who received an untreated UCB and a 16,16-dimethyl PGE₂ treated-UCB prepared using the 4°C/60 minutes incubation regimen, the 16,16-dimethyl PGE₂ treated-UCB was the dominant source of hematopoiesis in only 2 of 9 cases.

To date, only two cases of Grade 2 acute GvHD have been observed and there have been no observed cases of chronic GvHD. In addition, no cases of EBV- lymphoproliferative disease were noted. TRM was 9% (1 subject), and one patient has relapsed; 9 subjects remain alive without relapse with a median follow-up of 5.0 months (range 1.6-9.4).

### Conclusions

These data support the benefit of a novel *ex vivo* modulation approach to improving engraftment in patients undergoing UCB transplantation. Further, results from these experiments clearly demonstrated that increasing the incubation temperature from 4°C to 37°C and increasing the incubation time from 60 minutes to 120 minutes resulted in a profound increase in the biological activity of 16,16-dimethyl PGE₂ treated cells. This body of work provides an important example of how molecular profiling can have a direct impact on clinical medicine and demonstrates that a short *ex vivo* treatments with small molecules can enhance cell therapy.

### EXAMPLE 9

### HEAD TO HEAD ANALYSIS OF REPOPULATING ACTIVITY OF MOUSE BONE MARROW CELLS TREATED EX VIVO WITH DMPGE2 AT 4°C VERSUS 37°C

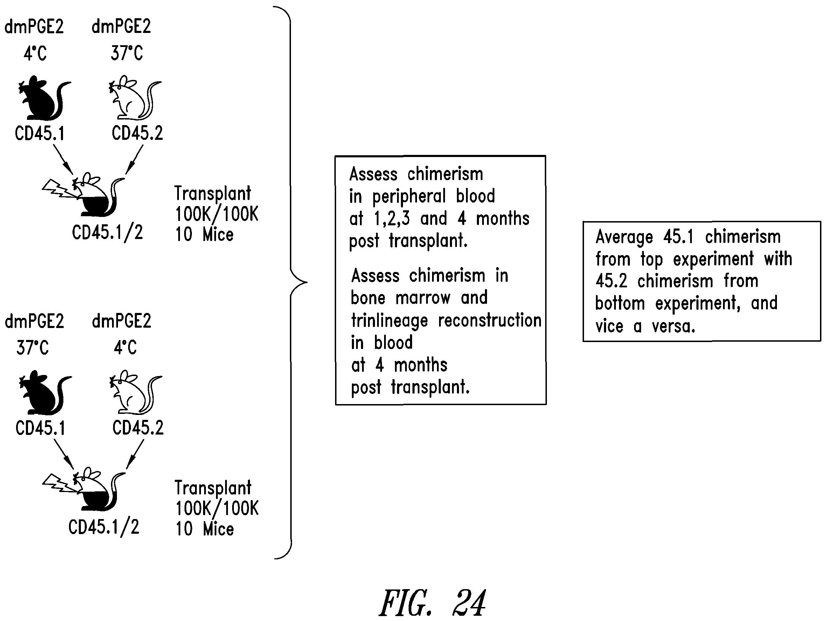

The invention demonstrates the effects of dmPGE₂ on WBC recovery, including enhanced recoveries of erythroid, platelet and neutrophil counts compared to controls when cells are pulsed at 37°C. The present study is a head to head comparison of the engraftment of cells treated *ex vivo* with dmPGE₂ at 4°C versus 37°C. Bone marrow cells from congenic CD45.1 and CD45.2 mice are treated *ex vivo* for 2 hours with 10 uM 16,16-dmPGE₂ and co-transplanted into lethally irradiated CD45.1/CD45.2 hybrid recipient mice. Groups of 10 hybrid mice receive 100,000 CD45.1 marrow cells treated with 16,16-dmPGE₂ at 4°C and 100,000 CD45.2 marrow cells treated with 16,16-dmPGE₂ at 37°C. A second cohort of 10 mice receive 100,000 CD45.2 marrow cells treated with 16,16-dmPGE₂ at 4°C and 100,000 CD45.1 marrow cells treated with 16,16-dmPGE₂ at 37°C to compensate for strain bias. Mice are bled at 1, 2, 3 and 4 months post transplant and CD45.1 and CD45.2 positive peripheral blood cells determined. At 4 months, tri-lineage reconstitution is evaluated to determine any lineage reconstitution bias or difference. Mice are euthanized and marrow chimerism performed at 4 months post transplant. Deviation from 50%/50% chimerism reflects alteration in engraftment capacity resulting from the treatment protocols. A graphic outline of the study is shown in Figure 24.

### EXAMPLE 10

### METHODS

### Isolation of Lin(-)CD34⁺ cells from treated whole cord blood

Human whole cord blood mononuclear cells were obtained from Stem Cell Technologies (Vancouver, Canada). Upon thawing, the cells were treated with 16,16-dimethyl PGE₂ or appropriate controls, e.g., DMSO, in LMD/5%HSA medium.

After treatment, the cells were washed with LMD/5%HSA medium, centrifuged for 10 minutes at 650 x g at room temperature and resuspended in a cold selection buffer (phosphate buffered saline (PBS) with no Ca⁺ or Mg⁺; 2mM EDTA; and 0.5% HSA). Magnetic selection was performed using the Lineage (Lin) Depletion Kit (Miltenyi Biotec, abrun, CA) followed by a CD34⁺ enrichment kit (Miltenyi Biotec). Lineage depletion and CD34⁺ cell enrichment were performed according to manufacturer's instructions using a QuadroMACS™ separator. During this process, the cells were kept at 4°C. Once the Lin-CD34⁺ cells were isolated from the treated whole cord blood, an aliquot was analyzed by flow cytometry to assess purity. Purity of the cells was greater than 90%. The majority of the cells were used for RNA extraction using the Pico Pure RNA Isolation Kit (Molecular Devices, Sunnyvale, CA) for Affymetrix analysis.

The various embodiments described above can be combined to provide further embodiments. All of the U.S. patents, U.S. patent application publications, U.S. patent applications, foreign patents, foreign patent applications and non-patent publications referred to in this specification and/or listed in the Application Data Sheet are incorporated herein by reference, in their entirety. Aspects of the embodiments can be modified, if necessary to employ concepts of the various patents, applications and publications to provide yet further embodiments.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

### ITEMS

1. A therapeutic composition comprising a population of cells comprising at least about one million human hematopoietic stem or progenitor cells wherein:
   a) the hematopoietic stem or progenitor cells have been contacted ex *vivo* at a temperature of about 37°C with an agent that increases CXCR4 gene expression in the cells;
   b) gene expression of CXCR4 is increased in the hematopoietic stem or progenitor cells by at least about 2 fold compared to the expression of CXCR4 in a non-contacted hematopoietic stem or progenitor cell; and
   c) wherein the therapeutic composition comprises a sterile, therapeutically acceptable suspension of hematopoietic stem or progenitor cells ready for administration to a patient.
2. The therapeutic composition of item 1 wherein gene expression of CXCR4 in the hematopoietic stem or progenitor cells is increased by at least about 3 fold compared to the expression of CXCR4 in a non-contacted hematopoietic stem or progenitor cell.
3. The therapeutic composition of item 1, wherein the agent that increases CXCR4 gene expression in the hematopoietic stem or progenitor cells is selected from the group consisting of a cAMP analogue or enhancer, a Gα-s activator, and a compound that selectively binds the PGE₂ EP₄ receptor.
4. The therapeutic composition of item 1, wherein the agent that increases CXCR4 gene expression in the hematopoietic stem or progenitor cells is PGE₂, or a PGE₂ analogue or derivative.
5. The therapeutic composition of item 1, wherein the agent that increases CXCR4 gene expression in the hematopoietic stem or progenitor cells is 16,16-dimethyl PGE₂.
6. The therapeutic composition of item 1, wherein the hematopoietic stem or progenitor cells have been contacted with the agent for a time of at least about one hour.
7. The therapeutic composition of item 1, wherein the hematopoietic stem or progenitor cells have been contacted with the agent for a time of about one hour to about six hours.
8. The therapeutic composition of item 1, wherein the hematopoietic stem or progenitor cells have been contacted with the agent for a time of about two hours to about six hours.
9. The therapeutic composition of item 1, wherein the hematopoietic stem or progenitor cells have been contacted with the agent for a time of about two hours.
10. The therapeutic composition of item 1, wherein the hematopoietic stem or progenitor cells comprise a gene expression signature wherein expression of one or more signature genes is increased by at least about 2 fold compared to the expression of the one or more signature genes in a noncontacted hematopoietic stem or progenitor cell, wherein the signature gene is selected from the group consisting of: hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), and Fos-related antigen 2 (FOSL2).
11. The therapeutic composition of item 10, wherein expression of at least two of the signature genes is increased by at least 5, 10, 15, or 20 fold compared to the expression of the two signature genes in a non-contacted hematopoietic stem or progenitor cell.
12. The therapeutic composition of item 10, wherein expression of each of the signature genes is increased by at least about 2 fold compared to the expression of the signature genes in a non-contacted hematopoietic stem or progenitor cell.
13. The therapeutic composition of item 1, wherein the population of cells comprises less than about .10, .50, 1.0, 3, 5, 10, 15, 20, or 30% CD34⁺ cells.
14. The therapeutic composition of item 1, wherein the population of cells comprises at least about .01% and no more than about 50% of CD34⁺ cells.
15. The therapeutic composition of item 1, wherein the population of cells is not expanded *ex vivo.*
16. The therapeutic composition of item 1, wherein the therapeutic composition is generated at a point-of-care and is administered into a patient without culturing the population of cells.
17. The therapeutic composition of item 1, wherein the therapeutic composition is substantially free of the agent.
18. The therapeutic composition of item 1, wherein the population of cells comprises less than about 30%, 25%, 20%, 15%, 10% or 5% mesenchymal stem cells.
19. The therapeutic composition of item 1, wherein the population of cells comprises less than about 30%, 25%, 20%, 15%, 10% or 5% endothelial progenitor cells.
20. The therapeutic composition of item 1, wherein at least about 15% of cells within the population of cells express CXCR4 protein.
21. The therapeutic composition of item 1, wherein the population of cells is obtained from bone marrow, umbilical cord blood, or mobilized peripheral blood.
22. The therapeutic composition of item 1, wherein the population of cells is HLA haplotyped.
23. The therapeutic composition of item 1, wherein the population of cells is HLA haplotyped based on the group consisting of HLA-A, HLA-B, HLA-C, and HLA-DRB1.
24. The therapeutic composition of item 1, wherein the population of HLA haplotyped cells is matched with a specific human subject.
25. The therapeutic composition of item 1, wherein the population of HLA haplotyped cells has 4 out of 6 HLA matches with a specific human subject.
26. A therapeutic composition comprising a population of cells comprising at least about one million human hematopoietic stem or progenitor cells wherein:
   a) the hematopoietic stem or progenitor cells have been contacted ex *vivo* at a temperature of about 37°C with 16,16-dmPGE₂ for a time of about two hours;
   b) the hematopoietic stem or progenitor cells comprise a collection of CD34⁺ cells wherein gene expression of CXCR4 is increased in the collection of CD34⁺ cells by at least about 3 fold compared to the expression of CXCR4 in non-contacted CD34⁺ cells; and
   c) wherein the therapeutic composition comprises a sterile, therapeutically acceptable suspension of hematopoietic stem or progenitor cells ready for administration to a patient.
27. The therapeutic composition of item 26 wherein the hematopoietic stem or progenitor cells comprise a gene expression signature wherein expression of one or more signature genes is increased by at least about 2 fold compared to the expression of the one or more signature genes in a noncontacted hematopoietic stem or progenitor cell, wherein the signature gene is selected from the group consisting of: hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), and Fos-related antigen 2 (FOSL2).
28. The therapeutic composition of item 26, wherein expression of at least two of the signature genes is increased by at least 5, 10, 15, or 20 fold compared to the expression of the two signature genes in a non-contacted hematopoietic stem or progenitor cell.
29. The therapeutic composition of item 26, wherein expression of each of the signature genes is increased by at least about 2 fold compared to the expression of the signature genes in a non-contacted hematopoietic stem or progenitor cell.
30. The therapeutic composition of item 26, wherein the population of cells comprises less than about .10, .50, 1.0, 3, 5, 10, 15, 20, or 30% CD34⁺ cells.
31. The therapeutic composition of item 26, wherein the population of cells comprises at least about .01 % and no more than about 50% of CD34⁺ cells.
32. The therapeutic composition of item 26, wherein the population of cells is not expanded *ex vivo.*
33. The therapeutic composition of item 26, wherein the population of cells is obtained from bone marrow, umbilical cord blood, or mobilized peripheral blood.
34. The therapeutic composition of item 26, wherein the population of cells is HLA haplotyped.
35. A therapeutic composition comprising a population of haplotyped cells comprising at least about one million human hematopoietic stem or progenitor cells wherein:
   a) the hematopoietic stem or progenitor cells have been contacted ex *vivo* at a temperature of about 37°C with an agent that increases CXCR4 gene expression in the cells;
   b) gene expression of CXCR4 is increased in the hematopoietic stem or progenitor cells by at least about 2 fold compared to the expression of CXCR4 in a non-contacted hematopoietic stem or progenitor cell; and
   c) wherein the therapeutic composition comprises a sterile, therapeutically acceptable suspension of hematopoietic stem or progenitor cells ready for administration to a patient.
36. The therapeutic composition of item 35, wherein the population of halpotyped cells is haplotyped based on the group consisting of HLA-A, HLA-B, HLA-C, and HLA-DRB1.
37. The therapeutic composition of item 35, wherein the population of halpotyped cells is haplotyped based on the group consisting of HLA-DRB3/4/5, HLA-DQB1, and DPB1.
38. The therapeutic composition of item 35, wherein the population of haplotyped cells is matched with a specific human subject.
39. The therapeutic composition of item 37, wherein the population of HLA haplotyped cells has 4 out of 6 HLA matches with a specific human subject.
40. The therapeutic composition of item 35, wherein gene expression of CXCR4 in the hematopoietic stem or progenitor cells is increased by at least about 3 fold compared to the expression of CXCR4 in a non-contacted hematopoietic stem or progenitor cell.
41. The therapeutic composition of item 35 wherein the agent that increases CXCR4 gene expression in the hematopoietic stem or progenitor cells is selected from the group consisting of a cAMP analogue or enhancer, a Gα-s activator, and a compound that selectively binds the PGE₂ EP₄ receptor.
42. The therapeutic composition of item 35, wherein the agent that increases CXCR4 gene expression in the hematopoietic stem or progenitor cells is 16,16-dimethyl PGE₂.
43. The therapeutic composition of item 35 wherein the hematopoietic stem or progenitor cells have been contacted with the agent for a time of about one hour to about six hours.
44. The therapeutic composition of item 35, wherein the hematopoietic stem or progenitor cells comprise a gene expression signature wherein expression of one or more signature genes is increased by at least about 2 fold compared to the expression of the one or more signature genes in a noncontacted hematopoietic stem or progenitor cell, wherein the signature gene is selected from the group consisting of: hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), and Fos-related antigen 2 (FOSL2).
45. The therapeutic composition of item 35, wherein expression of at least two of the signature genes is increased by at least 5, 10, 15, or 20 fold compared to the expression of the two signature genes in a non-contacted hematopoietic stem or progenitor cell.
46. The therapeutic composition of item 35, wherein expression of each of the signature genes is increased by at least about 2 fold compared to the expression of the signature genes in a non-contacted hematopoietic stem or progenitor cell.
47. The therapeutic composition of item 35, wherein the population of cells comprises less than about .10, .50, 1.0, 3, 5, 10, 15, 20, or 30% CD34⁺ cells.
48. The therapeutic composition of item 35, wherein the population of cells comprises at least about .01 % and no more than about 50% of CD34⁺ cells.
49. The therapeutic composition of item 35, wherein the population of cells is not expanded *ex vivo.*
50. The therapeutic composition of item 35, wherein the therapeutic composition is generated at a point-of-care and is administered into a patient without culturing the population of cells.
51. The therapeutic composition of item 35, wherein the population of cells is obtained from bone marrow, umbilical cord blood, or mobilized peripheral blood.
52. A method of preparing a therapeutic composition comprising:
   contacting a population of cells comprising hematopoietic stem or progenitor cells ex *vivo* with one or more agents at a temperature of about 37°C under conditions sufficient to modify the gene expression of the hematopoietic stem or progenitor cells to result in hematopoietic stem or progenitor cells comprising a gene expression signature comprising increased expression, as compared to non-contacted hematopoietic stem or progenitor cells, of one or more of the following genes: hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), Fos-related antigen 2 (FOSL2), or CXC chemokine receptor 4 (CXCR4).
53. The method of item 52, wherein the population of cells is obtained from bone marrow, umbilical cord blood, or mobilized peripheral blood.
54. The method of item 52, wherein at least one of the one or more agents increases PGE₂R₂ or PGE₂R₄ signaling in the hematopoietic stem or progenitor cells.
55. The method of item 52, wherein at least one of the one or more agents is selected from the group consisting of a cAMP enhancer, a Gα-s activator, and an agent that selectively binds the PGE₂EP₄ receptor.
56. The method of item 52, wherein the one or more agents comprises PGE₂ or an analogue thereof.
57. The method of item 56, wherein the PGE₂ analogue comprises 16,16-dimethyl PGE₂.
58. The method of item 52, wherein the conditions sufficient to modify the gene expression of the hematopoietic stem or progenitor cells comprise contacting the hematopoietic stem and progenitor cells with the one or more agents for a time of about 1 hour to about 6 hours, wherein at least one of the agents comprises an agent that increases PGE₂R₂ or PGE₂R₄ signaling in the hematopoietic stem or progenitor cells.
59. The method of item 58, wherein the hematopoietic stem and progenitor cells are contacted at a temperature of about 37°C for a time of about 2 hours with a concentration of about 10 µM of the agent that increases PGE₂R₂ or PGE₂R₄ signaling in the hematopoietic stem or progenitor cells.
60. The method of item 52, wherein the therapeutic composition is prepared in an endotoxin-free vessel comprising: a temperature indicating device comprising at least one temperature indicator that produces a signal that indicates the temperature of the vessel and an elapsed time indicating device that comprises at least one elapsed time indicator; and wherein the vessel is suitable for cell storage, treatment of cells, washing of cells, and cell infusion.
61. The method of item 52, wherein the therapeutic composition is administered to a subject in need of cell therapy.
62. The method of item 61, wherein the subject has acute myelogenous leukemia (AML), acute lymphoblastic leukemia (ALL), chronic myelogenous leukemia (CML), chronic lymphocytic leukemia (CLL), juvenile myelomonocytic leukemia, Hodgkin's lymphoma, non-Hodgkin's lymphoma, multiple myeloma, severe aplastic anemia, Fanconi's anemia, paroxysmal nocturnal hemoglobinuria (PNH), pure red cell aplasia, amegakaryocytosis/congenital thrombocytopenia, severe combined immunodeficiency syndrome (SCID), Wiskott-Aldrich syndrome, beta-thalassemia major, sickle cell disease, Hurler's syndrome, adrenoleukodystrophy, metachromatic leukodystrophy, myelodysplasia, refractory anemia, chronic myelomonocytic leukemia, agnogenic myeloid metaplasia, familial erythrophagocytic lymphohistiocytosis, solid tumors, chronic granulomatous disease, mucopolysaccharidoses, or Diamond Blackfan.
63. The method of item 61, wherein the subject has breast cancer, ovarian cancer, brain cancer, prostate cancer, lung cancer, colon cancer, skin cancer, liver cancer, pancreatic cancer, or sarcoma.
64. The method of item 61, wherein the subject has received bone marrow ablative or non-myeolablative chemotherapy or radiation therapy.
65. The method of item 61, wherein the subject is a bone marrow donor.
66. A method of increasing hematopoietic stem and progenitor cell engraftment in a subject comprising:
   (a) contacting a population of cells that comprises hematopoietic stem and progenitor cells *ex vivo,* at a temperature of about 37°C, with one or more agents selected from the group consisting of: a prostaglandin E₂ (PGE₂) and an agent having dmPGE₂ activity;
   (b) washing the population of cells to substantially remove the agent; and
   (c) administering the population of cells to a subject;
   wherein the population of cells is contacted with the agent under conditions sufficient to increase the engraftment of the contacted hematopoietic stem and progenitor cells in the subject compared to non-contacted hematopoietic stem and progenitor cells.
67. The method of item 66, wherein the population of cells is obtained from bone marrow, umbilical cord blood, or mobilized peripheral blood.
68. The method of item 66, wherein the agent is PGE₂ or an analogue thereof.
69. The method of item 68, wherein the PGE₂ analogue is 16,16-dimethyl PGE₂.
70. The method of item 66, wherein the conditions sufficient to increase engraftment comprise contacting the hematopoietic stem and progenitor cells:
   (a) at a concentration of about 10 µM or more 16,16-dimethyl PGE₂; and
   (b) for a time of about 1 hour to about 6 hours.
71. The method of item 66, wherein the hematopoietic stem and progenitor cells are contacted with a concentration of about 10 µM 16,16-dimethyl PGE₂; and for a time of about 2 hours.
72. The method of item 66, wherein the contacted hematopoietic stem and progenitor cells comprise an increase in gene expression of one or more of hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), Fos-related antigen 2 (FOSL2), or CXC chemokine receptor 4 (CXCR4), compared to non-contacted hematopoietic stem and progenitor cells.
73. The method of item 52 or 66, wherein the population of cells is contacted with the one or more agent in an endotoxin-free vessel comprising: a temperature indicating device comprising at least one temperature indicator that produces a signal that indicates the temperature of the vessel and an elapsed time indicating device that comprises at least one elapsed time indicator; and wherein the vessel is suitable for cell storage, treatment of cells, washing of cells, and cell infusion.
74. The method of item 66, wherein the population of cells comprises one or more cord blood units.
75. The method of item 74, wherein the subject is administered one or more cord blood units.
76. The method of item 74, wherein the subject is administered one cord blood unit or a partial cord blood unit.
77. The method of item 66, wherein the subject has acute myelogenous leukemia (AML), acute lymphoblastic leukemia (ALL), chronic myelogenous leukemia (CML), chronic lymphocytic leukemia (CLL), juvenile myelomonocytic leukemia, Hodgkin's lymphoma, non-Hodgkin's lymphoma, multiple myeloma, severe aplastic anemia, Fanconi's anemia, paroxysmal nocturnal hemoglobinuria (PNH), pure red cell aplasia, amegakaryocytosis/congenital thrombocytopenia, severe combined immunodeficiency syndrome (SCID), Wiskott-Aldrich syndrome, beta-thalassemia major, sickle cell disease, Hurler's syndrome, adrenoleukodystrophy, metachromatic leukodystrophy, myelodysplasia, refractory anemia, chronic myelomonocytic leukemia, agnogenic myeloid metaplasia, familial erythrophagocytic lymphohistiocytosis, or solid tumors.
78. The method of item 66, wherein the subject has breast cancer, ovarian cancer, brain cancer, prostate cancer, lung cancer, colon cancer, skin cancer, liver cancer, pancreatic cancer, or sarcoma.
79. The method of item 66, wherein the subject has received bone marrow ablative or non-myeolablative chemotherapy or radiation therapy..
80. The method of item 66, wherein the subject is a bone marrow donor.
81. The method of item 66, wherein the population of cells is autogeneic to the subject.
82. The method of item 81, wherein the population of cells is mobilized from the peripheral blood or bone marrow of the subject.
83. The method of item 66, wherein the population of cells is allogeneic to the subject.
84. A method of treating a subject in need of hematopoietic system reconstitution comprising:
   (a) selecting a subject in need of hematopoietic system reconstitution;
   (b) contacting a population of cells that comprises hematopoietic stem and progenitor cells *ex vivo,* at a temperature of about 37°C, with one or more agents selected from the group consisting of: a prostaglandin E₂ (PGE₂) and an agent having dmPGE₂ activity;
   (c) washing the population of cells to substantially remove the agent; and
   (d) administering the population of cells to the subject;
   wherein the population of cells is contacted with the agent under conditions sufficient to increase the engraftment of the contacted hematopoietic stem and progenitor cells in the subject compared to non-contacted hematopoietic stem and progenitor cells thereby treating the subject in need of hematopoietic system reconstitution.
85. The method of item 84, wherein the population of cells is obtained from bone marrow, umbilical cord blood, or mobilized peripheral blood.
86. The method of item 84, wherein the agent is PGE₂ or an analogue thereof.
87. The method of item 84, wherein the PGE₂ analogue is 16,16-dimethyl PGE₂.
88. The method of item 84, wherein the conditions sufficient to increase the engraftment comprise contacting the hematopoietic stem and progenitor cells:
   (a) at a concentration of about 10 µM or more 16,16-dimethyl PGE₂; and
   (b) for a time of about 1 hour to about 6 hours.
89. The method of item 88, wherein the hematopoietic stem and progenitor cells are contacted with a concentration of about 10 µM 16,16-dimethyl PGE₂; and for a time of about 2 hours.
90. The method of item 84, wherein the contacted hematopoietic stem and progenitor cells comprise an increase in gene expression of one or more of hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), Fos-related antigen 2 (FOSL2), or CXC chemokine receptor 4 (CXCR4), compared to non-contacted hematopoietic stem and progenitor cells, an increase in capacity for self-renewal compared to non-contacted hematopoietic stem and progenitor cells, and no substantial decrease in cell viability compared to non-contacted hematopoietic stem and progenitor cells.
91. The method of item 84, wherein the population of cells is prepared in an endotoxin-free vessel comprising: a temperature indicating device comprising at least one temperature indicator that produces a signal that indicates the temperature of the vessel and an elapsed time indicating device that comprises at least one elapsed time indicator; and wherein the vessel is suitable for cell storage, treatment of cells, washing of cells, and cell infusion.
92. The method of item 84, wherein the population of cells comprises one or more cord blood units.
93. The method of item 92, wherein the subject is administered a partial cord blood unit or one or more cord blood units.
94. The method of item 84, wherein the subject has acute myelogenous leukemia (AML), acute lymphoblastic leukemia (ALL), chronic myelogenous leukemia (CML), chronic lymphocytic leukemia (CLL), juvenile myelomonocytic leukemia, Hodgkin's lymphoma, non-Hodgkin's lymphoma, multiple myeloma, severe aplastic anemia, Fanconi's anemia, paroxysmal nocturnal hemoglobinuria (PNH), pure red cell aplasia, amegakaryocytosis/congenital thrombocytopenia, severe combined immunodeficiency syndrome (SCID), Wiskott-Aldrich syndrome, beta-thalassemia major, sickle cell disease, Hurler's syndrome, adrenoleukodystrophy, metachromatic leukodystrophy, myelodysplasia, refractory anemia, chronic myelomonocytic leukemia, agnogenic myeloid metaplasia, familial erythrophagocytic lymphohistiocytosis, or solid tumors.
95. The method of item 84, wherein the subject has breast cancer, ovarian cancer, brain cancer, prostate cancer, lung cancer, colon cancer, skin cancer, liver cancer, pancreatic cancer, or sarcoma.
96. The method of item 84, wherein the subject has received bone marrow ablative or non-myeolablative chemotherapy or radiation therapy..
97. The method of item 84, wherein the subject is a bone marrow donor.
98. The method of item 84, wherein the population of cells is autogeneic to the subject.
99. The method of item 98, wherein the population of cells is mobilized from the peripheral blood or bone marrow of the subject.
100. The method of item 84, wherein the population of cells is allogeneic to the subject.
101. A method of preparing a population of cells for increasing hematopoietic stem and progenitor cell expansion *in vivo* comprising: contacting a population of cells comprising hematopoietic stem and progenitor cells *ex vivo* at a temperature of about 37°C, with one or more agents selected from the group consisting of: a prostaglandin E₂ (PGE₂) and an agent having dmPGE₂ activity; wherein the population of cells is contacted with the agent under conditions sufficient to increase the expansion of the contacted hematopoietic stem and progenitor cells *in vivo* compared to non-contacted hematopoietic stem and progenitor cells.
102. The method of item 101, wherein the population of cells is obtained from bone marrow, umbilical cord blood, or mobilized peripheral blood.
103. The method of item 101, wherein the agent is PGE₂ or an analogue thereof.
104. The method of item 101, wherein the PGE₂ analogue is 16,16-dimethyl PGE₂.
105. The method of item 101, wherein the agent is a cAMP enhancer.
106. The method of item 101, wherein the conditions sufficient to increase the expansion of the hematopoietic stem and progenitor cells *in vivo* comprise contacting the hematopoietic stem and progenitor cells:
   (a) at a concentration of about 10 µM or more 16,16-dimethyl PGE₂; and
   (b) for a time of about 1 hour to about 6 hours.
107. The method of item 106, wherein the hematopoietic stem and progenitor cells are contacted with a concentration of about 10 µM 16,16-dimethyl PGE₂; and for a time of about 2 hours.
108. The method of item 106, wherein the population of cells is prepared in an endotoxin-free vessel comprising: a temperature indicating device comprising at least one temperature indicator that produces a signal that indicates the temperature of the vessel and an elapsed time indicating device that comprises at least one elapsed time indicator; and wherein the vessel is suitable for cell storage, treatment of cells, washing of cells, and cell infusion.
109. The method of item 106, wherein the population of cells is administered to a subject in need of cell therapy.
110. A method of increasing hematopoietic stem and progenitor cell expansion in a subject *in vivo* comprising:
   (a) contacting a population of cells comprising hematopoietic stem and progenitor cells *ex vivo,* at a temperature of about 37°C, with one or more agents selected from the group consisting of: a prostaglandin E₂ (PGE₂) or an agent having dmPGE₂ activity; and
   (b) administering the population of cells to a subject;
   wherein the population of cells is contacted with the agent under conditions sufficient to increase the expansion of the contacted hematopoietic stem and progenitor cells in the subject *in vivo* compared to non-contacted hematopoietic stem and progenitor cells.
111. The method of item 110, wherein the population of cells is obtained from bone marrow, umbilical cord blood, or mobilized peripheral blood.
112. The method of item 110, wherein the agent is PGE₂ or an analogue thereof.
113. The method of item 110, wherein the PGE₂ analogue is 16,16-dimethyl PGE₂.
114. The method of item 110, wherein the agent is a cAMP enhancer.
115. The method of item 112, wherein the conditions sufficient to increase the expansion of the hematopoietic stem and progenitor cells *in vivo* comprise contacting the hematopoietic stem and progenitor cells:
   (a) at a concentration of about 10 µM or more 16,16-dimethyl PGE₂; and
   (b) for a time of about 1 hour to about 6 hours.
116. The method of item 115, wherein the hematopoietic stem and progenitor cells are contacted with a concentration of about 10 µM 16,16-dimethyl PGE₂; and for a time of about 2 hours.
117. The method of item 110, wherein the population of cells is prepared in an endotoxin-free vessel comprising: a temperature indicating device comprising at least one temperature indicator that produces a signal that indicates the temperature of the vessel and an elapsed time indicating device that comprises at least one elapsed time indicator; and wherein the vessel is suitable for cell storage, treatment of cells, washing of cells, and cell infusion.
118. The method of item 110, wherein the population of cells comprises one or more cord blood units.
119. The method of item 118, wherein the subject is administered one or more cord blood units.
120. The method of item 118, wherein the subject is administered one cord blood unit or a partial cord blood unit.
121. The method of item 110, wherein the subject has acute myelogenous leukemia (AML), acute lymphoblastic leukemia (ALL), chronic myelogenous leukemia (CML), chronic lymphocytic leukemia (CLL), juvenile myelomonocytic leukemia, Hodgkin's lymphoma, non-Hodgkin's lymphoma, multiple myeloma, severe aplastic anemia, Fanconi's anemia, paroxysmal nocturnal hemoglobinuria (PNH), pure red cell aplasia, amegakaryocytosis/congenital thrombocytopenia, severe combined immunodeficiency syndrome (SCID), Wiskott-Aldrich syndrome, beta-thalassemia major, sickle cell disease, Hurler's syndrome, adrenoleukodystrophy, metachromatic leukodystrophy, myelodysplasia, refractory anemia, chronic myelomonocytic leukemia, agnogenic myeloid metaplasia, familial erythrophagocytic lymphohistiocytosis, or solid tumors.
122. The method of item 110, wherein the subject has breast cancer, ovarian cancer, brain cancer, prostate cancer, lung cancer, colon cancer, skin cancer, liver cancer, pancreatic cancer, or sarcoma.
123. The method of item 110, wherein the patient has received bone marrow ablative or non-myeolablative chemotherapy or radiation therapy..
124. The method of item 110, wherein the subject is a bone marrow donor.
125. The method of item 110, wherein the population of cells is autogeneic to the subject.
126. The method of item 125, wherein the population of cells is mobilized from the peripheral blood or bone marrow of the subject.
127. The method of item 110, wherein the population of cells is allogeneic to the subject.

## Claims

1. A method of preparing a therapeutic composition comprising: contacting a population of cells comprising hematopoietic stem or progenitor cells ex vivo at a temperature of about 33° C to 41°C with one or more agents that increase CXCR4 gene expression in the cells, wherein:
a) at least one of the one or more agents is a cAMP enhancer, a Gα-s activator, an agent that selectively binds the PGE2EP4 receptor, or comprises PGE2 or a PGE2R2 or PGE2R4 agonist;
b) gene expression of CXCR4 is increased in the hematopoietic stem or progenitor cells by at least 5-fold compared to the expression of CXCR4 in non-contacted hematopoietic stem or progenitor cells; and
c) wherein the therapeutic composition comprises a sterile, therapeutically effective amount of hematopoietic stem or progenitor cells ready for administration to a patient.

2. The method of claim 1 wherein:
a) expression of one or more signature genes is increased in the hematopoietic stem or progenitor cells by at least 2 fold compared to the expression of the one or more signature genes in a noncontacted hematopoietic stem or progenitor cell, wherein the signature gene is selected from the group consisting of: hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), and Fos-related antigen 2 (FOSL2);
b) expression of at least two signature genes selected from the group consisting of: hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), and Fos-related antigen 2 (FOSL2) is increased in the hematopoietic stem or progenitor cells by at least 5, 10, 15, or 20 fold compared to the expression of the two signature genes in a non-contacted hematopoietic stem or progenitor cell; or
c) expression of each signature gene selected from the group consisting of: hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), and Fos-related antigen 2 (FOSL2), is increased by at least 2 fold in the hematopoietic stem or progenitor cells compared to the expression of the signature genes in a non-contacted hematopoietic stem or progenitor cell.

3. The method of claim 1, wherein:
a) the population of cells comprises less than 0.10, 0.50, 1.0, 3, 5, 10, 15, 20, or 30% CD34+ cells;
b) the population of cells comprises at least 0.01% and no more than 50% of CD34+ cells;
c) the population of cells is not expanded;
d) the population of cells comprises less than 30%, 25%, 20%, 15%, 10% or 5% mesenchymal stem cells;
e) the population of cells comprises less than 30%, 25%, 20%, 15%, 10% or 5% endothelial progenitor cells;
f) the population of cells is obtained from bone marrow, umbilical cord blood, or mobilized peripheral blood;
g) the population of cells is HLA haplotyped;
h) the population of cells is HLA haplotyped based on the group consisting of HLA-A, HLA-B, HLA-C, and HLA-DRB1;
i) the population of cells is HLA haplotyped and matched with a specific human subject; or
j) the population of cells is HLA haplotyped and has 4 out of 6 HLA matches with a specific human subject.

4. The method of claim 1, wherein the therapeutic composition is substantially free of the one or more agents.

5. The method of claim 1, wherein at least 15% of cells within the population of cells express CXCR4 protein.

6. The method of claim 1, wherein the hematopoietic stem or progenitor cells are contacted with the one or more agents for a time of at least one hour.

7. The method of any one of claims 1-6, wherein the one or more agents comprises PGE2, 16,16-dimethyl PGE2 (dmPGE2), 16,16-dimethyl PGE2 p-(p-acetamidobenzamido) phenyl ester, 11-deoxy-16,16-dimethyl PGE2, 9-deoxy-9-methylene-16,16-dimethyl PGE2, 9-deoxy-9-methylene PGE2, 9-keto Fluprostenol, 5-trans PGE2, 17-phenyl-omega-trinor PGE2, PGE2 serinol amide, PGE2 methyl ester, 16-phenyl tetranor PGE2, 15(S)-15-methyl PGE2, 15(R)-15-methyl PGE2, 8-iso-15-keto PGE2, 8-iso PGE2 isopropyl ester, 20-hydroxy PGE2, 11-deoxy PGE1, nocloprost, sulprostone, butaprost, 15-keto PGE2, 19(R) hydroxy PGE2, 5-[(1E,3R)-4,4-difluoro-3-hydroxy-4-phenyl-1-buten-1-yl]-1-[6-(2H-tetrazol-5R-yl)hexyl]-2-pyrrolidinone; 2-[3-[(1R,2S,3R)-3-hydroxy-2-[(E,3S)-3-hydroxy-5-[2-oxo-cyclopentyl]-hept-5-enoic acid [2-(methoxymethyl)phenyl]pent-1-enyl]-5-oxocyclopentyl]sulfanylpropylsulfanyl] acetic acid; methyl 4-[2-[(1R,2R,3R)-3-hydroxy-2-[(E,3S)-3-hydroxy-4-[3-(methoxymethyl)phenyl]but-1-enyl]-5-oxocyclopentyl]ethylsulfanyl]butanoate; 16-(3-Methoxymethyl)phenyl-ω-tetranor-5-thiaPGE; 5-{3-[(2S)-2-{(3R)-3-hydroxy-4-[3-(trifluoromethyl)phenyl]butyl}-5-oxopyrrolidin-1-yl]propyl]thiophene-2-carboxylate; [4'-[3-butyl-5-oxo-1-(2-trifluoromethylphenyl)-1,5-dihydro-[1,2,4]triazol-4-ylmethyl]-biphenyl-2-sulfonic acid (3-methylthiophene-2-carbonyl)-amide]; or ((Z)-7-{(1R,4S,5R)-5-[(E)-5-(3-chloro-benzo[b]thiophene-2-yl)-3-hydroxy-pent-1-enyl]-4-hydroxy-3,3-dimethyl-2-oxo-cyclopentyl}-hept-5-enoic acid.

8. The method of any one of claims 1-7 wherein at least one of the one or more agents comprises PGE2 or 16,16-dimethyl PGE2.

9. Hematopoietic stem or progenitor cells for use in cell therapy in a subject wherein one or more agents are contacted with a population of cells that comprises hematopoietic stem and progenitor cells ex vivo, at a temperature of about 33° C to 41° C, and wherein the one or more agents are selected from the group consisting of: a prostaglandin E2 (PGE2) and an agent having dmPGE2 activity, and wherein the gene expression of CXCR4 is increased in the hematopoietic stem or progenitor cells by at least 5-fold compared to the expression of CXCR4 in non-contacted hematopoietic stem or progenitor cells.

10. The hematopoietic stem or progenitor cells for the use according to claim 9, wherein the population of cells is obtained from bone marrow, umbilical cord blood, or mobilized peripheral blood.

11. The hematopoietic stem or progenitor cells for the use according to claim 9 or claim 10, wherein the one or more agents comprises PGE2 16,16-dimethyl PGE2 (dmPGE2), 16,16-dimethyl PGE2 p-(p-acetamidobenzamido) phenyl ester, 11-deoxy-16,16-dimethyl PGE2, 9-deoxy-9-methylene-16,16-dimethyl PGE2, 9-deoxy-9-methylene PGE2, 9-keto Fluprostenol, 5-trans PGE2, 17-phenyl-omega-trinor PGE2, PGE2 serinol amide, PGE2 methyl ester, 16-phenyl tetranor PGE2, 15(S)-15-methyl PGE2, 15(R)-15-methyl PGE2, 8-iso-15-keto PGE2, 8-iso PGE2 isopropyl ester, 20-hydroxy PGE2, 11-deoxy PGE1, nocloprost, sulprostone, butaprost, 15-keto PGE2, 19(R) hydroxy PGE2, 5-[(1 E,3R)-4,4-difluoro-3-hydroxy-4-phenyl-1-buten-1-yl]-1-[6-(2H-tetrazol-5R-yl)hexyl]-2-pyrrolidinone; 2-[3-[(1R,2S,3R)-3-hydroxy-2-[(E,3S)-3-hydroxy-5-[2-oxo-cyclopentyl]-hept-5-enoic acid [2-(methoxymethyl)phenyl]pent-1-enyl]-5-oxocyclopentyl]sulfanylpropylsulfanyl] acetic acid; methyl 4-[2-[(1R,2R,3R)-3-hydroxy-2-[(E,3S)-3-hydroxy-4-[3-(methoxymethyl)phenyl]but-1-enyl]-5-oxocyclopentyl]ethylsulfanyl]butanoate; 16-(3-Methoxymethyl)phenyl-(o-tetranor-5-thiaPGE; 5-{3-[(2S)-2-{(3R)-3-hydroxy-4-[3-(trifluoromethyl)phenyl]butyl}-5-oxopyrrolidin-1-yl]propyl]thiophene-2-carboxylate; [4'-[3-butyl-5-oxo-1-(2-trifluoromethylphenyl)-1,5-dihydro-[1,2,4]triazol-4-ylmethyl]-biphenyl-2-sulfonic acid (3-methyl-thiophene-2-carbonyl)-amide]; or ((Z)-7-{(1R,4S,5R)-5-[(E)-5-(3-chloro-benzo[b]thiophene-2-yl)-3-hydroxy-pent-1-enyl]-4-hydroxy-3,3-dimethyl-2-oxo-cyclopentyl}-hept-5-enoic acid.

12. The hematopoietic stem or progenitor cells for the use according to any one of claims 9-11, wherein the one or more agents comprises 16,16-dimethyl PGE2.

13. The hematopoietic stem or progenitor cells for the use according to any one of claims 9-12, comprising contacting the hematopoietic stem and progenitor cells:
(a) at a concentration of 10 µM or more 16,16-dimethyl PGE2; and
(b) for a time of 1 hour to 6 hours.

14. The hematopoietic stem or progenitor cells for the use according to any one of claims 9-13, wherein the hematopoietic stem and progenitor cells are contacted with a concentration of 10 µM 16,16-dimethyl PGE2; and for a time of 2 hours.

15. The hematopoietic stem or progenitor cells for the use according to any one of claims 9-14, wherein the contacted hematopoietic stem and progenitor cells comprise an increase in gene expression of one or more of hyaluronan synthase 1 (HAS1), GTP-binding protein GEM (GEM), dual specificity protein phosphatase 4 (DUSP4), amphiregulin (AREG), Nuclear receptor related 1 protein (NR4A2), renin (REN), cAMP-responsive element modulator (CREM), collagen, type I, alpha 1 (COL1A1), or Fos-related antigen 2 (FOSL2), compared to non-contacted hematopoietic stem and progenitor cells.
